# EUROPEAN PATENT APPLICATION

(11) **EP 4 231 016 A1**
(43) Date of publication of application: **23.08.2023**
(21) Application number: 22157586.3
(22) Date of filing: 18.02.2022
(51) Int. Cl.: G01N 33/58, C07K 14/825, G01N 1/30, G01N 33/84, C07K 14/00

(54) **GENETICALLY CONTROLLED NANOSCOPY CONTRAST-GENERATING UNITS, GENETICALLY CONTROLLED STRUCTURAL ELEMENTS, GENETICALLY CONTROLLED SCAFFOLDS, NANOBIOMATERIAL BASED THEREON, AND USE THEREOF IN NANOSCOPY METHODS**

(71) Applicant: Westmeyer, Gil Gregor, 82319 Starnberg (DE); Helmholtz Zentrum München - Deutsches Forschungszentrum für Gesundheit und Umwelt (GmbH), 85764 Neuherberg (DE)
(72) Inventor: WESTMEYER, Gil Gregor, 82319 Starnberg (DE); SIGMUND, Felix, 80634 Munich (DE); PIOVESAN, Alberto, 80809 Munich (DE); TRUONG, Dong-Jiunn Jeffery, 85356 Freising (DE)
(74) Representative: Grund, Martin

(57) **Abstract**

The present invention relates to a genetically controlled nanoscopy contrast-generating unit comprising a metal interactor, wherein the metal interactor is compatible with nanoscopy fixation protocols, nanoscopy post-fixation protocols, and nanoscopy metal staining protocols and wherein the metal interactor is a molecule to which metal ions can bind to or react with. The metal interactor is not a ferroxidase and is not an enzymatic product from an exogenous substrate. The present invention also relates to a genetically controlled structural element, wherein said genetically controlled structural element organizes the genetically controlled nanoscopy contrast-generating unit. The present invention also relates to a genetically controlled scaffold, wherein said genetically controlled scaffold spatially organizes the genetically controlled structural elements. The present invention also relates to the use of such genetically controlled nanoscopy contrast-generating unit, such genetically controlled structural element, and/or such genetically controlled scaffold for nanoscopy detection methods. The present invention also relates to a nanobiomaterial consisting of the isolated genetically controlled structural elements, and/or genetically controlled scaffolds. The present invention also relates to vectors comprising a nucleic acid encoding a genetically controlled nanoscopy contrast-generating unit, a genetically controlled structural element, and/or a genetically controlled scaffold.

## Description

### FIELD OF THE INVENTION

The present invention relates to a genetically controlled nanoscopy contrast-generating unit comprising a metal interactor, wherein the metal interactor is compatible with nanoscopy fixation protocols, nanoscopy post-fixation protocols, and nanoscopy metal staining protocols and wherein the metal interactor is a molecule to which metal ions can bind to or react with. The metal interactor is not a ferroxidase and is not an enzymatic product from an exogenous substrate. The present invention also relates to a genetically controlled structural element, wherein said genetically controlled structural element organizes the genetically controlled nanoscopy contrast-generating unit. The present invention also relates to a genetically controlled scaffold, wherein said genetically controlled scaffold spatially organizes the genetically controlled structural elements. The present invention also relates to the use of such genetically controlled nanoscopy contrast-generating unit, such genetically controlled structural element, and/or such genetically controlled scaffold for nanoscopy detection methods. The present invention also relates to a nanobiomaterial consisting of the isolated genetically controlled structural elements, and/or genetically controlled scaffolds. The present invention also relates to vectors comprising a nucleic acid encoding a genetically controlled nanoscopy contrast-generating unit, a genetically controlled structural element, and/or a genetically controlled scaffold.

### INTRODUCTION

Biomedical research generates an increasing need for nanoscale mapping of cell-to-cell contacts as well as the intracellular distribution of organelles and biomolecules to decode, for example, structure-function relationships in tissue. Genetically-encodable fluorescent reporters have revolutionized biomedical research thanks to their ease of use and multicolor readout of cellular processes (Rodriguez et al., Trends in Biochemical Sciences, 2017). However, equivalent multiplexable genetic reporters for electron microscopy (EM) that are convenient to use while providing optimal resolution are not available yet.

Generally, it is desirable to achieve nanometer resolution to map, for example, cell-to-cell contacts. One example in which robust nanometer resolution is crucial is mapping cell-to-cell contacts of neuronal cells connected in neuronal networks. Here, the nanometer resolution has to be maintained over imaging volumes with edge lengths ranging from micro- to millimeters unequivocally disentangle intertwined neuronal processes.

Sparse labeling approaches range from silver-based stains used in conjunction with light microscopy (Pannese, Journal of the History of the Neurosciences, 1999) to fluorescent microscopy approaches such as transsynaptic viral tracing (Wickersham et al., Neuron, 2017) transsynaptic fluorescent protein complementation, and randomized combinatorial expression of fluorescent proteins. However, diffraction-limited fluorescence microscopy does not allow high-resolution imaging of, for example, the dense packing of neuronal processes in sub(-cortical) brain regions. Further sparse labeling approaches include super-resolution light microscopy (SRLM) and expansion microscopy (Schermelleh et al., Nature Cell Biology, 2019, Chen et al., Science 2015). SRLM can resolve synaptic contacts but involves detailed, cumbersome local analysis of selected structures labeled with dedicated fluorophores. Expansion microscopy can spatially separate selected molecular targets by pulling them apart via selective affinity handles connecting to an expanding polymer grid. This technology enables an effective sub-diffraction limit resolution with standard diffraction-limited microscopy. It thus allows high-throughput imaging with appropriate engineering solutions to automatize the clearance and isotropic expansion of the chosen targets, including membrane-bound targets to provide anatomical context.

In distinction to sparse labeling approaches, heavy metal stains for volumetric electron microscopy (Hua et al., Nature Communications, 2015) are used that densely label various biomolecules including lipids, proteins, and nucleic acids, which allows automated semantic segmentation of cellular membranes, organelles, protein complexes such as ribosomes, at nanometer-scale resolution over orders of magnitude larger volumes of interest in for example, brains of model organisms such as *C*. *elegans, Drosophila melanogaster, Danio rerio, Danionell translucida, Mus musculus,* as well as from human subjects. Established methods for volume electron microscopy include serial mechanical sectioning, collection, and arraying of individual slices, followed by either automated TEM or SEM/multi-SEM, or ion-based milling by either focused ion beam (FIB) or Gas Cluster prior to multiSEM. However, despite the rich anatomical information contained in the heavy-metal stained tissue, molecular information on the genetic identity and expression profile of cells of interest has to be filled-in via molecule-specific contrast, which is usually obtained via labor-intense correlation to antibody-targeted, or genetically expressed fluorophores. The 3D-distribution of these conventional markers can then be obtained via multiphoton microscopy pre-fixed tissue, followed by post-fixation, heavy-metal staining, and volume EM.

Antibody-coated gold nanoparticles have been used as non-genetic markers that work directly in EM without additional optical microscopy due to their precise site and strong EM contrast (Hainfeld, Science, 1987). However, there are technical problems related to tissue penetration and biodistribution, artificial clustering, and epitope masking due to fixation. Locally triggered polymerization of 3,3'-Diaminobenzidine (DAB) with subsequent heavy metal binding has also been used to generate EM contrast. While this technology has been successfully used in, for example, proximity labeling, specialized protocols for diffusing a polymerizing substrate and an oxidant (3,3'-Diaminobenzidine (DAB), H₂O₂) are required, and the polymerization may lead to spatially unbounded contrast. Accumulating metals directly on genetically encoded proteins bearing tetracysteins, or metallothioneins have also been attempted, but these systems necessitate laborious workflows and incubation with toxic metals or gold nanoparticles.

Therefore, there is an increasing demand for providing robust EM contrast compatible with high-throughput fixation, staining, and acquisition protocols for volume EM of cells, organoids, and tissues. Ideally, said EM contrast is both genetically defined and geometric multiplexed EM and, in addition, also provides robust signals for light microscopy.

The inventors have previously shown that encapsulins can be robustly and non-toxically expressed in mammalian cells as self-assembling nanocompartments that can be visualized in EM, if the native ferroxidase (IMEF) is co-expressed, which oxidizes ferrous iron and leads to the formation of iron-oxide biominerals inside the encapsulin lumen. However, the contrast achievable in most cell types, tissues, or model organisms, such as *Drosophila melanogaster,* is insufficient. This is most likely the case because ferrous iron is limiting.

To obtain sufficiently strong EM contrast with standard fixation, post-fixation, and heavy-metal stain protocols, the inventors have surprisingly found genetically controlled nanoscopy contrast-generating units comprising fixation-stable metal interactors, which can be spatially organized by genetically controlled structural elements to yield unique shapes, in particular rotationally invariant concentric barcodes, which can themselves be spatially organized by genetically controlled scaffolds to produce distinct geometric patterns. The inventors demonstrate *inter alia* how these distinct contrast geometries can be used to augment anatomical EM maps with multiplexed genetically controlled information, to map the subcellular distribution of biomolecules of interest, to sense cellular activation states, and to actuate cellular states and processes biomechanically or biochemically, or via the deposition of electromagnetic energy or magnetic gradients under nanoscopy control in cell culture and biomedical organisms.

### SUMMARY OF THE INVENTION

In one aspect, a genetically controlled nanoscopy contrast-generating unit comprising a metal interactor is claimed. The metal interactor is compatible with nanoscopy fixation protocols, nanoscopy post-fixation protocols, and nanoscopy metal staining protocols, wherein the metal interactor is a molecule to which metal ions can bind to or react with. Such metal interactor is not a ferroxidase and not an enzymatic product from an exogenous substrate.

In one embodiment, the metal interactor comprised in the genetically controlled nanoscopy contrast-generating unit comprises at least one of the following: a metal-interacting or metal-binding peptide or protein, a lipid-binding protein, an RNA-binding protein, a DNA or RNA molecule, a polymerizing or synthesizing enzyme, a biomineralizing enzyme, a bioconjugation tag, or a combination thereof.

In another embodiment, the metal interactor comprised in the genetically controlled nanoscopy contrast-generating unit comprises at least one of the following: a metallothionein, osmiophilic amino acids, a lead binder, a lanthanide binder, a uranyl-binding protein, a vanadium binder, a copper and silver binder, a fatty-acid-binding protein, preferably FABP, a lipid-binding domain of a cytochrome P450, preferably the lipid- and heme-binding domain of the cytochrome p450 BM3h (BMh3), an RNA aptamer binder:RNA aptamer pair, a programmable RNA binding protein, a polymerizing kinase, a phytochelatine synthase, or a tyrosinase, or mixtures thereof.

In yet another embodiment, the genetically controlled nanoscopy contrast-generating unit further comprises a fluorophore or chromophore. The fluorophore is a fluorescent protein, a co-factor in a fluorescent protein, or an exogenous fluorophore. The chromophore is a chromoprotein, a co-factor in a chromoprotein, or an exogenous chromophore. The exogenous fluorophore or the exogenous chromophore can bind directly to the metal interactor.

In one embodiment, the metal interactor comprised in the genetically controlled nanoscopy contrast-generating unit comprises at least one metallothionein, at least one fatty-acid binding protein, at least one lipid-binding domain, or at least one RNA molecule, and a fluorophore, wherein the fluorophore is a fluorescent protein.

In one embodiment, the genetically controlled nanoscopy contrast-generating unit comprises at least one attachment point. Said at least one attachment point is selected from the group consisting of nanobodies, frankenbodies, coiled-coil domains, isopeptide-forming partners, bioconjugation-tags, split-inteins, calmodulin-binding peptides, phosphorylatable peptides, and conformation-changing peptides including troponins, as well as combinations thereof.

In another aspect, a genetically controlled structural element is claimed, wherein said genetically controlled structural element spatially organizes the genetically controlled nanoscopy contrast-enhancing unit, wherein the genetically controlled structural element possesses a pre-determined shape and pre-determined nanoscale size. Said genetically controlled structural element generates a shape that can be differentiated at the nanoscale, and said shape is optionally a barcode.

In one embodiment, the genetically controlled structural element comprises a member of the encapsulin family, a vault, an MS2 phage, a Qbeta bacteriophage, an AP205 phage, an engineered mono-to-polyvalent hub, a filament, a linker, or combinations or variants thereof.

In another embodiment, the genetically controlled structural element comprises at least one attachment point. Said at least one attachment point is selected from the group consisting of nanobodies, frankenbodies, coiled-coil domains, isopeptide-forming partners, bioconjugation-tags, split-inteins, calmodulin-binding peptides, phosphorylatable peptides, and conformation-changing peptides including troponins, as well as combinations thereof. In addition, the genetically-controlled nanoscopy contrast generating unit interacts or binds to the at least one attachment point.

In another embodiment, the genetically controlled structural element is an encapsulin and the genetically controlled contrast-generating units are selected from the group of metallothionein MT3, a series of different metallothionein species, fatty acid-binding proteins, preferably FABPs, and the lipid-binding domain of the cytochrome P450, or combinations thereof. In said embodiment, the lipid-binding domain of the cytochrome P450 is optionally the lipid- and heme-binding domain of the cytochrome P450 BM3 (BM3h). In said embodiment, one to three copies of the MT3 or one to three different metallothionein species are bound to the N-terminus of the encapsulin structural element. In addition, one or more MT3s, FABPs, or BM3hs are bound to the surface of the encapsulin structural element, optionally via a spacer, wherein the spacer is optionally a SasG of variable length. Such structural and spatial organization is configured to generate the distinct shape of a barcode, wherein the barcode is a concentric barcode differentiable at the nanoscale. Such concentric barcode may encode information on cellular states.

In yet another aspect, a genetically controlled scaffold is claimed. The genetically controlled scaffold comprises and spatially organizes the genetically controlled structural element(s), whereby said spatial organization generates distinct geometric patterns that can be differentiated at the nanoscale. Optionally, said distinct geometric patterns encode information on cellular states.

In another embodiment, the genetically controlled scaffold is selected from the group consisting of an endogenous cellular scaffold, a *de novo* designed scaffold, a SasG element, a CsgA element, an RNA or DNA structure, or combinations thereof.

In one embodiment, the genetically controlled scaffold comprises a SasG of variable length, one or more encapsulins as structural element, and at least one contrast generating unit selected from MT3 or a series of different metallothionein species. In such scaffold, the encapsulins are connected and spatially organized via SasG, and comprise the MT3, or the series of different metallothionein species.

In one embodiment, the genetically controlled nanoscopy contrast-generating unit, the genetically controlled structural element and/or the genetically controlled scaffold is compatible with intact cell systems, preferably with mammalian cells and organoids or biomedical model organisms.

In one further aspect, the present invention refers to the use of the genetically controlled structural elements and/or the genetically controlled scaffolds for nanoscopy detection, wherein nanoscopy detection comprises molecular mapping and/or geometric sensing.

In one embodiment, the nanoscopy detection is molecular mapping. Said molecular mapping provides information on the subcellular distribution of a molecule of interest within a cell or tissue via the binding of genetically controlled structural elements and/or genetically controlled scaffolds to a molecule of interest, which thus determines the subcellular localization of the genetically controlled structural elements and/or the genetically controlled scaffolds.

In another embodiment, the nanoscopy detection is geometric sensing. Said geometric sensing provides information on a specific cellular state, a cellular process, the presence or absence of an analyte or environmental parameter of interest within a cell or tissue, or the response to an external stimulus. The shape generated by the genetically controlled structural element(s) and/or the geometric pattern generated by the genetically controlled scaffold(s) changes in response to a pre-defined cellular state, a pre-defined cellular process, at least one analyte, or at least one environmental parameter, or an external stimulus.

In another embodiment, the nanoscopy detection comprising mapping and/or geometrical sensing further comprises geometric actuation. Said geometric actuation alters a cellular state or process by a biomechanical, and/or biochemical effect or via an external stimulus, or the deposition of external energy, electromagnetic radiation, mechanical energy, or magnetic gradients.

In another aspect, the invention relates a nanobiomaterial consisting of isolated genetically controlled structural element(s), and/or the genetically controlled scaffold(s). The genetically controlled structural element(s), and/or the genetically controlled scaffold(s) are thus isolatable from the source of origin, which is preferably a genetically modified cell or a cell-free system.

In yet another aspect, the invention relates to a vector comprising a nucleic acid encoding the genetically controlled nanoscopy contrast-generating unit, the genetically controlled structural element and/or the genetically controlled scaffold.

### BRIEF DESCRIPTION OF THE DRAWINGS

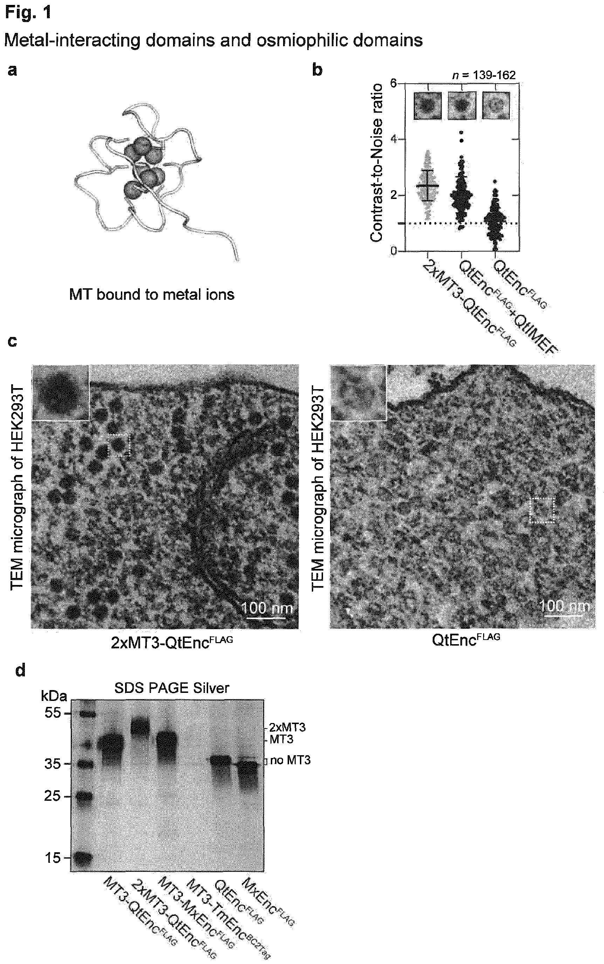

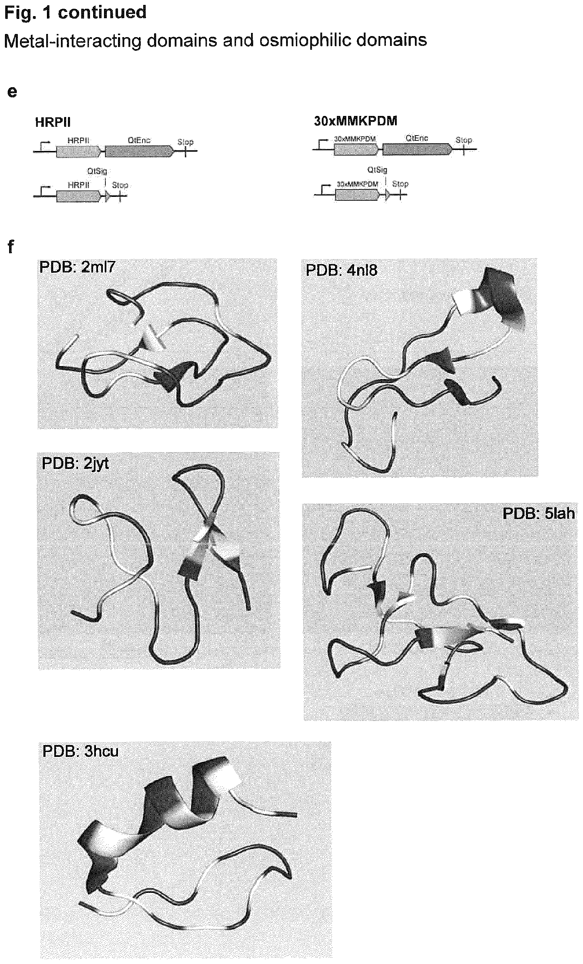

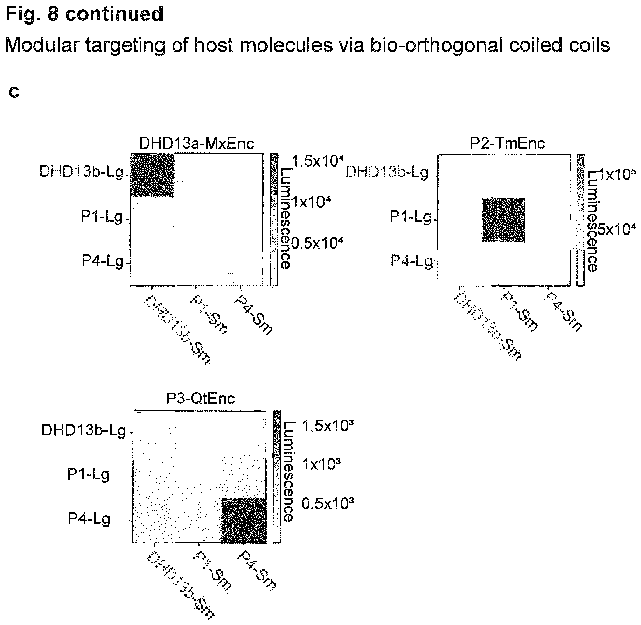

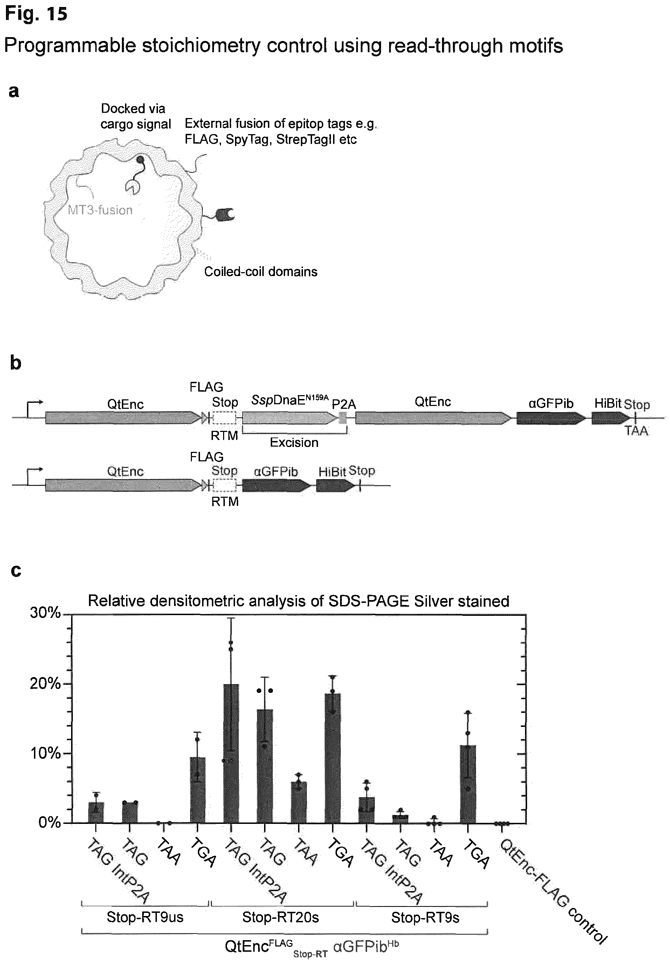

**Figure 1****: Metal-interacting domains and osmiophilic domains**
   **a)** Exemplary 3D structure of yeast metallothionein (MT) (Protein data bank (PDB) accession number: 1AQS) bound to metal ions. Based on its high density in cysteines, MTs are osmiophilic. MTs are also reported to have a high affinity to lead (Pb), which is contained in standard EM stains. Exemplary sequences for these constructs are SEQ ID NO: 1-5 **b)** Contrast-to-noise ratios of individual encapsulin particles as structural elements, which were either non-modified (QtEnc^{FLAG}; SEQ ID NO: 142), fused to a tandem MT3 metallothionein domain as a contrast-generating unit (2×MT3-QtEnc^{FLAG}_{;} SEQ ID NO: 143, or filled with iron-oxides mineralized via a ferroxidase (IMEF) (QtEnc^{FLAG}+QtIMEF) (SEQ ID NOs: 142 and 52), as native cargo protein. **c)** A strong contrast (with a certain shape) can be observed in TEM if the structural element QtEnc spatially organizes the genetically controlled nanoscopy contrast-generating unit comprising the metal-interacting protein MT3; left: TEM micrographs of HEK293T cells expressing QtEnc monomers, which assemble into structural elements N-terminally equipped with a tandem MT3 domain as a contrast-generating unit (2×MT3-QtEnc^{FLAG}); right: TEM micrographs of HEK293T cells expressing unmodified QtEnc structural elements, which form nanocompartments (QtEnc^{FLAG}). Scale bars are 100 nm. **d)** Silver-stained SDS-PAGE loaded with material from an anti-FLAG pull-down experiment from HEK293T cells expressing MT3 or tandem MT3 (2xMT3) in the context of different encapsulin shell monomers, *i.e.*, QtEnc, TmEnc, and MxEnc. The shift towards higher molecular weights indicates that MT domains are readily expressed as contrast generating units. Since the MT3-TmEnc sample has a BC2Tag (SEQ ID NO: 144) instead of a FLAG tag, no band is seen in the gel. **e)** Schematic of genetic expression constructs for the methionine-rich protein from *Pinctada fucata* (30xMMKPDM; SEQ ID NO: 6) and the histidine-rich protein 2 (HRPII), which are expressed as fusion proteins to QtEnc or connected to a targeting moiety (QtSig; SEQ ID NO: 16) for QtEnc encapsulation. **f)** Preferred proteins and protein motifs with high cysteine and/or histidine abundance as osmiophilic residues, identified by screening the Protein Data Bank (specific PDB accession number given) to achieve a high concentration of osmiophilic residues. Exemplary sequences for these constructs are SEQ ID NO: 6-11.

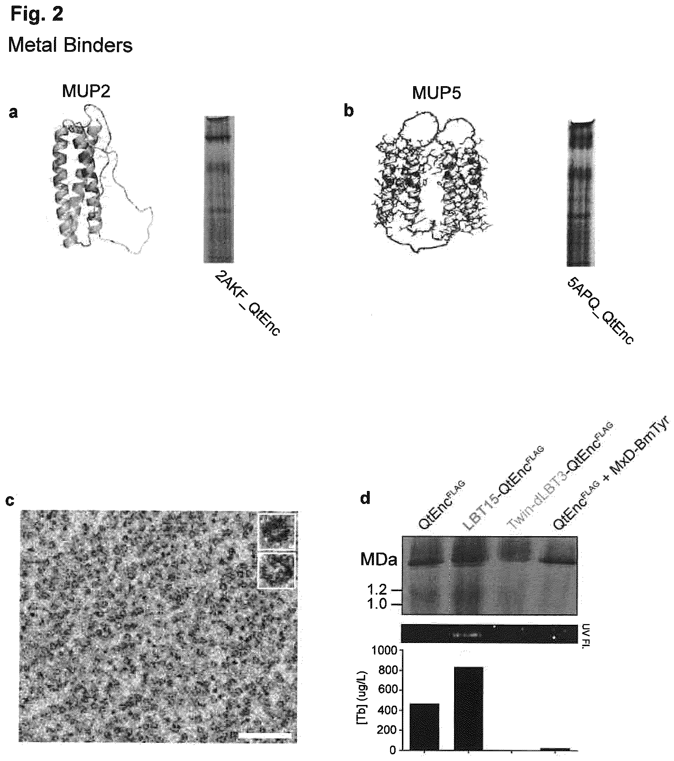

**Figure 2****: Metal-binding proteins**
   **a, b)** Selective examples for metal-binding domains, also called metal binders, which can coordinate heavy metals, such as uranyl, routinely contained in EM stains in the form of uranyl acetate. Shown are two domains into which metal binding sites were grafted, MUP2 (based on PDB:2AKF) SEQ ID NO: 45 and MUP5 (based on PDB:5APQ) SEQ ID NO: 46. The gels show a CN-PAGE (Clear Native Polyacrylamide Gel Electrophoresis) analysis of the expression of these domains as direct fusions to QtEnc encapsulin. **c)** Example of a TEM micrograph from MUP2-QtEnc (SEQ ID NO: 148) showing ring-contrasts (specific shape of a concentric barcode) in the 2D projection of the spherical QtEnc used as a structural element. Scale bar is 50 nm. **d)** BN-PAGE (Blue Native Polyacrylamide Gel Electrophoresis) analysis of encapsulin variants fused to lanthanide binding tags (LBT). The QtEnc variant fused to LBT15 yields a stable assembly (LBT15-QtEnc^{FLAG}; SEQ ID NO: 145). After adding terbium chloride, tryptophan-sensitized luminescence can be observed upon UV-illumination (UV-FL), demonstrating lanthanide binding to the structural element, also called nanosphere. This finding is supported by ICP-MS measurements (corresponding bar graph on bottom) showing Tb-enrichment in the LBT15 QtEnc variant compared to another variant (Twin-dLBT3; SEQ ID NO: 146) which worked less efficient and the empty encapsulin QtEnc^{FLAG}.

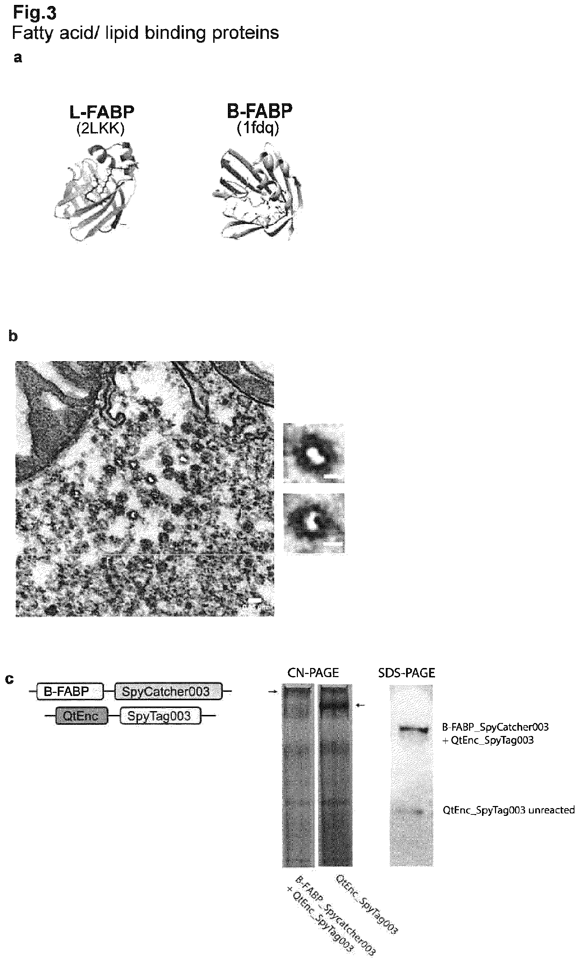

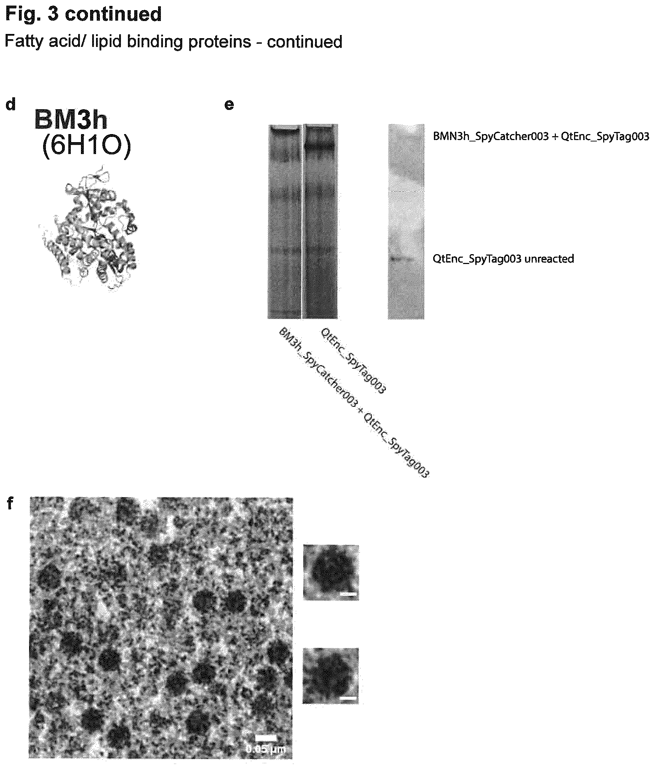

**Figure 3****: Fatty acid-/lipid binding proteins**
   **a)** Antiparallel β-barrel structures of L-FABP (PDB: 2LKK) and B-FABP (PDB: 1fdq) with an internal binding site that binds a fatty acid in an interior cavity. **b)** TEM image of HEK cells co-expressing QtEnc-SpyTag003 (SEQ ID NO: 147) and B-FABP-SpyCatcher003 SEQ ID NO: 150, respectively, resulting in contrast enhancement (shape of concentric barcodes) depending on how many B-FABP molecules are bound to a given QtEnc. Two zoom-ins placed on the right show the localized contrast enhancement. Scale bars for the zoom-ins are 20 nm. **c)** Schematic of the genetic constructs for a two-component system to fuse B-FABP via SpyCatcher003 as an attachment point to the encapsulin surface-functionalized with the corresponding SpyTag003 as attachment point (top). Clear-native PAGE gels (bottom left) showing the slowed-down native running behavior upon co-expression of both constructs vs. the expression of only encapsulin QtEnc as a control (bottom left). The corresponding Western Blot shows an additional band for the co-expression of both constructs (bottom right). **d)** Another preferred embodiment of metal interactor comprised in the contrast-generating unit is the ligand-binding heme-domain of the cytochrome P450 BM3 (BM3h), which has been shown to selectively bind arachidonic acid (AA), a polyunsaturated fatty acid, which is a preferred interaction site for osmium tetroxide. **e)** CN-PAGE (left) and (right) analysis of the co-expression of BM3h-SpyCatcher003 (SEQ ID NO: 151) with QtEnc decorated with SpyTags (SpyTag003) (SEQ ID NO: 147). **f)** TEM images (and zoom-ins on the right) of HEK cells co-expressing the structural element QtEnc-SpyTag003 comprising SpyCatcher-Bm3h resulting in contrast enhancement depending on how many BM3h molecules, which are larger than FABPs, are bound to a given QtEnc. The ratio can be controlled by co-expressing structural element QtEnc without SpyTag, which can be achieved via transient expression or via read-through control (Figure 15) also in stable cell lines. Scale bars for the zoom-ins are 20 nm.

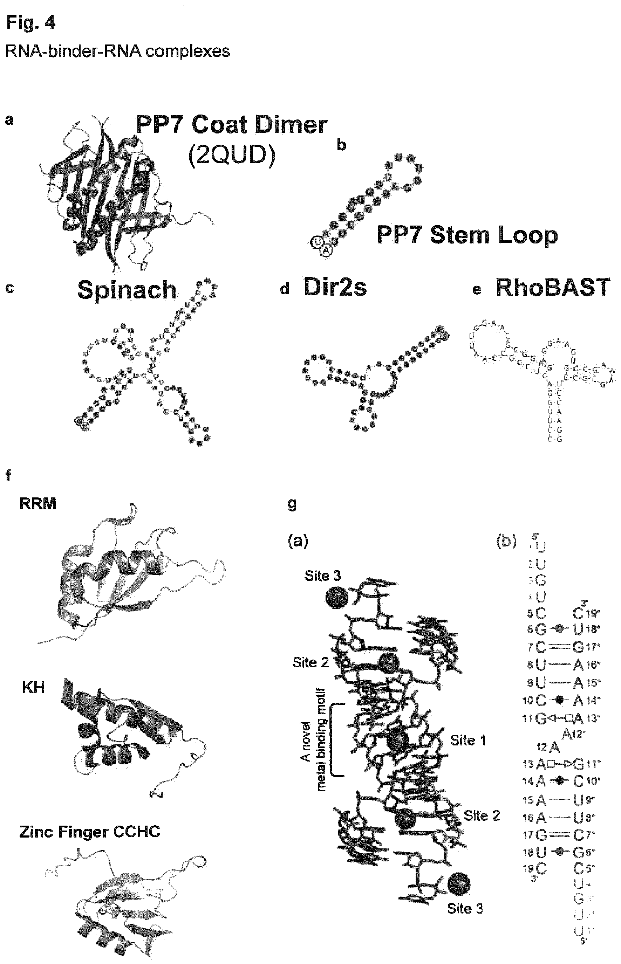

**Figure 4****: RNA, RNA aptamer** - **RNA aptamer-binding protein interactions**
   **a)** Shown is the RNA aptamer PP7 coat dimers (SEQ ID NO: 53), which interact with PP7 loops (SEQ ID NO: 56) attached to RNA aptamers. Other preferred aptamers are MS2 (SEQ ID NO: 152), which interacts with the protein MCP (SEQ ID NO: 100), or H23 (K-turn motif) (SEQ ID NO: 206), which interacts with the protein L7ae. (SEQ ID NO: 63). Here, the RNA-binding protein functions as a CGU. In other embodiments, the RNA aptamer-binding protein functions as a structural element. The aptamers, in this case, contain **b)** PP7 stem-loop. For dual detection by EM and fluorescent microscopy, RNA aptamers such as **c)** Spinach, SEQ ID NO: 55 **d)** Dir2s, SEQ ID NO: 57 and **e)** Rho-BAST SEQ ID NO: 59 may be chosen because they bind to differently colored fluorophores and also enable super-resolution SRLM. Aptamers may also contain multiple binding sites for small fluorophores. If necessary, the aptamers can be stabilized for mammalian expression via circularization. **f)** Alternatively, or supportive to the use of RNA aptamer-binding proteins such as PCP as the CGU or structural element, protein motifs such as vertebrate RNA-recognition motif (RRM, PDB:3MDF; SEQ ID NO: 60), the very well conserved human K homology domain (KH, PDB:5WWX; SEQ ID NO: 61), or the Zinc Finger CCHC-type which binds RNA specifically (CCHC, PDB:2E5H; SEQ ID NO:62) are applied, which can bind RNA also unspecifically. **g)** In addition to relying on the unspecific interaction of heavy metals with the phosphates of RNA, specific metal-binding sites such as the one shown here (*see* also Kanazawa and Kondo, Journal of Inorganic Biochemistry, (2017), 176:140-143) (SEQ ID NO: 58) can be used, also in combination with fluorophore-binding RNA aptamers such that co-localization upon formation of the antiparallel duplex can also be monitored via fluorescence imaging. Other metal-binding RNA motifs, including magnetic species such as nickel and cobalt, can be taken from metal-sensing ribozymes.

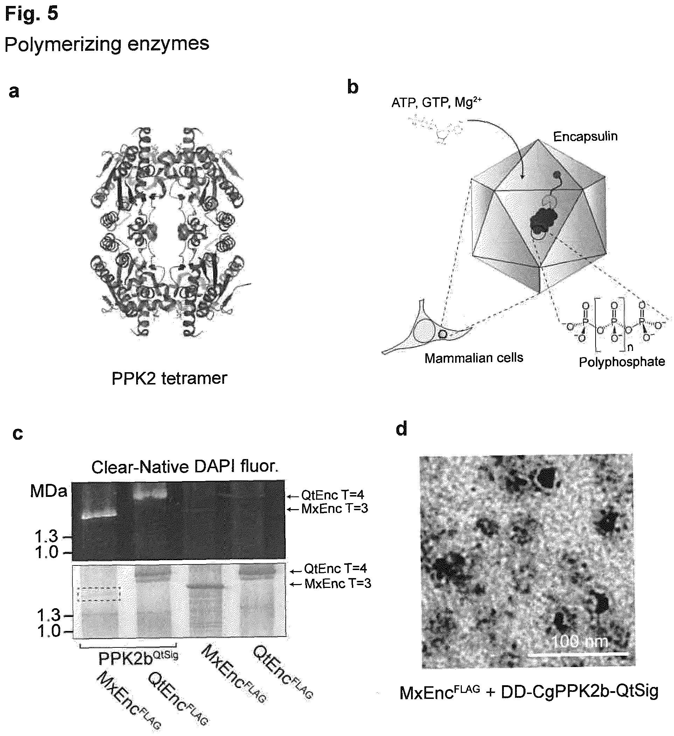

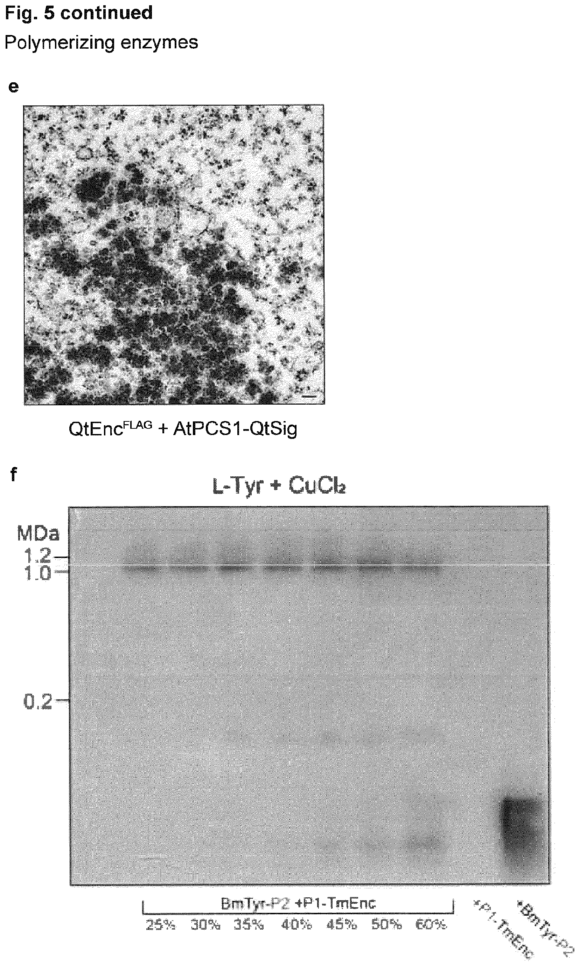

**Figure 5****: Polymerizing enzymes**
   **a)** 3D structure of the tetrameric polyphosphate kinase 2 (PPK2) of *F. tularensis* (PDB: 5LL0). PPKs can turn over the endogenously available substrates GTP or ATP to polymerize highly negatively-charged polyphosphate chains (polyP). **b)** Schematic of DD-CgPPK2b-QtSig (SEQ ID NO: 47) targeted to/spatially organized in the structural element QtEnc lumen via the endogenous QtEnc encapsulation signal (QtSig). The QtSig encapsulation signal is "KGFTVGSLIQ" (SEQ ID NO: 16). It is the C-terminal fraction after the 2xGGGGS-Linker (SEQ ID NO: 153) in the sequence "DD-CgPPK2b-QtSig". Cg stands for the organism of origin *Corynebacterium glutamicum.* DD stands for a degron motif (SEQ ID NO: 154), leading to the degradation of the cargo protein (in this case, the PPK2 kinase) unless it is encapsulated in the lumen of encapsulin. ATP/GTP, endogenously present in cells, can enter via the pores of the structural element and is polymerized into a polyP chain, which is trapped inside the encapsulin lumen **c)** Clear-Native PAGE analysis of lysates of HEK293T cells expressing QtEnc^{FLAG} (SEQ ID NO: 104) or MxEnc^{FLAG} (SEQ ID NO: 107) alone or co-expressing DD-CgPPK2b-QtSig. The gel was stained with DAPI, exhibiting a yellow shift upon binding to polyP. The UV fluorescence imaging of the gel (upper panel) reveals bands indicating intraluminal polyP accumulation. No UV fluorescent bands are observed when no cargo protein is co-expressed. **d)** TEM micrograph of HEK293T cells expressing MxEnc^{FLAG} + DD-CgPPK2b-QtSig. Inside the ^{~}32 nm encapsulins as structural elements, shapes generated by highly contrasted polyP cores can be observed. The scale bar is 100 nm. **e)** TEM micrograph of HEK293T cells expressing QtEnc^{FLAG} + Phytochelatine-synthase 1 fused to an encapsulation signal (AtPCS1-QtSig; SEQ ID NO: 49). "At" stands for the organism of origin *Arabidopsis thaliana.* The highly contrasted areas indicate heavy metal-binding to the intraluminal phytochelatins. The scale bar represents 100 nm. **f)** Clear native PAGE loaded with lysates of HEK293T cells co-expressing TmEnc comprising the coiled-coil P2 (SEQ ID NO: 159) as a cargo adapter fused to a bacterial tyrosinase (BmTyr; SEQ ID NO: 51) via the encapsulation signal P1. Bm stands for *Bacillus megaterium* as the organism of origin of the tyrosinase. Please also see preferred coiled-coil pairs for the respective encapsulins, as shown in Figure 8c. The gels were incubated with 100 µM copper chloride and 2 mM L-Tyrosine. The dark high molecular weight bands at 1.0 MDa represent melanin-loaded TmEnc nanospheres as structural elements that also attract heavy metal ions.

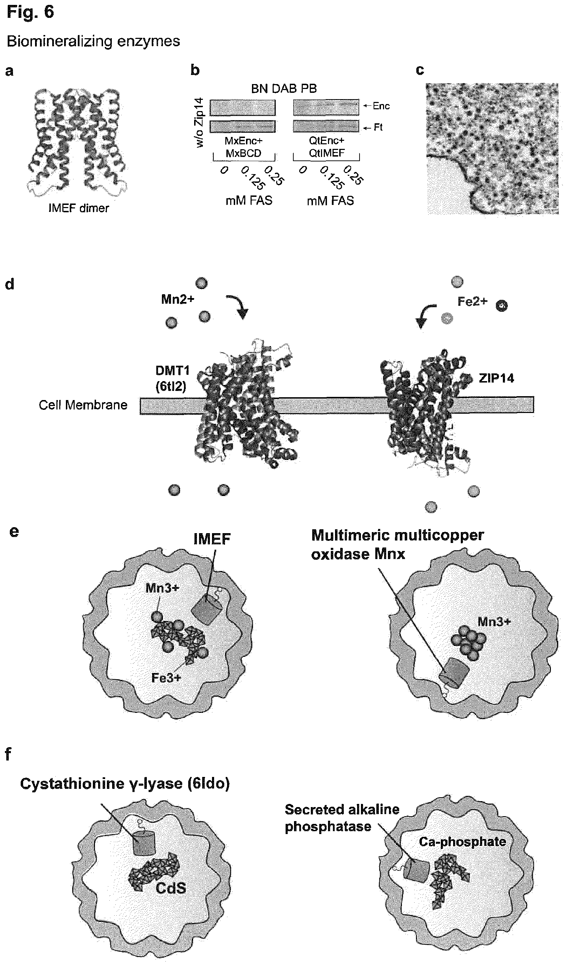

**Figure 6****: Biomineralizing enzyme**
   Enzymatically generated biominerals such as iron-oxide are a resin to which heavy metals such as lead can bind to in order to intensify EM contrast. The stand-alone ferroxidase within the encapsulin system (IMEF; SEQ ID NO: 52) generates iron-oxide precipitates within the encapsulin nanocompartments as structural elements that are themselves already electron-dense and are thus also suitable for EM techniques that do not involve heavy-metal stains, such as cryo-ET. **a)** 3D chart of a QtlMEF dimer. **b)** Coomassie and Prussian-Blue stained BN-PAGE demonstrating QtEnc iron loading in mammalian cells upon supplementation offerrous ammonium sulfate. **c)** TEM micrograph of HEK293Tcells showing contrasted shapes, generated by QtEnc as structural element with biomineralized iron oxide cores. **d)** The metal biomineralization can be supported by co-expression of metal importers for ferrous iron (Zip14, SEQ ID NO:219) and manganese (DMT1, SEQ ID NO: 218) such that doping of iron biominerals with manganese can be obtained. **e)** The ferroxidase activity of IMEF can here be supported by the multicopper oxidase Mnx (SEQ ID NOs: 222, 223, 224). **f)** Alternatively, cystathionine-y-lyase (SEQ ID NO:220) can lead to the release of cysteines and subsequent formation of Sulfur clusters. Alkaline phosphatase (SEQ ID NO:221) can trigger the intraluminal formation of calcium-phosphate precipitates to which heavy metals can bind.

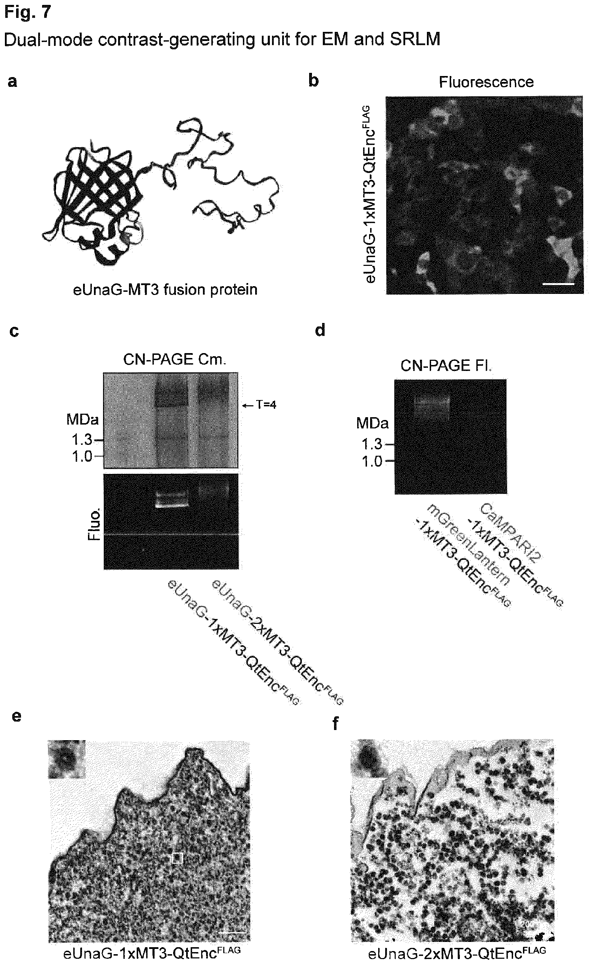

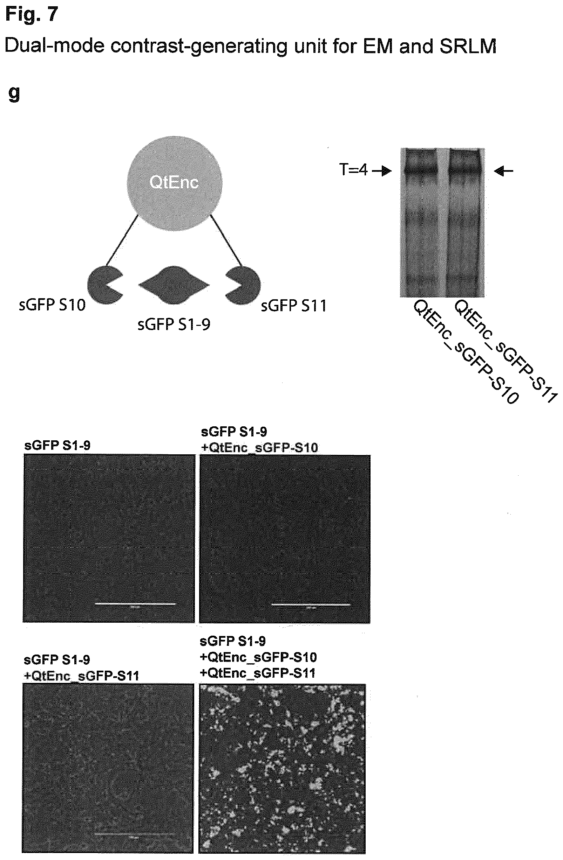

**Figure 7****: Dual-mode contrast-generating unit for EM and SRLM**
   **a)** Predicted 3D model of a fusion of the ultra-small engineered fluorescent protein eUnaG and MT3 yielding contrast for fluorescence techniques as well as EM. **b)** Widefield fluorescence microscopy of HEK293T cells expressing eUnaG-MT3-QtEnc^{FLAG} (SEQ ID NO: 155). **c)** CN PAGE analysis (upper blot) of lysates from HEK293 cells expressing either eUnaG-MT3-QtEnc or eUnaG-2xMT3-QtEnc^{FLAG} (SEQ ID NO: 156). The UV fluorescence image (lower blot) shows the bands corresponding to the assembled high-molecular weight encapsulin fusion emitting green light. **d)** CN PAGE loaded with lysates of HEK293T cells expressing either mGreenLantern (SEQ ID NO: 157) or CaMPARI2-MT3-QtEnc (SEQ ID NO: 158) fusions. The UV fluorescent image shows green fluorescent bands corresponding to the assembled nano-compartments as structural elements. These data show that even larger fluorescent protein cargos can be placed upstream of the EM-contrast generating unit MT3. **e)** TEM micrograph of HEK293T cells expressing eUnaG-MT3-QtEnc^{FLAG} showing highly contrasted shapes, *i.e.*, ^{~}40 nm spherical barcodes generated by the encapsulin with a distinct lumenal phenotype. A contrasted internal ring with a smaller diameter is visible, as well as a bright center spot (see, zoom-in). **f)** TEM micrograph of HEK293T cells expressing eUnaG-2xMT3-QtEnc^{FLAG}. The presence of double the numbers of MT3 contrast generating units fills the lumen of the encapsulin structural element such that it can be distinguished from e) as a distinct concentric barcode. **g)** While fusions of fluorescent proteins to the C-terminus of encapsulin monomers, facing to the outer surface of the structural element, are likely to prevent proper encapsulin assembly, the inventors could reconstitute tripartite split-GFP on the outer surface of encapsulin by co-expression of C-terminal fusions of GFP S10 (SEQ ID NO: 25) and GFP S11 (SEQ ID NO: 26) to QtEnc and co-expression (or alternatively also C-terminal fusion) of Split GFP1-9 (left scheme) (SEQ ID NOs: 23, 24 respectively). Correct assembly of the fusion constructs is shown via BN-PAGE, and reconstituted fluorescence is shown via epifluorescence microscopy only if all three components are expressed (on the right and bottom panels).

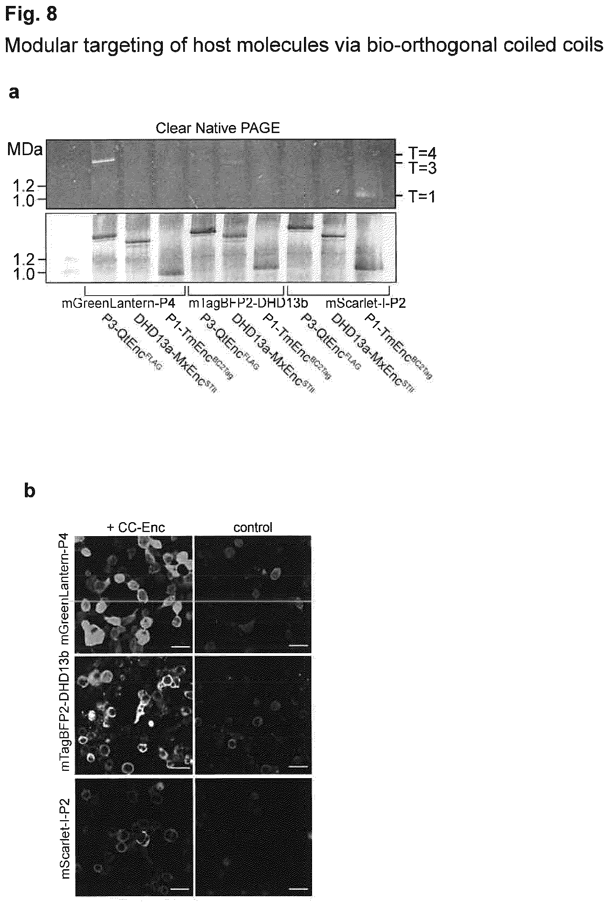

**Figure 8****: Modular targeting of host molecules**
   Multiplexed targeting of guest molecules to the inside of structural elements can be achieved via bioorthogonal coiled-coil libraries. This modular targeting enables multimodal detection in addition to EM contrast. **a)** Shown here are coiled-coils targeting different fluorescent proteins selectively to the lumen of encapsulins (with different triangulation numbers: QtEnc (T=4), MxEn (T=3), TmEnc (T=1)) harboring the corresponding coiled-coil binding partner. The CN-PAGE panel on top shows the fluorescent signal of the cargo proteins encapsulated in either the T1, T3, or T4 encapsulins. The figure demonstrates that the given fluorescent protein (FP) fused to a given coiled-coil will only be encapsulated if the encapsulin structural element carries the cognate coiled-coil (CC) partner. Best working CC-pairs to ensure bio-orthogonal targeting to the Tm, Mx, or Qt encapsulin species have been identified and described herein. This targeting strategy allows for bio-orthogonal targeting of fluorescent proteins, which is not possible with the promiscuous native encapsulation signal of encapsulins. Exemplary sequences for this construct is provided in SEQ ID NO: 159-164. **b)** Corresponding fluorescent microscopy images show an enrichment and stabilization of the coil-coil-tagged fluorescent proteins inside the lumen of the encapsulin shells with the corresponding coiled-coil binding partners. Alternatively or in addition, the CCs can also be linked with an MT3 linker to enable EM and fluorescent detection (SEQ ID NO: 21 with an example for a cognate coiled-coil binding partner with SEQ ID NO: 22). Alternatively, exogenous fluorophores can be bioconjugated via SNAPtags or Halotags fused to the inner surface of encapsulins. Exemplary sequences (SEQ ID NO: 210, 211) **c)** Independent demonstration of the highly selective binding of the selected pairs of coiled-coils inside the lumen of encapsulin, here measured by bioluminescent photon counts from a luciferase cargo. Coiled-coils were chosen from Lebar et al., Nat.Chem.Biol. (2020); Chen et al., Nature, (2019).

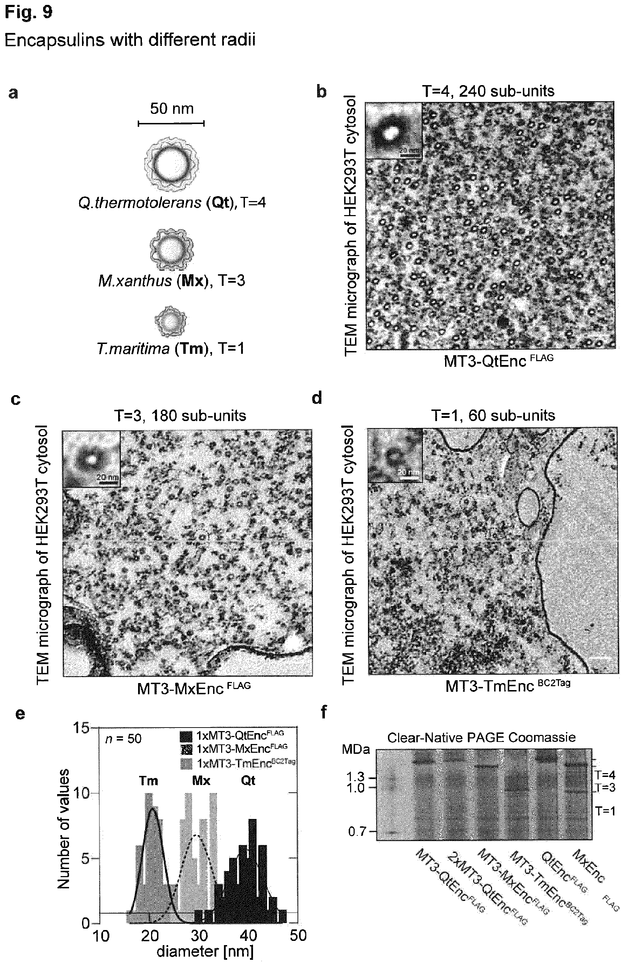

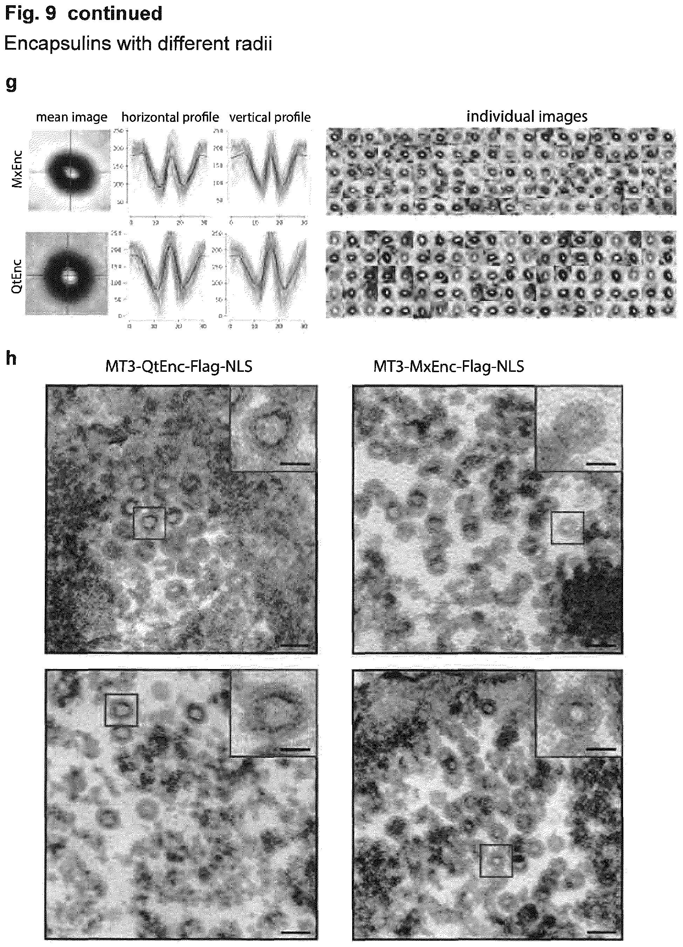

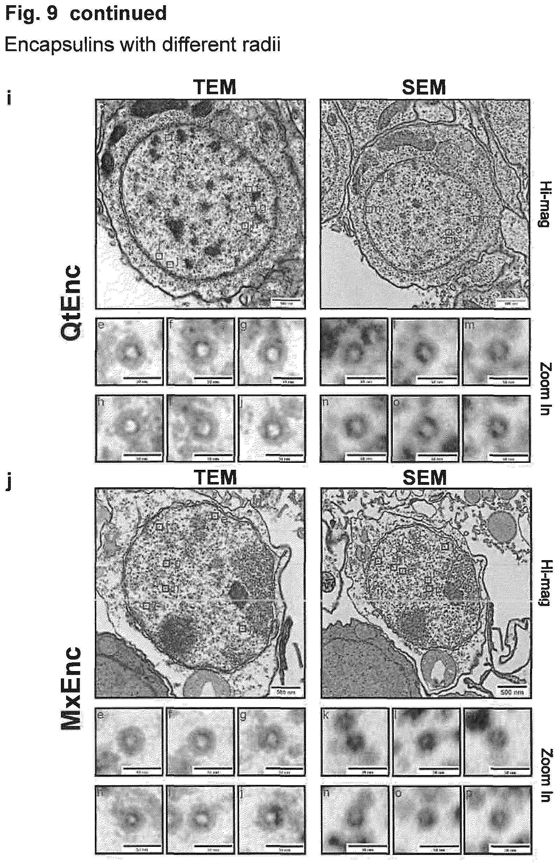

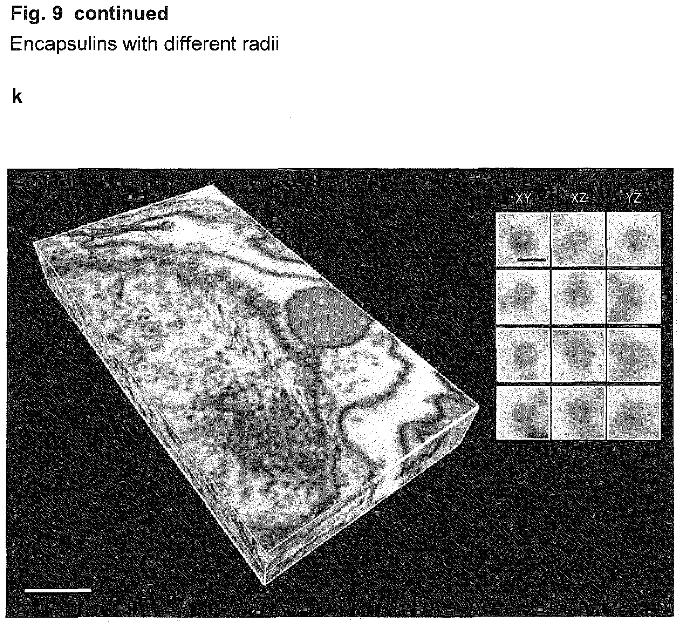

**Figure 9****: Encapsulins with different radii:**
   **a)** Schematic representation of encapsulin systems with different triangulation numbers, resulting in the following radii: *Q.thermotolerans* (Qt; T=4, 240 sub-units, 40 nm;), *M.xanthus* (Mx; T=3, 180 sub-units, 32 nm;), and *T.maritima* (Tm; T=1, 60 sub-units, 20 nm;). **b)-d)** ExemplaryTEM micrographs of HEK293T cells expressing either MT3-QtEnc^{FLAG} SEQ ID NO: 165), MT3-MxEnc^{FLAG} (SEQ ID NO: 166), or MT3-TmEnc^{BC2Tag} (SEQ ID NO: 144). The scale bar is 100 nm, and the scale bar in the zoom-ins is 20 nm. An exemplary sequence for this construct is provided in SEQ ID NO: 212. **e)** Frequency distribution of diameters of the encapsulin nano-spheres as structural elements shown in b)-d). **f)** CN-PAGE analysis of lysates of HEK293T cells expressing MT-fused encapsulin variants of varying radii. The MT-fused variants have the same electrophoretic mobility as the wild-type variants indicating that the MT domains (contrast-generating units) are lumenal and do not impair encapsulin assembly or change their geometric properties. **g)** Mean image (left) of 100 manually segmented instances of TEM images of MT3-MxEnc and MT3-QtEnc expressed in HEK293 cells (shown right). Intensity profiles along the indicated on the mean images on the left (center panels), showing the distinct inner diameters of the white center and the outer black ring. Importantly, the geometrically distinct EM contrast can not only be detected in TEM, capable of high magnifications, but also in SEM compatible with high-throughput analysis by volume EM. **h)** Higher-magnification TEM micrographs of *Drosophila* neurons in the optic lobe expressing MT3-QtEnc-FLAG-NLS (left) or MT3-Mx-FLAG-NLS (right). Scale bars are 50 nm (20 nm for inset). (Exemplary sequence for a fusion of the NLS to FLAG is provided in SEQ ID: 167). **i)** juxtaposition of TEM and SEM images taken from brains of a *Drosophila melanogaster* line (elav-Gal4 × UAS-QtEnc^{FLAG}-NLS; SEQ ID NO: 168), which was fixed and stained with standard protocols. Ultrathin sections were then captured either on TEM grids or on silica wafers for subsequent analysis by SEM (inverted contrast), allowing for the analysis of the identical cell. j) Same protocol as in h) but with MxEnc expressed in all neurons of the nervous system of *Drosophila melanogaster* (elav-Gal4 × UAS-MxEnc^{FLAG}-NLS; SEQ ID NO: 167). **k)** MT3-QtEnc expressed in *Drosophila melanogaster* neurons. The tissue was fixed, embedded, stained, and prepared for FIB milling. FIB milling uses a focused ion beam to sequentially ablate material from a sample to enable volumetric imaging. After each milling step, an SEM image is acquired, and 3D information can be reconstructed from the resulting image series. An image volume of 3696 nm × 1956 nm × 204 nm (image width × image height × milling length) was acquired on a Crossbeam 550 FIB-SEM (Zeiss) with a voxel size of 4 nm using an InLens detector at 1.3 kV and a working distance of 5 mm. FIB beam settings: 30 kV/700 pA. Acquisition time: 67 minutes. The scale bar in the overview image represents 400 nm, the scale bar in the cropped image represents 50 nm. Inset: Ortho-slices through selected nanospheres.

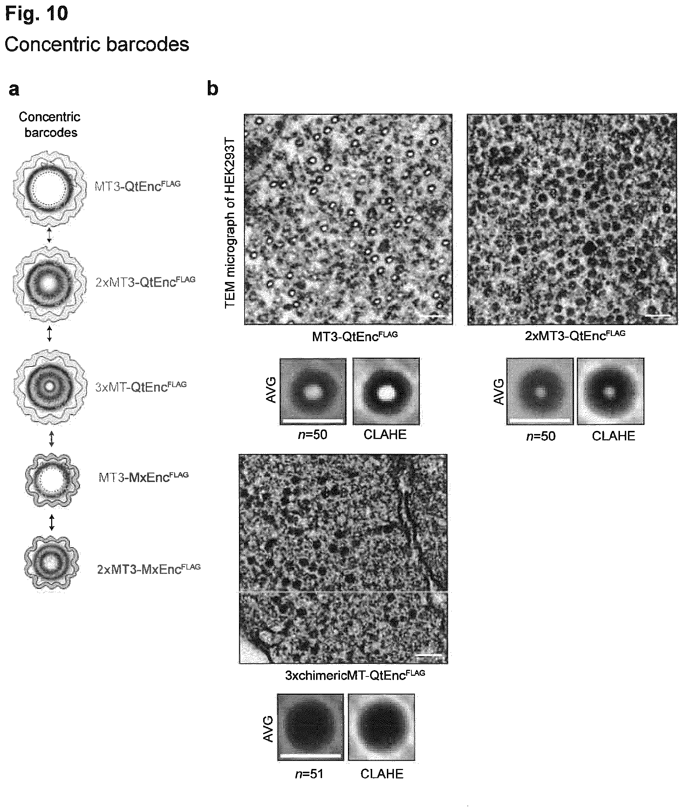

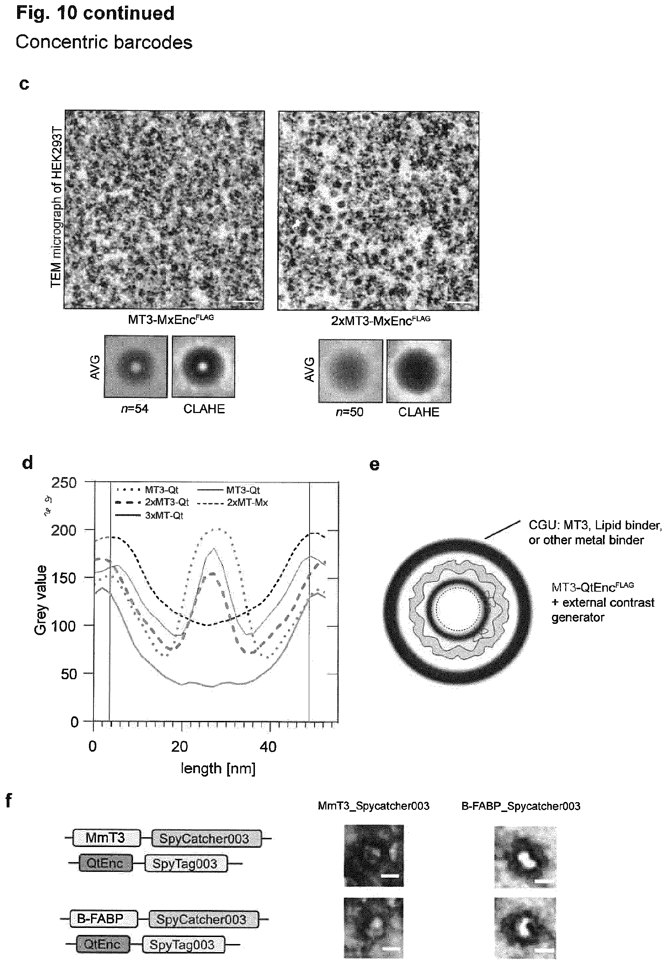

**Figure 10****: Concentric barcodes**
   Structural elements based on the concatenation of MT3 domains fused to Enc monomers produce differential concentric barcodes. **a)** Scheme showing the single, double, or triple repeat MT-domains fused to the N-terminus of QtEnc, which provides increasingly more osmiophilic cysteine residues that also reach further into the lumen of the encapsulin shell. Note that for the triple repeat, a chimeric metallothionein series from multiple organisms was used to minimize redundant DNA sequences, which can be unstable (*see*, *e.g*., SEQ ID NOs: 5, 169). **b)** TEM image of ultra-thin heavy metal-stained slices of HEK293T cells expressing MT3-QtEnc^{FLAG} resulting in an annular electron-dense ring with a bright center (left), 2xMT3-QtEnc^{FLAG} with a much smaller bright center, and 3xchimericMT3-QtEnc^{FLAG}, which adds an additional layer of contrast into the spherical structural element, *i.e.*, an additional concentric ring in the 2D projection of the TEM, leaving no white center. **c)** Corresponding barcode shapes for the MxEnc with smaller outer diameter. Singular or tandem MT-domains fused to the N-terminus of MxEnc. **d)** Line plots through the center of an exemplary MT3-QtEnc^{FLAG} and 2xMT3-QtEnc^{FLAG} particle. Scale bar: 100 nm. **e)** Schematic showing how additional contrast generating units (CGU), such as MT3 or FABP can be added to the outer surface of encapsulins (or other structural elements) via direct fusion, SpyTag/SpyCatcher interaction, or intrabody/XFP interaction (SEQ ID NOs: 191, 192). The spacing between such additional concentric barcodes can be controlled by a rigid spacer such as SasG. The rotational invariance of the concentric barcodes ensures a robust readout in nanoscopy even if an isotropic resolution cannot be obtained. In addition to encapsulins, the concentric assembly can also be achieved by other spherical structural elements such as ferritin or viral capsids, such as MS2 (SEQ ID NO: 100), Acinetobacter phage AP205, or Qbeta phages. **f)** Examples for outer contrast rings obtained by either binding the contrast-generating units MmMT3 (Mm stands for *Mus musculus,* SEQ ID NO: 1) or B-FABP (exemplary sequence SEQ ID NO: 36) to the outer surface of encapsulin via the isopeptide forming partners SpyTag/SpyCatcher. The construct comprising MmT3 is given as SEQ ID NO: 171. The construct comprising B-FABP is given as SEQ ID NO: 150. The TEM micrographs on the right show the corresponding concentric barcodes added to the outer surface of encapsulin, which can be modularly combined with intraluminal modifications with MT3.

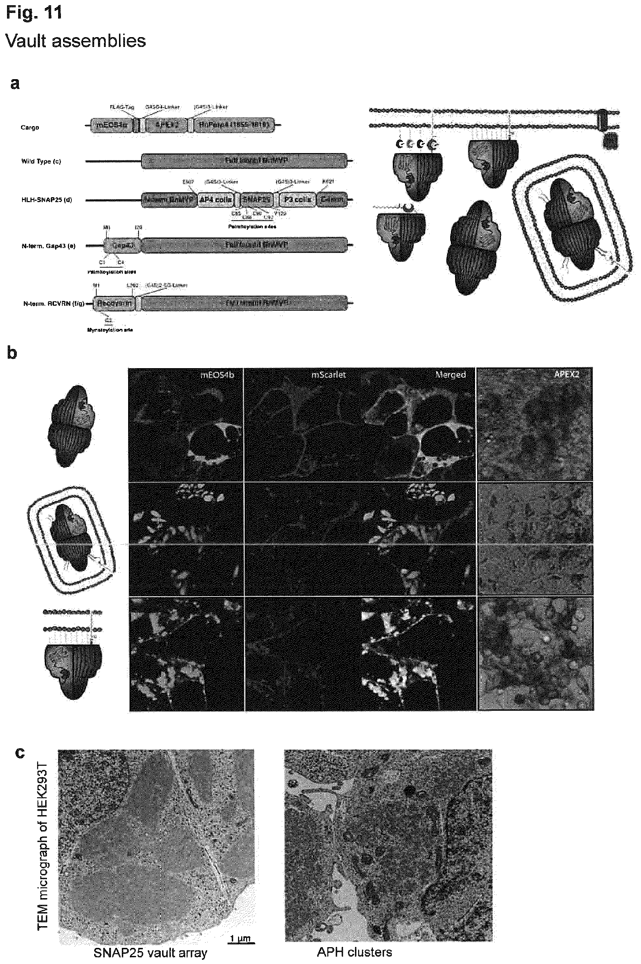

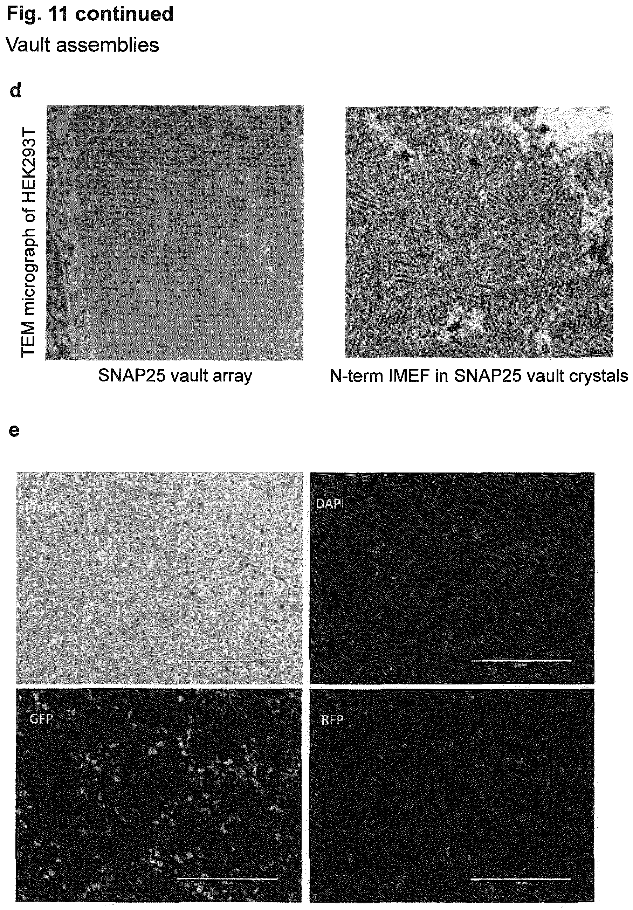

**Figure 11****: Vault assemblies**
   The claimed structural elements comprise vaults. Vaults consist of 2 hemi-vaults, each consisting of 78 sub-units of the major vault protein (PDB: 6BP7). **a)** The schematic (left) and graphics (right) show several of the engineered vault constructs. The bimodal fluorescent and EM visible cargo (mEoS4b fused to APEX2; SEQ ID NO: 208) are connected to a targeting moiety (mINT, SEQ ID NO: 170), enabling cargo packaging into the vault compartment. In comparison to the wild-type vault, in which both termini face to the inside of the nanocompartment, an external attachment point is installed via insertion of palmitoylation sites contained within SNAP25 (SEQ ID NO: 78) via two helix-loop-helix motifs (antiparallel (AP) AP4 and P3 coils). Alternatively, an S-palmitoylation signal contained in Gap43 (SEQ ID NO: 209) is placed on the N-terminus to prevent the holovault assembly from two halves but instead target a hemi-vault to the membrane. **b)** The corresponding fluorescent images show the distribution of mEos4b as destabilized cargo to the vault compartments. Whereas the distribution of mEos4b inside the wild-type holovaults is homogeneous inside the cytosol, the SNAP25-modified vaults show micrometer-sized green-fluorescent arrays, while the Gap43-modified variants SEQ ID NO: 209 show intracellular membraneous clusters. **c)** TEM micrographs showing the large arrays of the SNAP25-modified vaults (left) (SEQ ID NO: 78). In comparison, the expression of vaults modified with APH peptides (using regular and truncated antiparallel CCs from GA354 (SEQ ID NO: 79) and GA355 (APH) (SEQ ID NO: 80), on their surface results in clusters of different packing. **d)** Comparison of distinct patterns generated in EM by vault constructs with external SNAP25 motif (left) and with IMEF as N-terminal fusion (right). **e)** Fluorescence microscopy images of HEK293T cells expressing the major vault protein where a small fluorescent UnaG protein was fused to the internal HLH domain, and the red fluorescent protein mScarlet was targeted to the lumen via an mINT motif. An exemplary sequence for this construct is provided in SEQ ID NOs: 77-85.

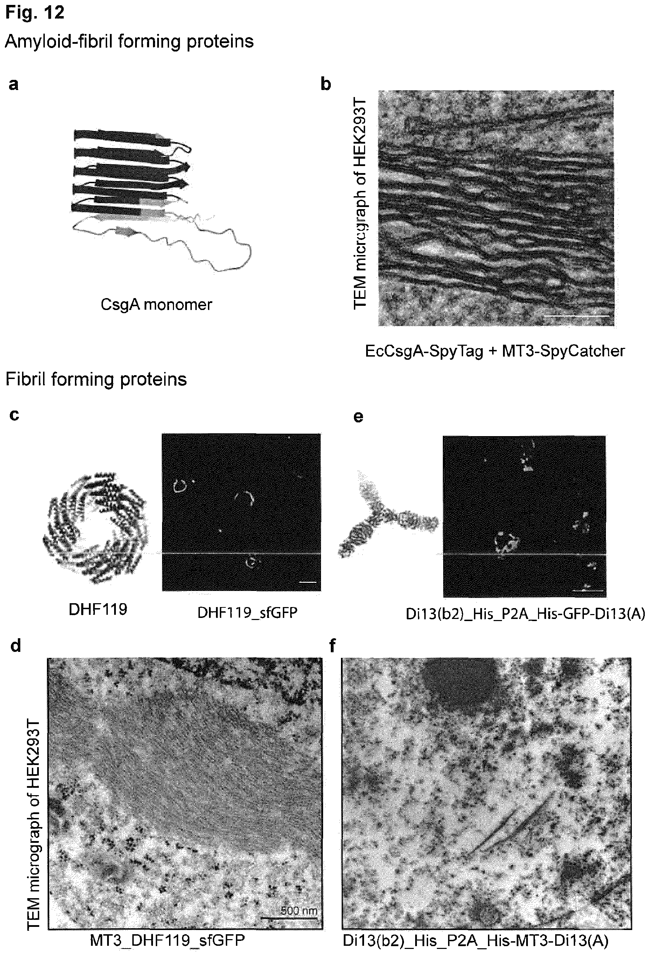

**Figure 12****: Nanofibrils and clusters**
   Contrast-generating units such as MT3 can be added to naturally occurring or *de novo* designed fibrils or lattice-forming components to generate EM-contrasted structural elements. **a)** AlphaFold prediction of the major curlin subunit CsgA from *E.coli.* This protein has been shown to form long amyloid-like fibers. **b)** CsgA optimized for intracellular expression fused to a SpyTag was co-expressed with MT3 fused to the cognate SpyCatcher binding partner (SEQ ID NOs: 112, 171), yielding contrast-enhanced fibers. The TEM micrograph shows bundles of these highly contrasted fibers in the cytosol of HEK293T cells. As an alternative embodiment, *de novo* designed fibril forming proteins may be used for the generation of a structural element: **c)** 3D image of a cutaway view of a *de novo* designed filament-forming protein (DHF119) (left). Confocal fluorescence microscopy of HEK293T cells expressing DHF119-sfGFP (right) (DHF119 published in Shen et al., Science (2018)). DAPI-stained nuclei are depicted blue, whereas the green signal shows the perinuclear DHF119 filaments. The scale bar is 20 µm. **d)** Corresponding TEM micrograph of HEK293T cells expressing the contrast generating unit MT3 fused to DHF119_sfGFP filaments (SEQ ID NO: 110). **e)** Two-component lattices (Di13b2 and Di13a, both published in Ben-Sasson et al., Nature (2021)) fused to GFP, which yield lattices as shown on the confocal fluorescent microscopy image. Scale bar is 20 µm. **f)** Corresponding TEM micrograph in which MT3 is fused as a contrast-generating unit (SEQ ID NO: 1).

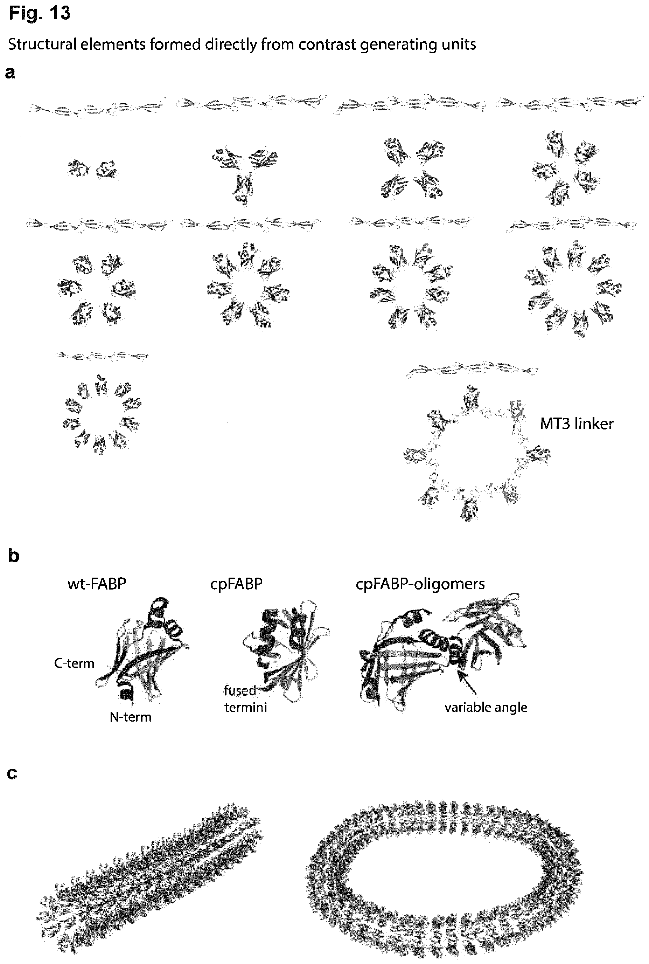

**Figure 13****: Concatenation of contrast generating units**
   The contrast generating units can also be concatenated via short linkers to generate nanometer-sized contrast-generating units. **a)** Shown here is the progression from a dimer, trimer, tetramer up to a decamer of FABP using its natural termini. One G-E-G repeat of SasG (^{~}16 nm) is shown as a reference for scale (top of each image). Repeating sequences on the nucleotide level can be avoided by exploiting sequence differences between the subtypes from the large FABP family. Individual FABP contrast-generating elements can also be connected via MT3, serving as a flexible linker and, in addition, as a contrast-generating unit. Exemplary sequences for these constructs are given in SEQ ID NOs: 194, 195. **b)** To increase the geometric diversity of the resulting structural elements, FABPs can be circularly permutated (cpFABP; SEQ ID NO: 193) to generate new termini between the alpha-helices. If those termini are fused between two cpFABPs to generate dimers, the angle can be controlled by the linker used for the fusion, **c)** Via side-by-side concatenation of FABPs, cylindrical shapes can be generated that can be closed to toroidal shapes, which can be stabilized via hydrophobic moieties and further be spatially organized via, *e.g.*, isopeptide bonds and inteins. An exemplary sequence for these constructs is given in SEQ ID NO: 196.

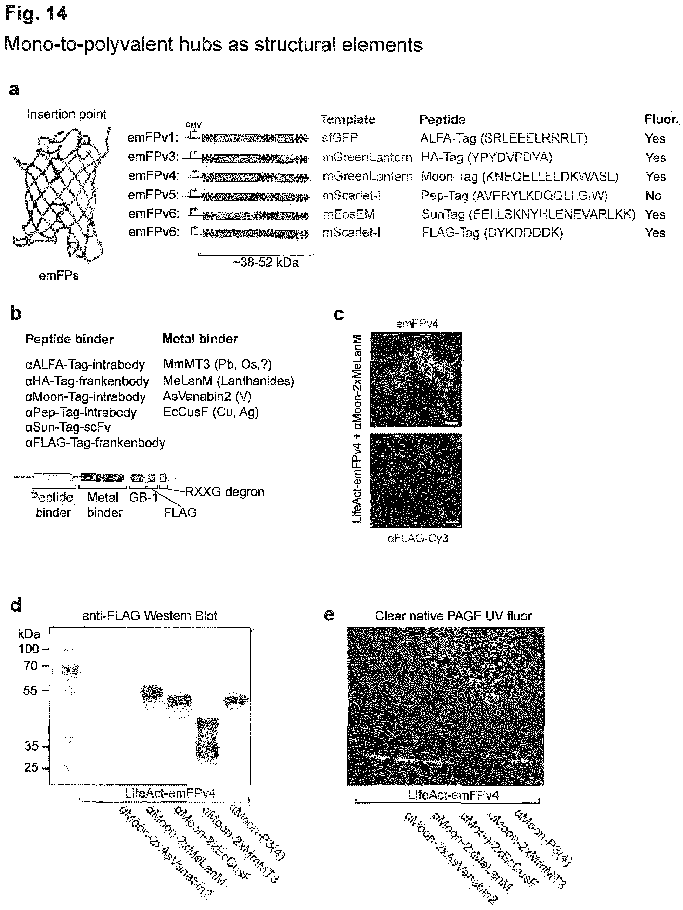

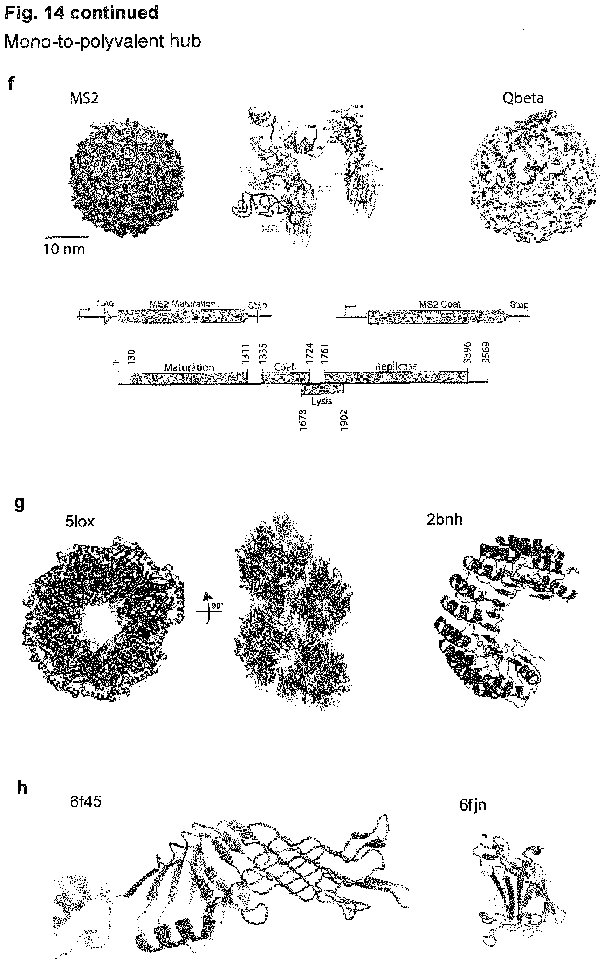

**Figure 14****: Mono-to-polyvalent hubs as structural elements**
   While the substantial size that can be obtained with self-assembling nanostructures is an advantage for building nanoscopy-readable structures, it comes with the natural disadvantage that the resulting structure is symmetric and multivalent. Monovalent hubs or adapters to which several contrast-generating units can bind are thus beneficial. **a)** (left) 3D image of an exemplary emFP (electron microscopy Fluorescent Protein) based on GFP showing the tag insertion points at the termini and an internal loop. (Right) Linear scheme showing the different emFP variants with the insertion points and a number of their respective intrabody-addressable tags. Exemplary sequences for these constructs are given in SEQ ID NOs: 92-99, 172-178. **b)** Schematic of the contrast unit binding to the emFPs consisting of intracellularly-expressable intrabody or scFv fragment, the respective metal interacting unit (or a tandem thereof), GB-1 (to enhance solubility), a FLAG-epitope for detection, and a C-terminal RxxG degron for background suppression. Exemplary sequences for these constructs are given in SEQ ID NOs: 172-178. **c)** Immunocytochemistry of HEK293 cells expressing LifeAct-emFPv4 (SEQ ID NO: 179) localizing to filamentous actin. The green signal (top) represents the emFPv4 (SEQ ID NO: 94) itself, whereas the red signal (bottom) corresponds to the FLAG-tag on the contrast unit, which colocalizes with the green signal indicating successful binding to the hub. **d)** Anti-FLAG western blot of whole-cell lysates from HEK293T cells co-expressing LifeAct-emFPv4 (SEQ ID NO: 179) plus the respective metal binders. The bands correspond to the expressed contrast units demonstrating their successful expression in mammalian cells. **e)** UV fluorescence clear native PAGE analysis of lysates from HEK293T cells co-expressing LifeAct emFPv4 and the respective contrast unit. The green signal at the bottom corresponds to non-bound emFPv4, whereas the higher molecular smears correspond to emFPv4 bound to several contrast units. **f)** Furthermore, a few selected viral capsids break the symmetry with portal, tails, or accessory proteins as known for picorna-, parvo-, micro-, and leviviridae. A specific example would be the maturation protein of MS2, Acinetobacter AP205 phages, or the Allolevivirus Qbeta phage. The maturation protein is a specific component in the MS2 capsid, which represents a unique single attachment point. Based on the detailed structural information, a monomeric attachment of, *e.g.,* MS2 capsids to *F-pilus* motifs can be designed via the maturation protein, while the MS2 RNA (or a subset thereof harboring the critical stem-loops) can provide EM contrast. The mammalian expression vectors are shown on the bottom (*see* also sequences of SEQ ID NOs: 100, 101, 102, 103). **g)** A preferred instantiation for engineered mono-to-polyvalent adapters or hubs is based on coiled-coil-rich proteins such as the Anbu protease (left) (SEQ ID NO: 88) or toroid protein (right) (SEQ ID NO: 89), in which the external coiled-coils can be replaced by designed coiled-coils that have high-affinity binding partners. PDB entry numbers are given in the figure. At the termini, all of these surface-exposed coiled-coils can be augmented by CCC-tags or Suntags. CCC-tags are a linear concatenation of coiled-coils. Suntag allows for recruiting up to 24 copies via a single-chain variable fragment (scFv) antibody to which a contrast-generating unit as described above can be attached. **h)** Other preferred examples of proteins with engineerable hypervariable loops that can be replaced with epitopes, coiled coils, or isopeptide-bonds are adhesins (left) (SEQ ID NOs: 89, 90) and fiber-knob domain proteins (right) (SEQ ID NO: 91). PDB entry numbers are given in the figure.
**Figure 15****: Programmable stoichiometry control using read-through motifs**
   **a)** Structural elements can be equipped with genetically controlled internal and external attachment points to bind contrasting units, target the structures to molecules of interest, or generate geometric patterns. Protein domains might be directly fused to the termini or an internal region of the structural element. Protein cargos might be internally docked via endogenous targeting signals such as encapsulation signals in the case of encapsulin and mINT signals in the case of vaults. Furthermore, attachment points in the form of nanobodies, CC domains, SpyTag or SpyCatcher domains, SNAP/Clip-Tag domains, and Halo-Tag domains might be fused. **b)** Since most structural elements are self-assembling and highly symmetrical, programmable translational read-through can be applied to regulate the stoichiometry of (surface) modifications. The schematic shows the genetic makeup of an exemplary programmable read-through (RT) cassette consisting of a QtEnc monomer gene with a C-terminal FLAG epitope followed by a stop codon and read-through promoting sequences (*e.g.*, SEQ ID NO: 180, including a CTATCC DNA sequence after the stop codon, which promotes ribosomal (translational) read-through). When ribosomal read-through occurs, the ribosome skips the stop codon and continues to synthesize the nascent polypeptide chain. By using different RT motifs, different degrees of read-through can be obtained, yielding encapsulin nanostructures that consist of monomers with a C-terminal FLAG epitope (immediate stop, no RT) or with a FLAG with an additional intrabody domain (stop skipped, read-through occurred). The rate of read-through is determined by the stop codon and the respective RT motif (Exemplary SEQ ID NO: 74-76). The RT rate is defined as the number of monomers with the additional intrabody, which arise from the RT divided by the total number of monomers. Another variant of the RT cassette uses a fast-cleaving intein mutant (SspDnaE; SEQ ID NO: 181), followed by a P2A peptide to yield two fully independent, unfused, scarless polypeptide chains since the middle intein module splices on the N-terminus and downstream polypeptide will be liberated via the P2A peptide (SEQ ID NO: 182). Therefore, the RT system can be used to co-express two unrelated polypeptides at different ratios depending on the RT motif used. **c)** Densitometric analysis of RT rates depending on different stop-codon RT motif combinations. The combination of stop codons with RT motifs allows for adjusting RT rates between ^{~}1 and 20%. Another way of quantifying the RT rate is based on the complementation of a split NanoLuciferase. If RT occurs, a HiBit epitope will be translated downstream of the intrabody sequence. After cell lysis, purified LgBit protein is added, which reconstitutes a functional luciferase yielding a luminescence signal serving as a proxy for RT.

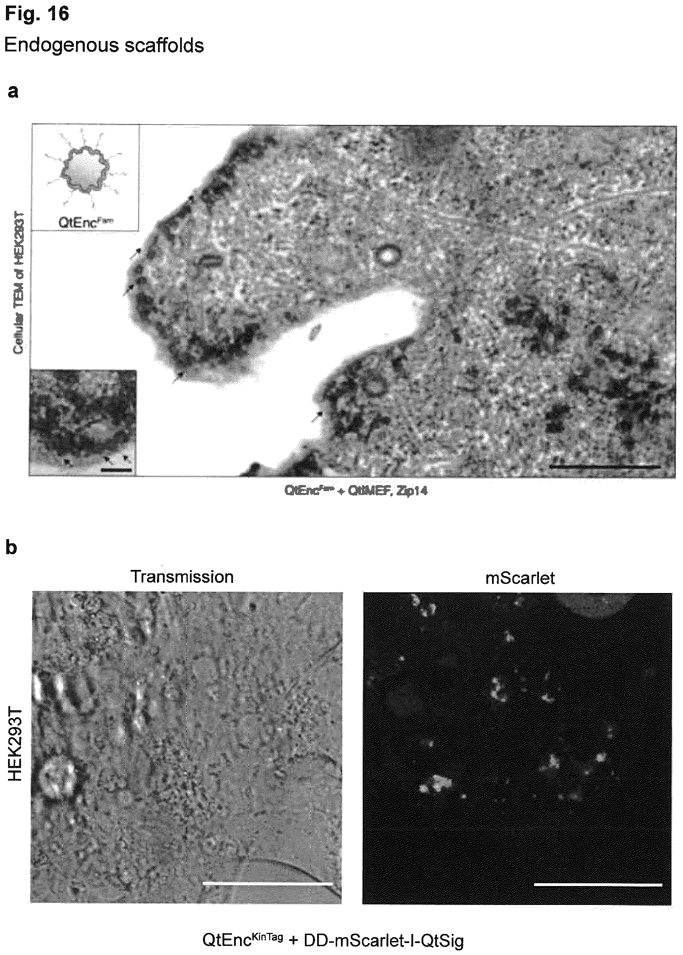

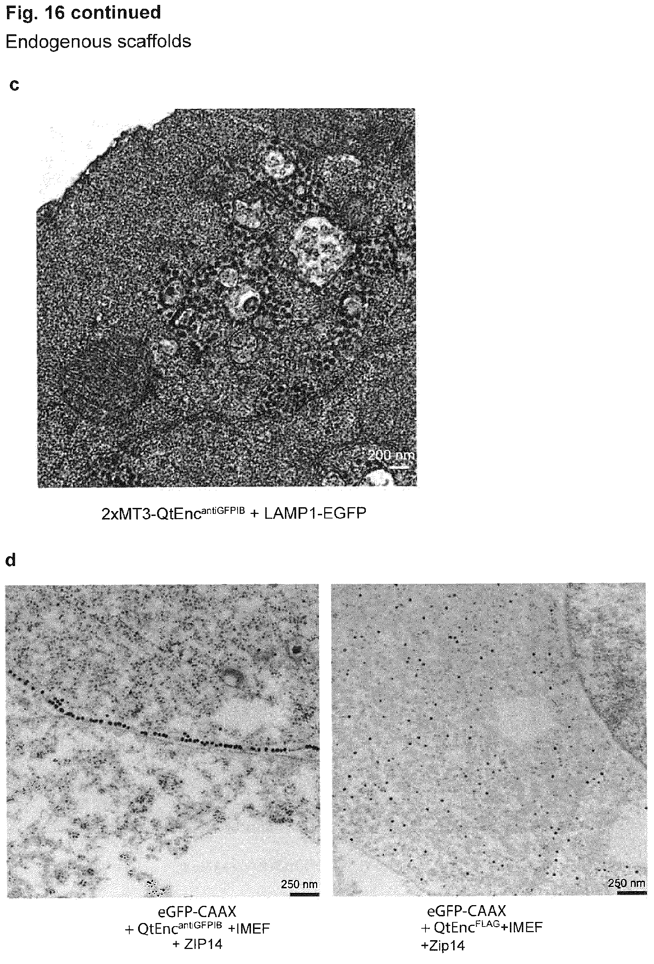

**Figure 16** **Endogenous scaffolds**
   **a)** Structural elements can be spatially patterned via naturally occurring cellular scaffolds. The TEM micrograph of HEK293T cells expressing QtEnc modified with C-terminal farnesylation signal (SEQ ID NO: 40) shows the QtEnc capsids targeted to cellular membranes. **b)** Structural elements can also be arranged on the cytoskeleton. The confocal fluorescence microscopy images show HEK293T cells expressing mScarlet-I-loaded QtEnc C-terminally modified with a KinTag peptide (Cross et al., Cell Chemical Biology (2021); SEQ ID NO: 113) mimicking a cargo adaptor connecting to endogenous motor proteins. The fluorescent signal represents the location of the encapsulins nano-compartments as structural elements within the cell, which can be seen as dotted signals in the perinuclear region and the periphery. **c)** TEM micrograph of HEK293T cells co-expressing 2xMT3-QtEnc^{antiGFPIB} (SEQ ID NO: 183) and the lysosomal marker LAMP1-GFP, which presents GFP on the outside of the lysosomes. The micrograph shows that highly contrasted QtEnc nanostructures co-localize with the lysosomes. **d)** In this example, the membrane is used as a scaffold via expressing membrane-localized GFP (GFP-CAAX) to which the anti-GFP intrabody on the surface of QtEnc binds to (left). In the control condition, GFP-CAAX is again expressed, but the intrabody is replaced with a FLAG tag (right). The contrast in both examples is provided by co-expression of the metal importer Zip14 and the ferroxidase IMEF targeted to the encapsulin via an encapsulation signal.

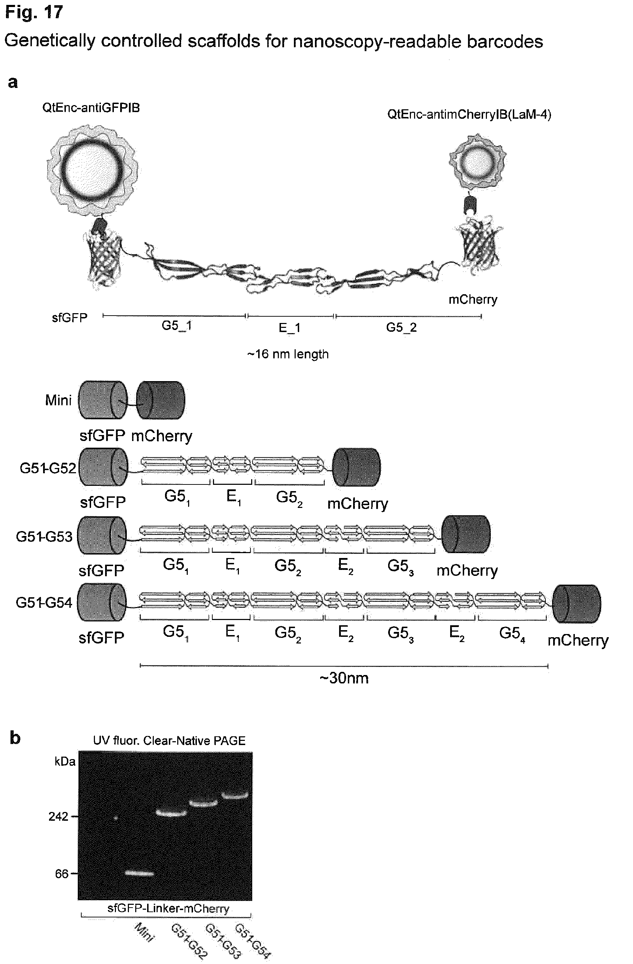

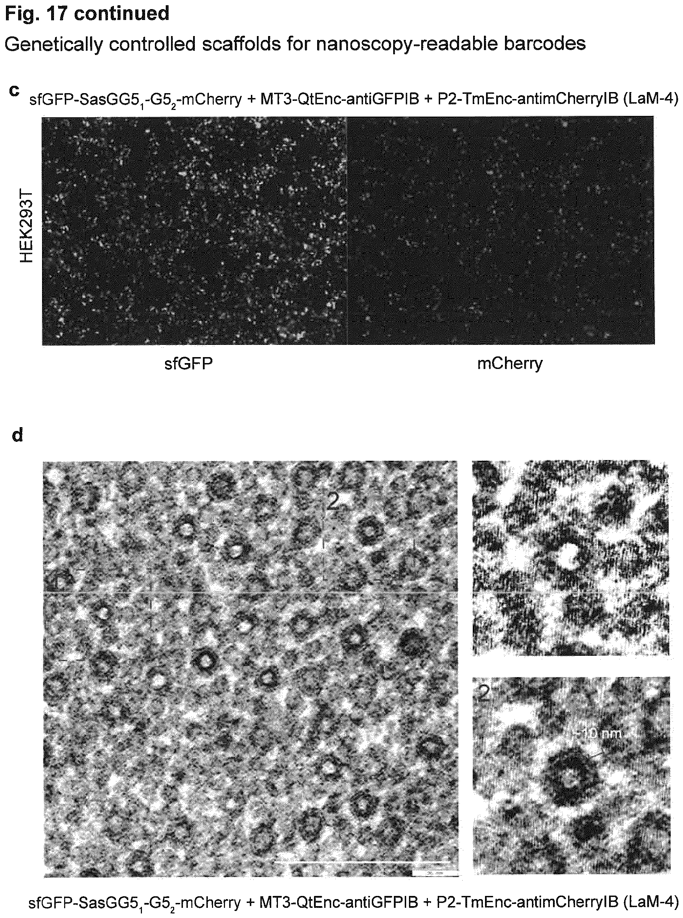

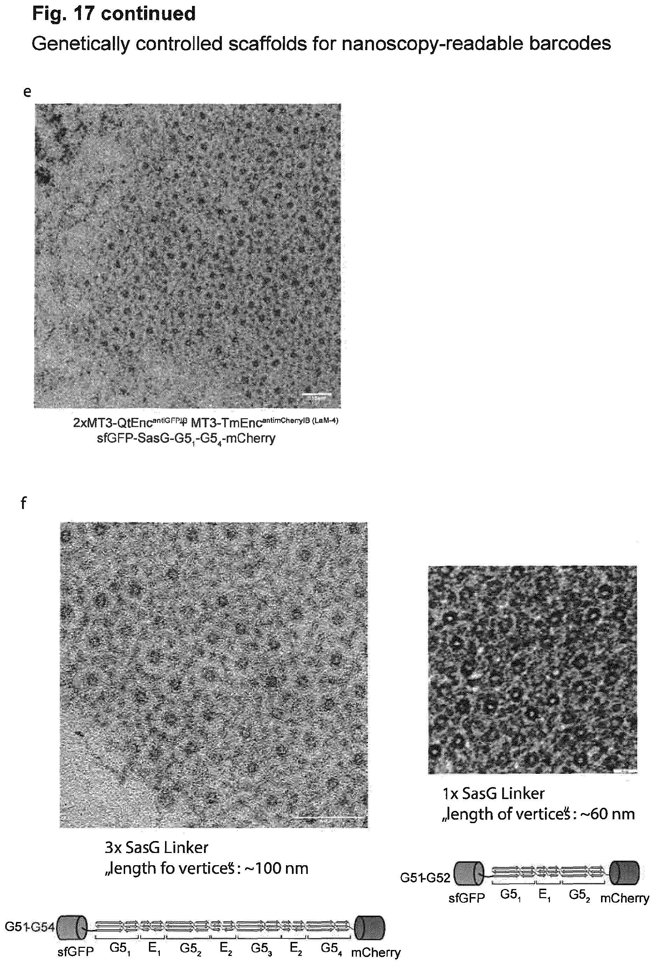

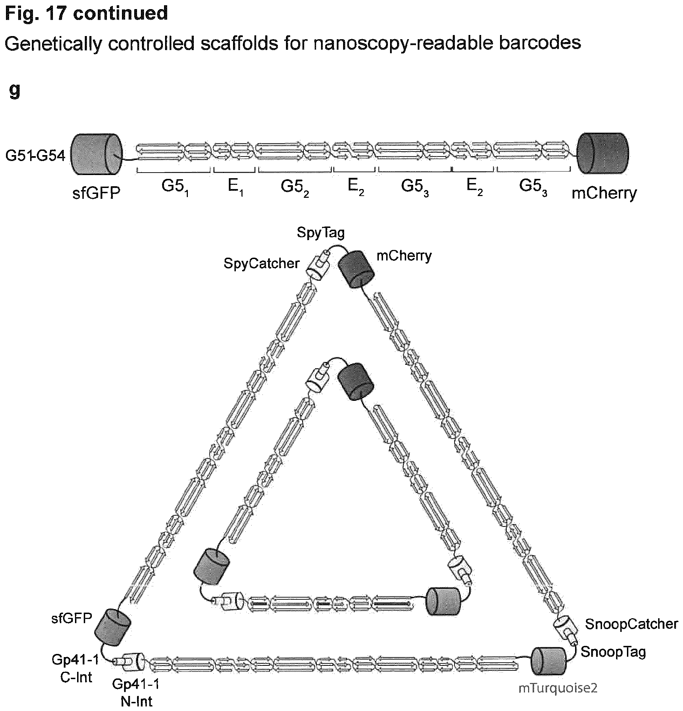

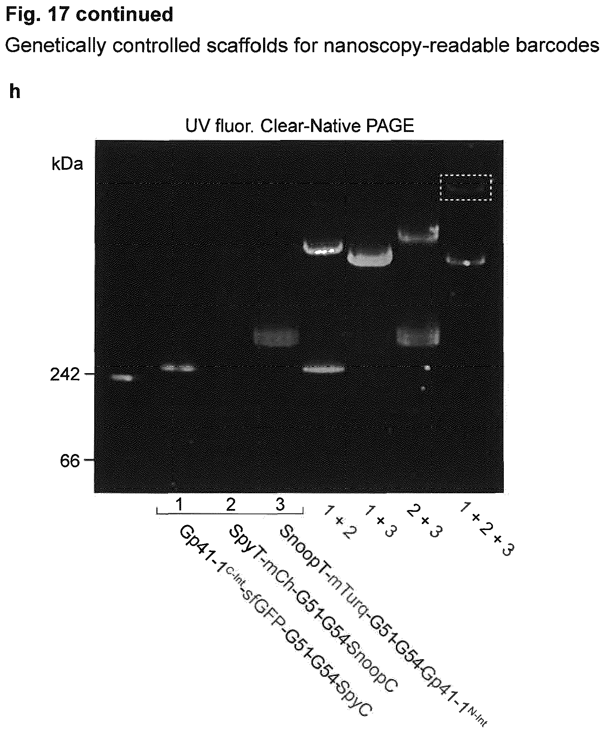

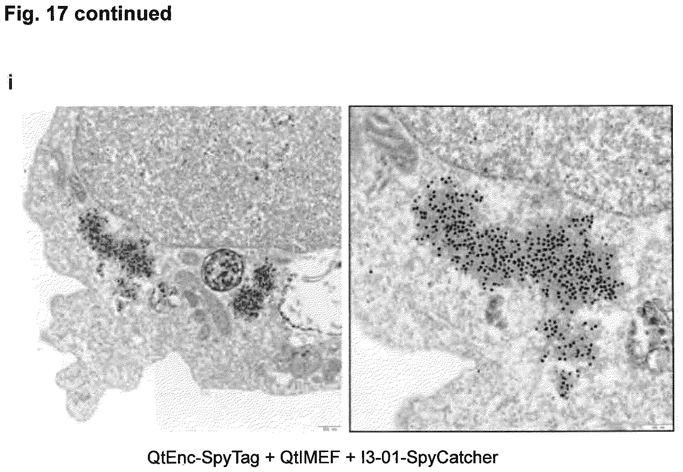

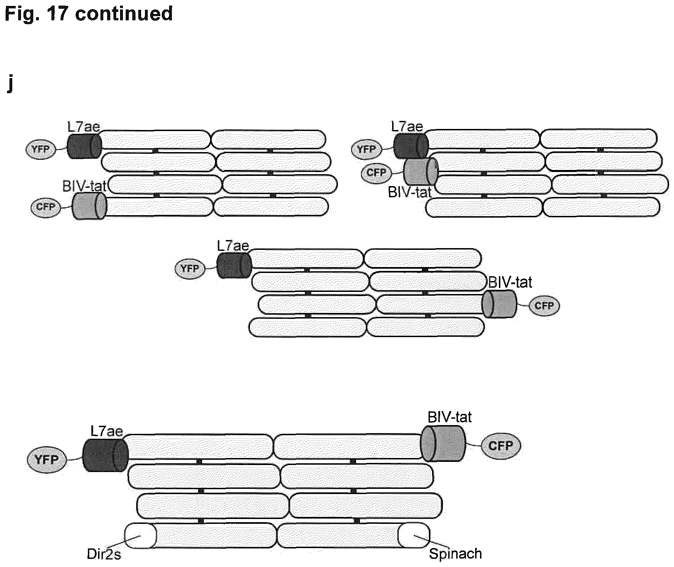

**Figure 17** **Genetically controlled scaffolds for barcodes readable by nanoscopy**
   **a)** (top) Schematic of the use of the microbial filamentous protein SasG, a rigid heterobifunctional scaffold from *S.aureus,* to enable nanoscopically visible shapes, preferably barcodes. Of note, SasG consists of a repeating pattern of G5 and E units. G5_1-G5_2 is an exemplary representation of the length, wherein G5_1 and G5_2 are connected via E(1). G5_1-E(1)-G5_2, for instance, yields 16 nm. G5_1-G5_4 means G5_1-E(1)G5_2-E(2)-G5_3-E(3)-G5_4 yields 30 nm. Exemplary sequences for these constructs are given in SEQ ID NOs: 114-123. The termini are fused to sfGFP (SEQ ID NO: 184) and mCherry (SEQ ID NO: 18, respectively, which can be addressed with structural elements, in this case, with QtEnc modified with an antiGFP intrabody and TmEnc with an anti-mCherry intrabody (LaM-4) (SEQ ID NOs: 186, 187). Of note, SasG is here used as a genetically controlled scaffold. (bottom) Schematic representation of modular make-up of SasG filaments consisting of alternating G5 and E units yielding different sizes. sfGFP-G51-G54-mCherry (SEQ ID NO: 188) yields a length of approximately 30 nm. **b)** Clear native PAGE analysis of lysates from HEK293T cells expressing different sfGFP-SasG-mCherry variants yielding different lengths. The CN-PAGE gel shows an sfGFP-mCherry tandem as well as different lengths of SasG as a spacer between sfGFP and mCherry. **c)** Live wide-field fluorescence microscopy imaging of HEK293T cells expressing sfGFP-SasG_G51-G52-mCherry + MT3-QtEnc^{antiGFPIB} + P2-TmEnc^{antimCherryIB} (LaM-4) (SEQ ID NOs: 114, 189, 109). The red and green fluorescent channels demonstrate the expression of the heterobifunctional scaffold. Clusters are visible, which indicates the interaction between the scaffold and the polyvalent encapsulins serving as structural elements. **d)** Corresponding TEM micrographs of HEK293T cells co-expressing sfGFP-SasG_G51-G52-mCherry + MT3-QtEnc^{antiGFPib} + P2-TmEnc^{antimCherryIB} (LaM-4). Clusters of 40 nm QtEnc structures surrounded by TmEnc capsules can be observed. The zoom-in shows the distinct spacing between the patterning of QtEnc and TmEnc, which can be used to encode multiplexed information in an analogy to a QR barcode. **e)** In distinction to d), a longer SasG with 4 G5 units (SasG-G51-G54) was co-expressed here. **f)** Here, clusters containing sfGFP-SasG_G51-G52-mCherry linkers (^{~}16 nm) and clusters containing sfGFP-SasG_G51-G54-mCherry (^{~}30 nm length) are juxtaposed for comparison. **g)** In addition to linear scaffolds, the rigidity of SasG also allows for more complex scaffolds. Depicted here is an example with triangular shapes in which isopeptide bonds (SpyTag/SpyCatcher, SnoopTag/SnoopCather) or inteins (SEQ ID NOs: 115-117) were used as unique connectors between the edges. The fluorescent proteins sfGFP, mCherry, and mTurquoise are also positioned at the edges to serve as binding sites for structural elements. **h)** CN-PAGE analysis of lysates from HEK293T cells expressing different combinations of the components shown in g Under fluorescence illumination, the CN-PAGE gel demonstrates the expected combination of individual elements (color-coded). The bio-orthogonal reaction of the components is apparent from the mass shifts in the gels and the color mixing (not apparent in a black/white copy of this figure). The last lane (1+2+3) shows a high molecular weight band (dashed box) corresponding to the assembled triangle. The fluorescent proteins positioned at the vertices can be replaced by emFPs (SEQ ID NOs: 92-97) or other hubs (*see*, Figure 15) or contrast generating units. **i)** Alternatively, unbounded clusters can be generated by linking together structural elements such as shown for encapsulins and multivalent *de novo* cages. The TEM micrograph shows HEK293T cells co-expressing iron-mineralizing QtEnc C-terminally modified with a SpyTag, which covalently clusters with I3-01 (Hsia et al., Nature (2016)), a *de novo* designed icosahedron, presenting 60 cognate SpyCatcher binding partners. **j)** As an alternative to the proteinaceous scaffold exemplified by SasG, RNA scaffolds can be designed via ROAD (Geary et al., Nat. Chem., (2021)) can be used but using L7ae and BIV-tat (SEQ ID NOs: 63, 64) instead of MCP and PCP as attachment points and incorporating fluorescent aptamers such as Spinach (SEQ ID NO: 55) and Dir2s (SEQ ID NO: 57).

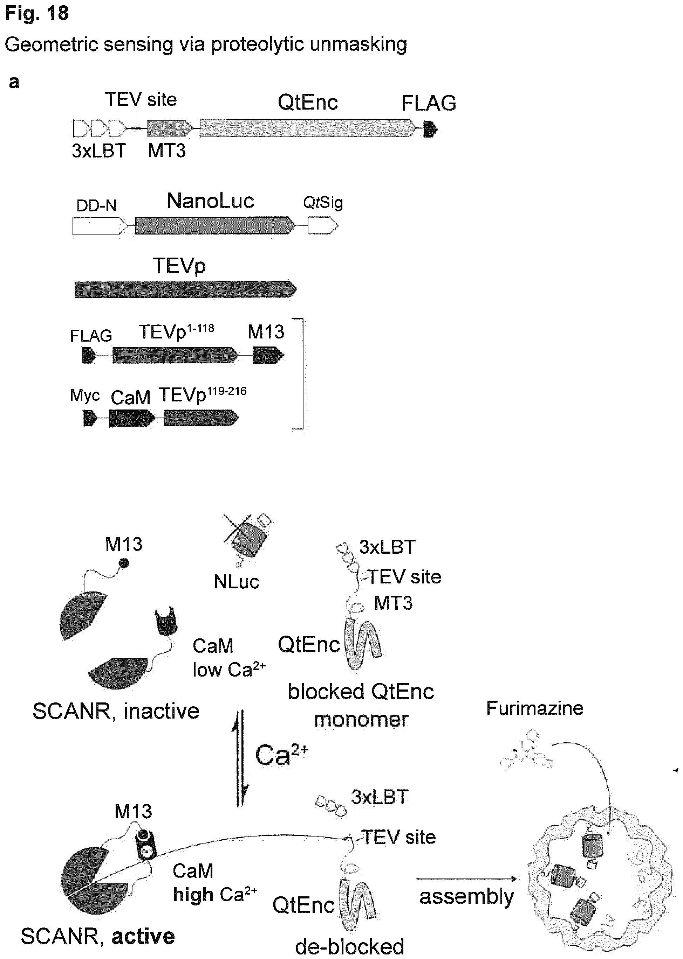

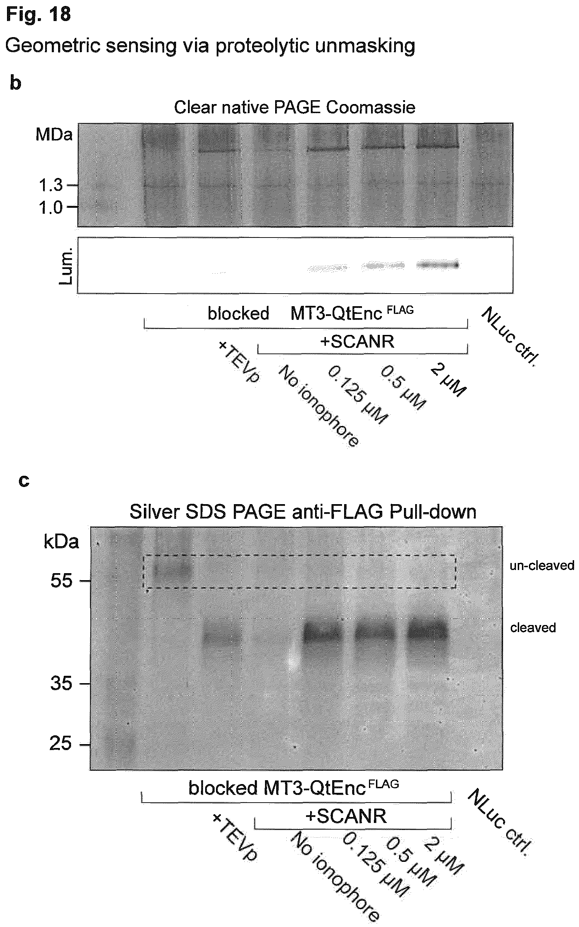

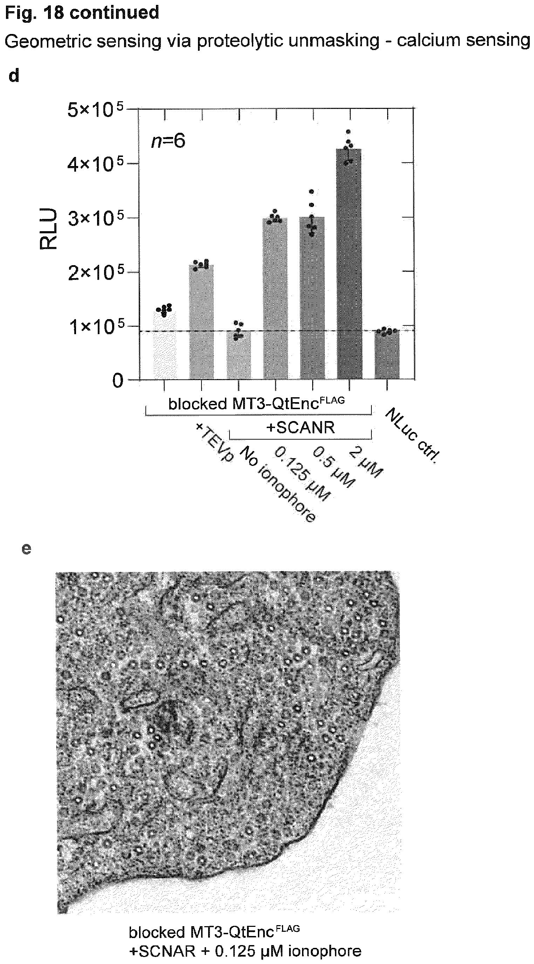

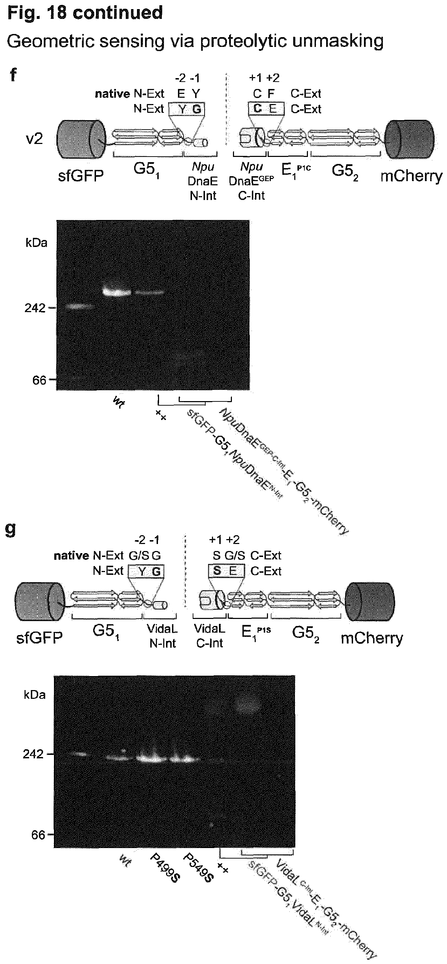

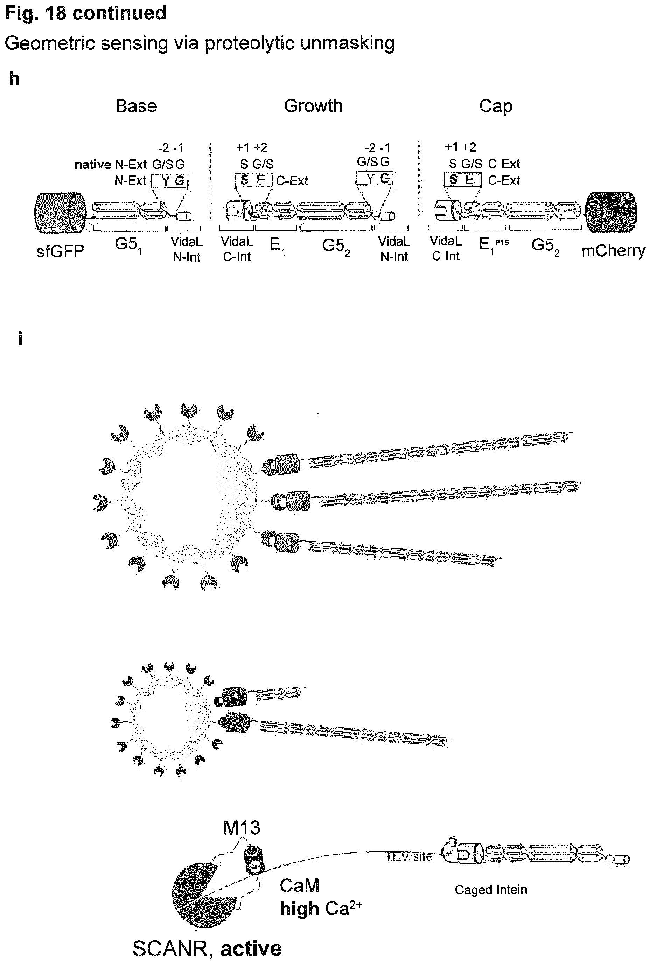

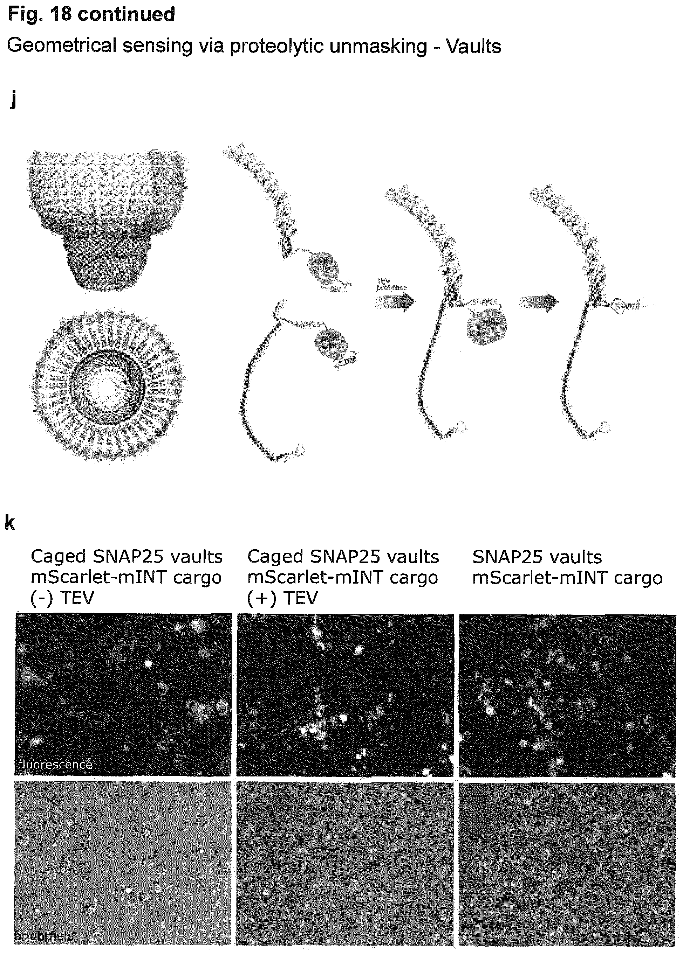

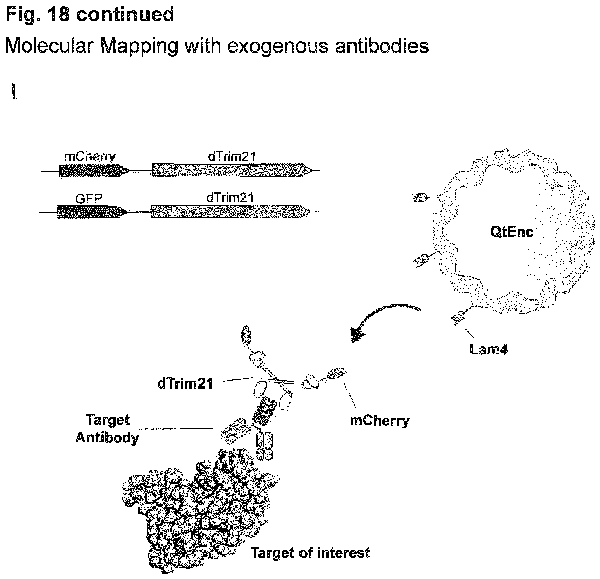

**Figure 18** **Geometric sensing via proteolytic unmasking and molecular mapping**
   Assembly of structural elements can be controlled via proteolytic de-blocking of monomeric subunits. **a)** Schematic of calcium-dependent proteolytic unmasking of sterically blocked QtEnc monomers. The scheme shows the different genetic components, including the blocked monomer with a TEV cleavage site (3xLBT15-TEVsite-QtEnc-FLAG; SEQ ID NO: 128), the two calcium-dependent Split TEV parts (SCANR; published in O'Neil et al, ACS Chemical Biology, (2018)), a degron-tagged NanoLuc-cargo; SEQ ID NOs: 129, 130), which serves as a reporter for assembly because it will be protected upon proteolytic de-blocking, and a regular TEV protease. Upon elevated Ca²⁺ levels, the SCANR parts will be reconstituted, the re-complemented TEV protease will cleave off the sterical 3xLBT block, the encapsulin nanocompartment will assemble and encapsulate the Luciferase cargo enzyme such that it is protected from degradation and give bioluminescent signal. **b)** In this example, QtEnc monomers are blocked with a triple LBT15 domain but can be unblocked via a bio-orthogonal protease such as TEV (TEVp), which leads to the assembly of the encapsulin nanospheres as shown by CN-PAGE. The protease may be expressed constitutively, under control of a promoter of interest, or can be made responsive to a second messenger such as calcium by using an engineered calcium-sensitive protease such as SCANR. Upon co-expression of SCANR, encapsulin assembly gradually increases with calcium, as can be seen by the increasingly stronger bands for the encapsulin on CN PAGE as well as an increasing luminescence signal obtained from the gel from a co-expressed NLuc cargo, which is destabilized unless it gets encapsulated inside the encapsulin shell. **c)** SDS-PAGE of material from a FLAG pull-down from cells expressing the conditions in b). The molecular weight shift indicates calcium-dependent cleavage of the blocking domain when either TEVp or SCANR + ionophore are present. **d)** Bulk luminescence reading of the experiment described in b). **e)** TEM micrograph of HEK293T cells expressing the blocked monomer + SCNAR 24 h post ionophore treatment. Assembled MT3-QtEnc^{FLAG} nanocompartments (structural elements) are visible throughout the cytosol. **f)** and **g)** SasG filaments can also be engineered to increase in length upon a cellular signal or state. This can be achieved by using engineered split intein domains, which can be fused to fragments of SasG to reconstitute SasG filaments of different lengths. In this example, sfGFP-SasG_G51 units are reconnected to SasG_E1_G52_mCherry units via different split intein systems (VidaL in f) and *Npu* DnaE in g); SEQ ID NOs: 121-123 and 118-120). The reconstitution of the fragments is indicated by the yellow band running above 242 kDa on CN-PAGE, which has the same electrophoretic mobility as the sfGFP_SasG-G51_E1_G52_mCherry construct. **h)** SasG filaments can also grow conditionally by using a self-growing EG fragment that has split-intein domains on either side of the building block (SEQ ID NOs: 120, 123). The growth can then be capped off by a capping unit (SEQ ID NOs: 119, 122). **i)** This conditional growth can be made directional by using QtEnc^{antiGFPIB} as a scaffold, in which a calcium-dependent TEV (SCANR) would un-cage a caged split-intein (Gramespacher *et al.,* 2017) and initiate growth. Upon a certain cellular signal of interest, the growth can be capped off by expressing the capping unit. **j)** Proteolytically triggered nanostructure assembly can also be realized with vaults. The schematic shows a split vault monomer, in which each half is blocked by split intein that can be activated via proteolytical cut by, *e.g.*, TEV, thus leading to the intein-mediated (supported by a coiled-coil motif) fusion of an intact vault monomer, exposing a SNAP25 motif. **k)** The fluorescence images show the TEV-dependent agglomeration of a fluorescent cargo (mScarlet docking via the mINT binding site) compared to the condition in which non-split vault monomers are used (right column). **I)** To complement molecular mapping via direct binding to the target biomolecule of interest, a deactivated version of Trim21 (dTrim21, SEQ ID NOs: 138, 139), incapable of auto-ubiquitinylation, can be co-delivered to cells (or expressed) together with standard antibodies to a target of interest, to act as an adapter to a structural element or scaffold. Shown here is a preferred instantiation, in which a fusion of mCherry or GFP to dTrim21 is used in combination with an encapsulin as a structural element harboring an anti-mCherry (Lam4) (SEQ ID NOs: 186, 187) or anti-GFP intrabody on the surface.

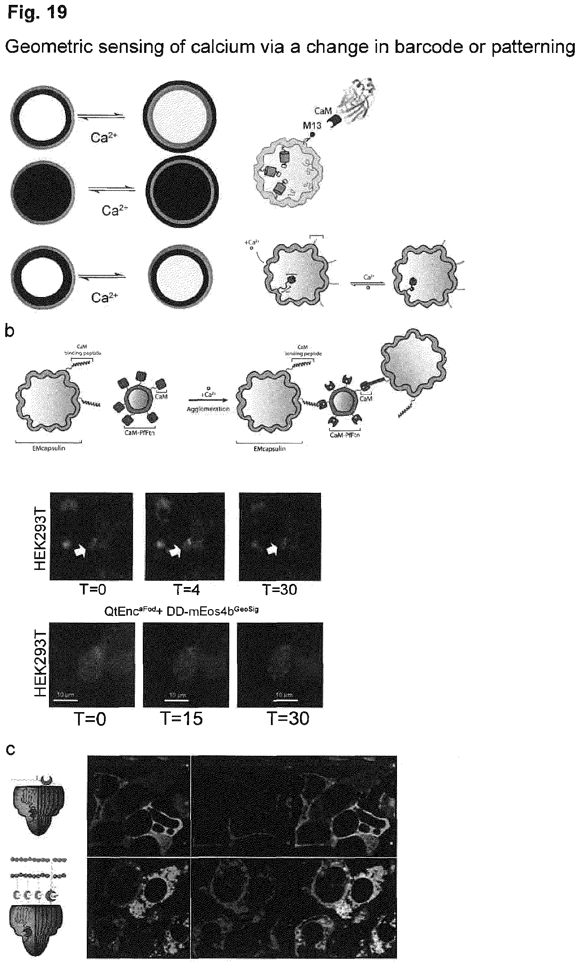

**Figure 19****: Geometric sensing via a change in the concentric barcode or patterning**
   **a)** Geometric sensing can be conducted via recruitment of contrast generating units to the surface of structural elements. Shown here is the specific case of FABPs recruited via the interaction of calmodulin and calmodulin-binding peptides to encapsulins (SEQ ID NOs: 197, 198), which alters the contrast contour of the concentric barcode. Please note that this mode is the same as in Figure 10 e) and f), except that the addition of the external concentric barcode is dependent on the calcium-driven binding of calmodulin to calmodulin-binding peptides. Bottom: In the encapsulin lumen, concentric barcodes can also be modified by using contractile elements such as engineered contractile elements from troponin that will shorten the distance to the protein shell upon binding of calcium (SEQ ID NOs: 136, 137). **b)** In another preferred embodiment, the agglomeration state of structural elements such as encapsulin can be changed via calcium-mediated interactions of calmodulin and calmodulin-binding peptides. As another polydentate kernel for agglomeration, ferritin such as Helicobacter pylori can be used as a fusion to calmodulin (SEQ ID NO: 134). The data show the formation of fluorescent clusters of encapsulins loaded with fluorescent cargo and equipped with respective calcium-binding motives after adding 10 µM calcium ionophore. An analogous system can be built for phosphorylatable peptides (*e.g.* SEQ ID NO 239) and phosphopetide-binding proteins such as SEQ-ID NO: 206. **c)** Alternatively, a calcium integrator by patterning can also be achieved by changing the subcellular localization of the nanostructure as a function of calcium concentration. Shown here is a preferred embodiment in which an N-myristoylation signal contained within recoverin (PDB: 1jsa; SEQ ID NO: 238) is fused to the N-terminus to enable calcium-dependent relocalization. As known for recoverin, this membrane anchor swings out in the presence of calcium, causing relocalization to membranes. As shown by the green fluorescent mEOS4b signal, the resultant protein complexes are homogeneously distributed in the cytosol (top) unless calcium influx is triggered, leading to the generation of clusters, similar to the constitutively available S-palmitoylation signal (bottom) (SEQ ID NO:209).

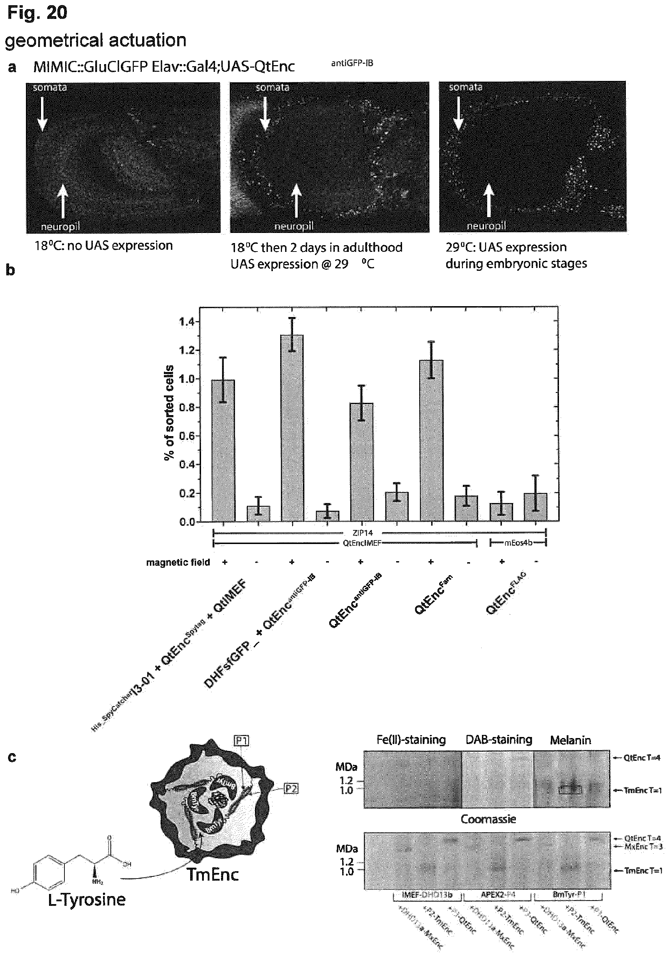

**Figure 20****: Geometrically precise actuation via biomechanical actuation and/or deposition of local energy**
   **a)** The genetically controlled structural elements and scaffolds can exert biomechanical forces by polyvalent interactions with their surfaces while providing monitoring of the manipulations via nanoscopy. Shown here is the clustering of endogenous glutamate receptors in *Drosophila* neurons via multivalent encapsulins surface-functionalized with intrabodies against GFP, which co-cluster GFP-tagged receptors. The control (left) shows the regular distribution of GluCI receptors labeled with GFP in *Drosophila* brains when encapsulins, surface-decorated with anti-GFP intrabodies, were not expressed via a temperature-sensitive promoter. When expression of MT3-QtEnc^{antiGFPintrabody} was permitted at 29°C (using the established system with a temperature-sensitive allele of GAL80) either after sparing early developmental phases (middle panel) or during development (right panel), mild or strong clustering of GluCI receptors in the somata was observed (McGuire et al, Science, (2003). Thanks to the controllable spatial arrangement of polyvalent binders by scaffolds such as SasG, more complex spatial manipulations can be systematically optimized via the nanoscopy readout. **b)** Geometrically defined actuation can also be achieved via local deposition of energy through magnetic gradient fields. Shown here is the magnetic cell sorting (MACS) of HEK293 cells overexpressing encapsulins with the ferroxidase QtEnc_IMEF encapsulated as a host enzyme (and the iron importer Zip14, or mEos4b as control), which leads to the biomineralization of biomagnetic iron-oxide species. In addition, the iron-oxide-filled encapsulins are surface-functionalized with different attachment points (SpyTag, anti-GFP intrabody) to spatially organized via different co-expressed scaffolds, including the *de novo* cage I3_01 functionalized with a SpyCatcher (*see* Figure 17i)), the filament DHF functionalized with GFP (see Figure 12c),d)). In addition, a farnesylation tag (*see* Figure 16a)) is used to target the encapsulins to the membrane. A FLAG tag serves as control. After expression in HEK293 cells (grown with ferrous ammonium sulfate), the cells were dissociated and passed over a commercial magnetic column (Miltenyi) placed either inside the magnetic field or outside as a control. The data (mean with SEM) show that magnetic gradients exert sufficient mechanical forces to retain entire cells that are expressing the geometrically organized magnetic and EM-visible geometric patterns. **c)** Similar to magnetic gradients, encapsulins can also be equipped with photoabsorbers such as biosynthetic pigments, which allow for energy deposition via visible or infrared electromagnetic radiation. Shown here as an example are encapsulins that have the enzyme tyrosinase encapsulated via coiled-coil pairs (P1/P2 in the case of TmEnc (SEQ ID NOs: 159, 190), other combinations are given in the figure), which generate the strongly absorbing polymer melanin from the endogenously available amino acid tyrosine. This polymerization reaction occurs inside the lumen of the nanocompartment, which prevents the product melanin from diffusing out, while the substrate tyrosine can diffuse into the lumen through the encapsulin pores. Spatially organized patterns of these photoabsorbing encapsulin species can be used to photoablate cellular structures. Monitoring can also be achieved via photoacoustic imaging, which picks up photoabsorption.

### DEFINITIONS

Unless otherwise defined, all technical and scientific terms used herein have the same meaning as commonly understood by one of ordinary skill in the art to which the present invention belongs.

The singular forms "a", "an", and "the" include references to the plural unless explicitly stated otherwise in the context.

The term "comprising" means "including" as well as "consisting", *e.g.*, a composition "comprising" X may consist exclusively of X or may include something additional, *e.g.*, X + Y.

**"Genetically controlled"** means that at least one element is a genetically encoded biomolecule, such as DNA, RNA, or protein, which may interact with non-genetically encoded molecules such as, for example, certain metal-interactors or metal-binders or substrates for enzymatic activity. Genetically controlled is therefore not limited to genetic material that has been artificially introduced into the cell but comprises endogenous genetic material expressed by the cell as well. Artificially introduced (generic) material is herein described as "exogenous" material.

**"Nanoscopy"** refers to imaging-based methods that resolve structures with nanometer spatial resolution, such as electron microscopy (EM), super-resolution-light microscopy (SRLM), or absorbance-based methods such as optoacoustics. Preferred are high-throughput nanoscopy methods that allow for volume nanoscopy of tissue.

**"Metal interactor"** is a molecule to which metal ions can bind to or with which they can react.

A **"metal-interacting peptide",** for instance, includes molecules, which can reversibly bind to metals or react with a metal. Osmium, for instance, reacts with osmiophilic residues. Metal-binding (metal chelation) may still occur. As an example, MT3 is osmiophilic but may still be able to bind to lead after osmium tetroxide treatment.

A **"metal-binding peptide",** for instance, a lead binder, or a lanthanide binder is a molecule that (reversibly) binds to (instead of reacts with) metals.

**"Exogenous"** means not genetically controlled from within the biological system, *e.g.*, a cell, but added to the biological system, *e.g.*, the cell, from the outside as a compound or material.

**"Genetically controlled nanoscopy contrast-generating unit"** refers to a genetically controlled molecule which alters the interaction of electrons with matter, *e.g.*, by the local enrichment of electron-dense elements, such as metals, for example, heavy-metals, such that contrast in electron microscopy is generated, and/or genetically encoded chromophores or fluorophores such that photons are absorbed and optionally emitted to generate a localized signal at the nanoscale, or a genetically controlled molecule that determines the local enrichment of a cofactor for a chromophore or fluorophore, or an exogenous chromophore or fluorophore, for the use in super-resolution light-microscopy methods (SRLM) or absorbance based super-resolution methods, *e.g.*, based on optoacoustics.

**"Fluorophore"** refers to any type of fluorescent molecule.

**"Chromophore"** refers to, for example, a protein binding a chromophore as a cofactor, such as biliverdin or phycocyanobilin, or an enzymatically generated polymer such as melanin. Chromophores such as biliverdin are also comprised as well as light-absorbing, light-refracting, light-scattering, or light-reflecting parts within a molecule, which are responsible for its color.

**"Genetically controlled structural element"** refers to a structural element with geometrically precise nanometer-sized features, that spatially organizes contrast-generating units to sufficiently large nanoscale size, such that their spatial distribution is discernable as a distinct geometric shape by nanoscopy, *e.g.*, EM or super-resolution-light-microscopy (SRLM).

**"Spatially organized"** means that the elements are arranged in a specific manner to create specific shapes or patterns.

A **"geometric shape"** is generated when genetically controlled nanoscopy contrast-generating units are spatially organized by a structural element.

A **"barcode"** or **"nanoscopy-readable barcode"** refers to a distinct spatial distribution of contrast, in particular via a series of contrast-rich and contrast-poor regions with defined spatial intervals, such as a series of high-contrast parallel bars with defined width and defined low-contrast intervals.

A **"concentric barcode"** refers to a series of contrast-rich or contrast-poor concentric spheres or rings. In nanoscopy, concentric barcodes can be read as the cross-section or intensity projection of certain spherical structural elements. A concentric barcode can be rotationally invariant and can intrinsically also encode directionality since it has a defined center and a surround such an inside-out or outside-in directionality can be defined.

**"Genetically controlled scaffold"** refers to a structure that can spatially organize structural elements to generate distinct geometric patterns discernable by nanoscopy.

A **"geometric pattern"** is a nanoscale pattern distinguishable in nanoscopy detection methods. Such geometric patterns are generated when genetically controlled structural elements are spatially organized by a scaffold.

An **"attachment point"** refers to a motif on a contrast-generating unit, a structural element, or a scaffold that enables specific binding between those elements or to a cellular structure or biomolecule of interest.

**"Geometric multiplexing"** refers to the parallel encoding of distinct pieces of information via multiple and distinct geometric shapes or patterns.

**"Molecular mapping"** refers to the nanoscopic visualization of the biodistribution of a molecule or molecules of interest with a genetically controlled structural element or scaffold.

**"Geometric sensing"** refers to the detection of an analyte or environmental parameter of interest (*e.g.*, temperature, pH) via a change of the geometric shape or geometric pattern.

Geometric sensing can also refer to the detection of multiple analytes or environmental parameters of the physiological or pathophysiological state of a cell, such as the response of a cell, ion flux in a cell, a response of the cell, a therapeutic response of the cell, an activation of the cell, or external stimulus to the cell, or combinations thereof.

**"Geometric actuation"** refers to the manipulation of cellular structures or processes by geometrically distinct patterns of biomolecules organized by structural elements and/or scaffold, which are also readable/visible by nanoscopy. The actuation may occur biomechanically, biochemically, and/or via deposition of external energy, *e.g.*, via electromagnetic radiation (*e.g.*, photoabsorption, or radiofrequency absorption), or mechanical energy (*e.g.*, ultrasound energy), and/or magnetic gradients.

**"Nanobiomaterial"** refers to a nanoscale biomaterial, which is produced in an isolatable form from a biological cell or a cell-free system and comprises nanoscale genetically controlled geometric features, based on structural elements, scaffolds, attachment points, and comprising biominerals, biopolymers, or enzymatic products for use in downstream applications. A nanobiomaterial can be used to, for example, treat, strengthen or substitute a part, such as a tissue, organ, or function of a living system. Alternatively, a part of a non-living system can be strengthened or substituted, such as, for example, a prosthesis.

The term **"vector"** as used in this disclosure comprises the nucleic acid encoding the genetically controlled nanoscale contrast-enhancing unit, the genetically controlled structural element, and/or the genetically controlled scaffold under control of a target cell-specific promoter. Such vector is suitable to transfer the nucleic acid encoding the genetically controlled nanoscale contrast-enhancing unit, the genetically controlled structural element and/or the genetically controlled scaffold to a target cell or target tissue. Such vector may be a viral vector, a plasmid, an agrobacterium, an exosome or a virus or virus-like particle, or any other vector known in the art and suitable for the transfection of a specific target cell.

As used herein, a **"eukaryotic cell",** organ or organism may be yeast, fungus, protozoa, plant, higher plant, and insect, or amphibian cells, organs or organisms, such as the brain of *Drosophila melanogaster,* or **"mammalian cells"** such as HEK293 cultured cells (*in vitro*)*,* graft cells and primary cell culture (*in vitro* and *ex vivo*)*,* and *in vivo* cells, and also mammalian cells including human, which are commonly used in the art, without limitation.

**"SEQ ID NO."** refers to the number of the respective sequence in the sequence listing. Comprised by this invention are also sequences having 80%, 90% or 95% sequence identity to the respective sequences given in the sequence listing.

### DETAILED DESCRIPTION

### Genetically controlled nanoscopy contrast-generating unit (CGU)

In one aspect, the invention relates to a genetically controlled nanoscopy contrast-generating unit comprising a metal interactor. The metal interactor is compatible with nanoscopy fixation protocols, nanoscopy post-fixation protocols, and nanoscopy metal staining protocols. Moreover, the metal interactor is a molecule to which metal ions can bind to or react with, wherein the metal interactor is not a ferroxidase and not an enzymatic product from an exogenous substrate, such as 3,3'-diaminobenzidine that is turned over by an enzyme such as HRP, APEX, or APEX2, or by a light-triggered reaction mediated by miniSOG variants to generate a polymer as a product with which heavy metals can interact with.

The nanoscopopy fixation protocols, nanoscopy post-fixation protocols, and nanoscopy metal staining protocols are preferably electron microscopy fixation protocols, electron microscopy post-fixation protocols and electron microscopy metal staining protocols, respectively. Thus, the genetically controlled nanoscopy contrast-generating unit is compatible with, for example, aldehydes such as formaldehyde, glutaraldehyde and mixtures thereof, which are commonly used in electron microscopy fixation protocols. The genetically controlled nanoscopy contrast-generating unit is also compatible with, for example, osmium tetroxide and tannic acid which are commonly used in electron microscopy post-fixation protocols. Furthermore, in preferred embodiments, the genetically controlled nanoscopy contrast-generating unit is also compatible with heavy metals, for example, uranium and lead which are commonly used in electron microscopy heavy metal staining protocols, as well as with lanthanides, which may be used as an alternative stain. In yet another embodiment, the genetically controlled nanoscopy contrast-generating unit mainly provides contrast by interaction with osmium tetroxide and partly by binding of a heavy-metal such as lead, after post-fixation.

Without being bound by theory, the inventors assume that osmium interferes with numerous metal-coordination sites. Thus, the genetically controlled nanoscopy contrast-generating units comprise metal interactors. Said metal-interactors interact with, for example, osmium tetroxide. It is assumed that the metal interactor provides contrast by osmiophilicity and metal binding, *inter alia.* For example, the metal interactor comprises one or more osmiophilic protein domains or peptides, thereby providing the desired contrast. Alternatively, a lipid-binding protein provides the desired contrast by interacting with, for example, osmium tetroxide. One example of a lipid-binding protein according to the invention is a fatty acid-binding protein. Thus, in one embodiment the metal interactor may react with osmium tetroxide such that osmium accumulates locally, whereby said locally accumulated osmium provides further interaction or binding sites for other metals. In another embodiment, the metal-interactor does not react with osmium tetroxide such that the metal-binding site provides further interaction or binding sites with heavy metals.

The metal-interactor comprised in the genetically controlled nanoscopy contrast-generating unit may comprise at least one metal-interacting peptide or a metal-interacting protein. In an embodiment, the metal-interacting peptide is a metallothionein, preferably MT3 or a series of different metallothionein species. In a preferred embodiment, the metal interactor is a metallothionein, for example a murine metallothionein. In a preferred embodiment, the metallothionein is MT3, even more preferred a eukaryotic MT3 domain, or preferably a mammalian MT3 domain. The MT3 domain may comprise one, two (tandem MT3), or three MT3 molecules. In a specific embodiment, the MT3 domain is a mouse MT3 domain, wherein the mouse MT3 domain is a single or tandem domain, in other words, comprises one (SEQ ID NO: 1) or two (SEQ ID NO: 4) mouse MT3s. In another specific embodiment, a series of three MT molecules are chosen from different species to avoid redundant sequences on the DNA level which interfere with genetic stability (*e.g*., SEQ ID NO: 5). An exemplary MT3 is MmMT3 having SEQ ID NO: 1. Exemplary sequence IDs for a series of MT3 molecules are SEQ ID NOs: 1-5.

In another embodiment, the metal-interacting protein may comprise a high abundance of osmiophilic amino acids. The metal interactor comprised in the genetically controlled nanoscopy contrast-generating unit may thus comprise at least one osmiophilic amino acid or one or more osmiophilic proteins, rich in cysteines, methionines, and/or tryptophanes. In a preferred embodiment, the osmiophilic amino acid/protein comprises one or more cysteine residue. An exemplary osmiophilic protein is MT3 (SEQ ID NO: 1), HRPII (*e.g*., SEQ ID NO: 17 fused to the encapsulation signal), or MMKPDM (SEQ ID 6). Alternatively, the metal-interacting peptide/protein may be selected from PDB entries 2ml7, 4nl8, 2jyt, 5lah, 3hcu.

The metal-interactor comprised in the genetically controlled nanoscopy contrast-generating unit may comprise at least one metal-binding peptide or metal-binding protein. In an embodiment, the metal-binding protein is a lead binder, such as MT3 having SEQ ID NO: 1. In an embodiment, the metal interactor is a lanthanide binder, such as lanmodulin (SEQ ID NO: 18). In another embodiment, the metal-binding protein is a uranyl binder, such as MUP2, or MUP5, having SEQ ID NOs: 45 and 46. In a preferred embodiment, the metal interactor is a super-uranyl-binding protein (SUP), a *de novo* designed protein having the sequence set forth in SEQ ID NO: 225. In another embodiment, the metal-binding protein is a vanadium binder, such as vanabin 2 (SEQ ID NO: 14). In another embodiment, the metal-binding protein is a copper and/or silver binder. In one embodiment, the metal interactor is one or more copper-binding peptide, such as CusF (SEQ ID NO: 15). In another embodiment, the metal interactor is one or more silver binding peptide, such as AG4 (SEQ ID NO: 199). In even another embodiment, the metal interactor is one or more copper and silver binding peptide, such as AG4 (SEQ ID NO:199).

Therefore, the difference between a metal-reacting and a metal-binding peptide/protein is that the metal-binding does not irreversibly modify the peptide/protein, *i.e*, it can also unbind/dissociate again.

The metal-interactor comprised in the genetically controlled nanoscopy contrast-generating unit may comprise at least one lipid-binding peptide or a lipid-binding protein. The bound lipid interacts with, for example, a heavy-metal stain. In an embodiment, such lipid-binding protein is a member of the family of fatty acid-binding proteins (FABPs). Given that lipids/fatty acids are a primary target for heavy metals, in particular, the EM post-fixative and contrast agent osmium tetroxide, the inventors identified fatty acid-binding proteins (FABPs) as a class of proteins that can generate genetically controlled and spatially confined EM contrast-generating units. FABPs are a family of proteins (liver (L-), intestinal (I-), heart (H-), adipocyte (A-), epidermal (E-), ileal (II-), brain (B-), myelin (M-) and testis (T-) that share very similar three-dimensional structures (PRINTS database pattern FATTYACIDBP; accession number PR00178), containing an antiparallel β-barrel structure with an internal binding site that holds the fatty acid in an interior cavity. Exemplary sequences are given in SEQ ID NOs: 30-39. Variants that preferentially bind polyunsaturated fatty acids, such as PDB-1fdq and PDB-2LKK (Fig. 3a), are preferred embodiments. In a preferred embodiment, the fatty acid-binding protein is PDB: 1fdq (SEQ ID NO: 36), a protein that binds polyunsaturated fatty acids, such as arachidonic acid, known to interact with osmium tetroxide. In another embodiment, such lipid-binding protein is the lipid-binding domain of a cytochrome P450, such as BM3. Preferably the lipid- and heme-binding domain of the cytochrome P450 BM3 (BM3h; SEQ ID NO:29) or variants thereof. Alternatively, the lipid-binding peptide/protein may be selected from the list comprising farnesylation, prenylation, and myristoylation motifs with exemplary SEQ ID NOs: 40-43.

The metal interactor of the genetically-controlled nanoscopy contrast-generating unit may comprise at least one RNA or a DNA molecule. Nucleic acids interact with heavy metals that are used in EM stains. The metal interactor of the genetically-controlled nanoscopy contrast-generating unit may comprise an RNA-binding protein bound to such RNA via an RNA aptamer. Preferably, the metal interactor is an RNA aptamer-binding protein bound to RNA which is modified with aptamer motifs. RNA aptamer binder: RNA aptamer pair interaction is preferably taken from viruses. The bound RNA interacts with the heavy-metal stain. In one embodiment, the RNA-binding protein is at least one RNA-binding protein, wherein the RNA-binding protein preferably binds RNA, which comprises an RNA aptamer. A pair of an RNA aptamer: RNA aptamer-binding protein is thus preferred. In a preferred embodiment, a pair of an RNA aptamer: RNA aptamer-binding protein is, for example, PP7: PCP (SEQ ID NO: 56 and 53, 54), MS2:MCP (SEQ ID NO: 152 and 100), H23:L7ae (SEQ ID NO:206 and 63), K-turn motif:L7kk (SEQ ID NO: 206 and 63). In another embodiment, the RNA-binding protein comprises a programmable RNA-binding protein, such as dCas13 (*e.g.,* the dCas13 of *Prevotella sp* , SEQ ID NO: 200), or PUF proteins/pumilios (e.g., human pumilio homology domain, SEQ ID NO: 201), or polyA-binding protein (Kuehn *et al,* Biochimica et Biophysica Acta (BBA, 2004). In a preferred embodiment, said bound RNA comprises a secondary structure, preferably a geometrically distinct secondary structure. In another embodiment, the RNA aptamer is selected from RNA aptamers that bind fluorophores, such as Spinach, Dir2s, and RhoBAST (SEQ ID NOs: 55, 57, 59). In another preferred embodiment, the RNA contains an engineered metal-binding with an exemplary sequence given in SEQ ID NO: 58.

The metal interactor comprised in the genetically controlled nanoscopy contrast-generating unit may comprise at least one polymerizing or synthesizing enzyme, by which a product is formed which in turn interacts with, for example, a heavy-metal stain. In one embodiment, the polymerizing or synthesizing enzyme comprises a kinase, a tyrosinase, a synthase, an RNA-dependent RNA polymerase transcriptase, an RNA-dependent DNA polymerase such as reverse transcriptase, a DNA polymerase, or a lipid synthetase. In a preferred embodiment, the polymerizing or synthesizing enzyme is a kinase or a tyrosinase. In one embodiment, the polymerizing enzyme may be a polymerizing kinase that can turn over, for example, GTP or ATP under the formation of highly negative-charged polyphosphate chains. In a preferred embodiment, the kinase is tetrameric polyphosphate kinase 2 (PPK2b), preferably having SEQ ID NO: 48. In a particularly preferred embodiment, the kinase is tetrameric polyphosphate kinase 2 (PPK2) of F. *tularensis* (PDB: 5LL0). In one embodiment, the synthesizing enzyme may be a tyrosinase. In a preferred embodiment, the tyrosinase is a bacterial tyrosinase that generates the strongly absorbing polymer melanin from the endogenously available amino acid tyrosine. Therefore, the tyrosinase may form melanin which may interact with, for example, a heavy-metal stain. In an even more preferred embodiment, the tyrosinase is a *Bacillus megaterium* tyrosinase (BmTyr), preferably having SEQ ID NO: 51. In yet another embodiment, the synthesizing enzyme is a phytochelatin synthase. In a preferred embodiment, the phytochelatin synthase is phytochelatin synthase 1 (AtPCS1), preferably having SEQ ID NO: 49.

The metal interactor comprised in the genetically controlled nanoscopy contrast-generating unit may comprise at least one biomineralizing enzyme. The resulting biomineral itself is electron-dense, or interacts with other electron-dense materials. In one embodiment, the biomineralizing enzyme comprises iron-oxide biominerals. In another embodiment, the biomineralizing enzyme comprises ferroxidase. In yet another embodiment, the biomineralizing enzyme is an iron-oxide biomineral such as a ferroxidase. In another embodiment, the biomineralizing enzyme is a ferroxidase from the Iron-Mineralizing Encapsulin-Associated Firmicute (IMEF, SEQ ID NO: 52) or Ferritin (PfFt, SEQ ID NO: 213). In another embodiment, the biomineralizing enzyme is complemented by the iron importer Zip14 (SEQ ID NO: 219) and the manganese importer DMT1 (SEQ ID NO: 218), such as to achieve doping of the produced iron oxide biomineral with manganese. In another embodiment, the enzyme is alkaline phosphatase, in a preferred embodiment not membrane-bound (SEQ ID NO: 221), which can lead to calcium-phosphate formation. In another embodiment, the enzyme is cystathionine-y-lyase with an exemplary sequence (SEQ ID NO: 220).

The metal interactor comprised in the genetically controlled nanoscopy contrast-generating unit may comprise at least one bioconjugation tag. Without being bound by theory, the bioconjugation tag provides contrast by conjugation to metal complexes and/or exogenous fluorophores or chromophores. In one embodiment, the bioconjugation tag is, for example, SpyTag/SnoopTag-Catcher, SNAP, Clip, Halo, coiled coils, or inteins. In a preferred embodiment, an exogenous compound binding and/or reacting with the bionconjugation tag is, for example, a chromophore and/or fluorophore or a tag-compatible metal-chelator such as macrocyclic compounds, i.e., a DOTA, DO3A, DOTAM, DTPA, NOTA, NODAGA derivative or a ring or linear polymers such as dendronized polymers. In another preferred embodiment, the bioconjugation tag is a pair of coiled coils. In another embodiment, the bioconjugation tags are coiled coils, and said coiled coils are utilized to generate super-resolution contrast by PAINT (points accumulation for imaging in nanoscale topography). In yet another embodiment, a low concentration of bioconjugation tags is used. A low concentration of bioconjugation tags depends on various parameters *inter alia,* such as the type of bioconjugation tag utilized and the type of genetically controlled contrast-enhancing unit, *inter alia.*

The metal interactor comprised in the genetically controlled nanoscopy contrast-generating unit may comprise a combination of at least two of said metal-interacting peptide/protein, metal-binding peptide/protein, lipid-binding protein, RNA-binding protein, RNA or DNA molecule, polymerizing or synthesizing enzyme, or bioconjugation tag. This means that all of the above-listed contrast-providing genetically controlled nanoscopy contrast-generating units can be combined.

Without being bound by theory, the metal-interactor provides contrast by osmiophilicity and metal-binding, *inter alia.* Furthermore, all of the contrast-generating units of the invention generate not only contrast in electron microscopy but may also be detected by fluorescence, bioluminescence, absorbance, magnetic measurements such as NMR, MRI, NanoSIMS, nitrogen-vacancy magnetometry, mass spectrometry imaging, and/or ultrasound. In one embodiment, the genetically controlled nanoscopy contrast-generating unit is detectable by both electron microscopy and fluorescence microscopy or optoacoustic microscopy techniques. In yet another embodiment, the genetically controlled nanoscopy contrast-generating unit is compatible with both electron microscopy and fluorescence microscopy techniques.

Therefore, in one preferred embodiment, the genetically controlled nanoscopy contrast-generating unit comprises a metal interactor, and further comprises a fluorophore or a chromophore. The fluorophore is, for example, a fluorescent protein, a co-factor in a fluorescent protein, or an exogenous fluorophore such as an Alexa dye or an indocyanine derivative. The fluorophore may also be a fluorophore-binding RNA aptamer. In one embodiment, the fluorescent protein is for example UnaG, preferable eUnaG (SEQ ID NO: 202), mGreenLantern *(e.g,* SEQ ID NO: 203), emFP *(e.g,* SEQ ID NO: 92-99), CaMPARI2 *(e.g,* SEQ ID NO: 204), mCherry *(e.g,* SEQ ID NO:185) or mRFP *(e.g,* SEQ ID NO: 205). Any other fluorescent protein known in the art and suitable for this purpose is encompassed in this embodiment. In another embodiment, the fluorophore-binding RNA aptamers are, for example, spinach (SEQ ID NO: 55) and/or DIR2s (SEQ ID NO: 57).

In one preferred embodiment, the genetically controlled nanoscopy contrast-generating unit comprises a metal interactor, wherein the metal interactor comprises at least one metallothionein, at least one fatty acid-binding protein, at least one lipid-binding domain or at least one RNA molecule and further comprises a fluorophore, wherein the fluorophore is a fluorescent protein.

In one embodiment, the metal interactor is an MT3 domain and the fluorophore is GreenLantern, CaMPARI2, mEos4b (SEQ ID NO:214 ), or mEosEM (SEQ ID NO:215); in other words, the genetically controlled nanoscopy contrast-generating unit is a GreenLantern-MT3 or a CaMPARI2-MT3, mEos4b-MT3, mEosEM-MT3 fusion protein. In another embodiment, the metal is an MT3 domain and the fluorophore is UnaG, preferably enhanced UnaG (eUnaG) (SEQ ID NO: 202); in other words, the genetically controlled nanoscopy contrast-generating unit is a eUnaG-MT3 fusion protein.

In another embodiment, the metal interactor is an RNA-binding protein bound to RNA via aptamers. The RNA aptamers may be spinach, which may further bind to exogenous fluorophores. Said RNA aptamers thus allow dual detection by both electron microscopy and fluorescence microscopy.

In another embodiment, the genetically controlled nanoscopy contrast-generating unit comprises a metal interactor and a chromophore. The chromophore may be a chromoprotein, a co-factor in a chromoprotein, or an exogenous chromophore such as a dark quencher. The chromoprotein is, for example, any fluorescent protein, a protein binding a chromophore as a co-factor, such as biliverdin (SEQ ID NO: 216) or phycocyanobilin (SEQ ID NO: 217), or an enzymatically generated polymer such as melanin, or light-absorbing, light-refracting, light-scattering, or light-reflecting parts within a molecule which are responsible for its color. For instance, the genetically controlled nanoscopy contrast-generating unit comprises a eUnaG-MT3 fusion protein, wherein eUnaG binds the endogenous ligand bilirubin.

Alternatively, the genetically controlled nanoscopy contrast-generating unit comprises a metal interactor and further comprises a bioluminescent protein, for example firefly luciferase, nanoluciferase, renilla luciferase, or AkaLuc.

The genetically controlled nanoscopy contrast-generating unit may comprise at least one attachment point. Said at least one attachment point is selected from the group consisting of nanobodies, frankenbodies, coiled-coil domains, isopeptide-forming partners such as SpyTag or SpyCatcher domains, SnoopTag/SnoopCatcher, DogTag/DogCatcher, bioconjugation tags such as SNAP/Clip-Tag domains, and/or Halo-Tag domains, split-inteins, calmodulin-binding peptides, phosphorylatable peptides, conformation-changing peptides such as troponins, as well as combinations thereof. In one embodiment, the genetically controlled nanoscopy contrast-generating unit comprises internal attachment points. Said internal attachment points are selected from the group consisting of nanobodies, frankenbodies, coiled-coil domains, isopeptide-forming partners such as SpyTag or SpyCatcher domains, SnoopTag/SnoopCatcher, DogTag/DogCatcher, bioconjugation tags such as SNAP/Clip-Tag domains, and/or Halo-Tag domains, split-inteins, calmodulin-binding peptides, phosphorylatable peptides, conformation-changing peptides such as troponins, as well as combinations thereof. In another embodiment, the genetically controlled nanoscopy contrast-generating unit comprises external attachment points. Said external attachment points are selected from the group consisting of nanobodies, frankenbodies, coiled-coil domains, isopeptide-forming partners such as SpyTag or SpyCatcher domains, SnoopTag/SnoopCatcher, DogTag/DogCatcher, bioconjugation tags such as SNAP/Clip-Tag domains, and/or Halo-Tag domains, split-inteins, calmodulin-binding peptides, phosphorylatable peptides, conformation-changing peptides such as troponins (Thestrup et al., Nature Methods,2014), as well as combinations thereof. In yet another embodiment, the genetically controlled nanoscopy contrast-generating unit comprises internal attachment points and external attachment points. Said internal and external attachment points are selected from the group consisting of nanobodies, frankenbodies, coiled-coil domains, isopeptide-forming partners such as SpyTag or SpyCatcher domains, SnoopTag/SnoopCatcher, DogTag/DogCatcher, bioconjugation tags such as SNAP/Clip-Tag domains, and/or Halo-Tag domains, split-inteins, calmodulin-binding peptides, phosphorylatable peptides, conformation-changing peptides such as troponins, as well as combinations thereof.

In a preferred embodiment, the genetically controlled nanoscopy contrast-generating unit comprises MT3, FABP, or BM3h as the metal interactor as either direct fusions or via attachment points chosen from coiled-coils, isopeptide forming pairs or inteins, wherein via the attachment point the CGU can be attached to structural elements such as encapsulins, vaults, emFP, or SasG.

### Genetically controlled structural elements (SEs)

In one aspect, the invention relates to a genetically controlled structural element (SE). The genetically controlled structural element comprises and spatially organizes at least one genetically controlled nanoscopy contrast-enhancing unit. The genetically controlled structural element interacts or binds to the genetically controlled nanoscopy contrast-enhancing unit and therefore, organizes the genetically controlled nanoscopy contrast-enhancing unit. In general, the genetically controlled structural element has to be of a sufficient size that is detectable by, for example, in high-throughput EM using, *e.g.**,*** a pixel size of 1 to 4 nanometers. The genetically controlled structural element possesses a pre-determined shape and pre-determined nanoscale size and generates a shape that can be differentiated at the nanoscale, *i.e.**,*** it can be detected and distinguished from shapes of other SEs or other (cell) structures in EM and other nanoscale detection methods. Such a distinct shape is preferably a barcode, even more preferably a concentric barcode.

The distinct shape is a function of the 3D distribution of the genetically controlled nanoscopy contrast-generating units within the genetically controlled structural element. In other words, the geometrically distinct shape is generated by the interaction of the structurally organized genetically controlled nanoscopy contrast-generating units and the genetically controlled structural element. In standard EM methods, these 3D distributions are commonly detected or presented as 2D cross-sections or projections onto a plane.

If such a shape generates a distinct spatial distribution of contrast, in particular, a series of contrast-rich and contrast-poor regions with defined spatial intervals, such as a series of high-contrast parallel bars with defined width and defined low-contrast intervals, the shape is called a "barcode". In a preferred embodiment, the distinct shapes are concentric rings, *i.e.**,*** an annular shape, which in the limit converges to a disc shape. In one embodiment, the distinct shape is, for example, a ring, a disc, a rectangle, a triangle etc..

In another preferred embodiment, concatenated MT3 domains fused to encapsulin monomers produce an annular or disc shape. In one embodiment, single or tandem MT3 domains are fused to the N-terminus of QtEnc. In another embodiment, tandem MT3 domains provide twice the number of osmiophilic residues which reach further into the lumen of the encapsulin. More contrast is generated, and thus the diameter of the "inner circle" of the ring shape decreases, resulting in a disc. If three MT3 domains are fused to an encapsulin, the shape is a completely "dark" disc (see, **Figure 10b****).** In one embodiment, MT3-QtEnc^{FLAG} is a ring. In other words, one MT3 is fused to the interior of an encapsulin, resulting in a ring shape. In said embodiment, the ring is an annular electron-dense ring with a bright center. In another embodiment, 2xMT3-QtEnc^{FLAG} is a disc. In other words, two MT3s are fused to the interior of an encapsulin, resulting in a disc shape. Said disc has a darker appearance than the MT3-QtEnc^{FLAG} ring. In an alternative embodiment, 3×MT3-QtEnc^{FLAG} adds another layer of contrast to the lumen, which appears as a disc. In other words, three MTs from different species (3xchimericMT, SEQ ID NO: 169) are fused to the interior of an encapsulin, resulting in a disc shape. All three shapes are called "concentric barcodes," which can be complemented by additional shapes generated by adding more layers of contrast to the outer surface of the spherical nanostructure **(****Figure 10e****,f).**

The genetically controlled structural element is preferably a self-assembling structure. A self-assembling structure has the advantage of consisting of only a few building blocks while having the disadvantage of being polyvalent, thus making it challenging to include sparse attachment points that can be used to bind to a target of interest or attach to a scaffold to generate a precise geometric pattern. In one embodiment, the genetically controlled structural element is not self-assembling. In another embodiment, the genetically controlled structural element is self-assembling. In one embodiment, the genetically controlled structural element is not symmetrical. In another embodiment, the genetically controlled structural element is symmetrical. In one embodiment, the genetically controlled structural element is not self-assembling and not symmetrical. In another embodiment, the genetically controlled structural element is self-assembling and symmetrical. An encapsulin is an example of a genetically controlled structural element, which is self-assembling, symmetrical, and spherical. A vault is an example of a genetically controlled structural element that is self-assembling and symmetrical in one axis but is not a sphere. Obtaining a low valency, let alone a single valency on the outer surface, is very challenging if a self-assembling and symmetrical structure is used. A low valency is preferred such that cross-linking of, for example, the target is avoided.

A solution to the problem is provided by genetically controlling the stoichiometry of self-assembling building blocks with or without distinct attachment points. This objective can be achieved via changing the concentration of plasmids or viruses encoding the respective genetically encoded building blocks in transient transfection or transduction systems. For a stable genetic modification, a solution is provided by a translational read-through system (Figure 15), which uses several variants of termination codons to regulate the probability of ribosomes passing through the termination codon to prolong the translated peptide, *e.g.* by a terminal attachment point.

In one embodiment, the stoichiometry of surface modifications of the self-assembling and symmetrical genetically controlled structural element can thus be regulated. In one embodiment, said regulation takes place by programmable translational read-through. In a preferred embodiment, programmable translational read-through entails a programmable RT cassette comprising a structural element with a stop codon and a read-through promoting sequences. In another preferred embodiment, the programmable RT cassettes comprise a QtEnc monomer gene with a C-terminal FLAG epitope followed by a stop codon and read-through promoting sequences. In one embodiment, the programmable translational read-through is determined by the stop codon and the respective read-through motif. In one embodiment, the stop codon is selected from the group consisting of UAG, UAA, and UGA. In a preferred embodiment, the stop codon is TGA. In one embodiment, the read-through motif is selected from the group consisting of 3 SEQ ID NOs: 74-76. In one embodiment, the combination of the stop codon and read-through motif allows for adjusting read-through rates between about 1 and 20 %.

In an embodiment, the genetically controlled structural element is self-assembling, symmetrical, and rotationally invariant. A member of the encapsulin family is an example of a genetically controlled structural element that is self-assembling, symmetrical, and rotationally invariant. Taking the example of encapsulins, a maximum number of internal attachment points is desirable to maximize the density of contrast-generating units.

In one embodiment, the genetically controlled structural element comprises a member of the encapsulin family, a vault, an MS2 phage, a Qbeta phage, an AP205 phage, an engineered mono-to-polyvalent hub, a filament, or a linker, or combinations or variants thereof.

The genetically controlled structural element may comprise a member of the encapsulin family. In a preferred embodiment, the genetically controlled structural element is an encapsulin, preferably from the species *Q*. *thermotolerans* (QtEnc; T=4, 40 nm in diameter), *M. xanthus* (MxEnc; T=3, 32 nm in diameter), and *T. maritima* (TmEnc; T=1, 20 nm in diameter). In another embodiment, the encapsulin has a triangulation number of four, three, or one. In even another embodiment, the encapsulin has a size of between about 20 to 45 nm, about 35 to 45 nm, about 27 to 37 nm, or about 15 to about 25 nm in diameter. In a preferred embodiment, the encapsulin has a size of about 40 nm, 30 nm or 20 nm in diameter. In another preferred embodiment, the encapsulin has a triangulation number of four and a size of about 40 nm, a triangulation number of three and a size of about 30 nm, a triangulation number of 1, and a size of about 20 nm all in diameter.

In another embodiment, the genetically controlled structural element comprises an encapsulin and at least one genetically controlled nanoscopy contrast-generating unit comprising a metal interactor wherein the encapsulin spatially organizes the genetically controlled nanoscopy contrast-generating unit. In another embodiment, the genetically controlled structural element organizing the genetically controlled nanoscopy contrast-generating unit is a fusion protein. In a preferred embodiment, the genetically controlled structural element organizing the genetically controlled nanoscopy contrast-generating unit is a fusion protein comprising an encapsulin and at least one metal interactor. In a preferred embodiment, the at least one metal interactor is a metallothionein, preferably MT3. In another preferred embodiment, the encapsulin is fused with a tandem MT3 domain, in other words, comprises two MT3 domains. In one embodiment, an encapsulin is N-terminally fused to a tandem mouse MT3 domain. In another preferred embodiment, the fusion protein is MT3-QtEnc, MT3-MxEnc, or MT3-TmEnc. In yet another preferred embodiment, the fusion protein is MT3-QtEnc-FLAG, MT3-MxEnc-FLAG, or MT3-TmEnc-BC2Tag.

In another preferred embodiment, the genetically controlled structural element is an encapsulin, and the at least one metal interactor is RNA.

In yet another preferred embodiment, the genetically controlled structural element is an encapsulin, and the at least one metal interactor is an RNA-binding protein.

In even another preferred embodiment, the genetically controlled structural element is an encapsulin, and the at least one metal interactor is a lipid-binding protein, preferably a fatty-acid binding protein.

In one embodiment, the genetically controlled structural element is an encapsulin, the at least one metal interactor is RNA and the genetically controlled nanoscopy contrast-generating unit additionally comprises a kinase, IMEF, with co-expression of ZIP14, DMT1, or a tyrosinase.

In yet another preferred embodiment, the genetically controlled structural element is an encapsulin, the at least one metal interactor is an RNA-binding protein and the genetically controlled nanoscopy contrast-generating unit additionally comprises a kinase, IMEF, or a tyrosinase.

In even another preferred embodiment, the genetically controlled structural element is an encapsulin, the at least one metal interactor is a lipid-binding protein, preferably a fatty-acid binding protein and the genetically controlled nanoscopy contrast-generating unit additionally comprises a kinase, IMEF, or a tyrosinase.

In a preferred embodiment, the genetically controlled nanoscopy contrast-generating unit is the synthetic enzyme tyrosinase and the structural element is an encapsulin comprising the tyrosinase. Melanin is generated in the encapsulin lumen by the tyrosinase (an exemplary tyrosinase is BmTyr having SEQ ID NO: 51). In one embodiment, melanin binds to heavy metals. In another embodiment, the melanin is contrasted without binding to heavy metals in EM.

In another embodiment, the genetically controlled nanoscopy contrast-generating unit is the polymerizing enzyme kinase and the structural element is an encapsulin comprising the kinase, preferably PPK2. ATP or GTP enters the genetically controlled structural element via its pores, where it is polymerized into a polyphosphate chain trapped inside the encapsulin lumen. The result is highly contrasted polyphosphate pores. In this particular embodiment, the resulting structures are from about 25 to 33 nm, preferably about 32 nm, and comprise MxEnc^{FLAG} + DD-CgPPK2b-QtSig (SEQ ID NOs: 107, 47).

In another embodiment, the encapsulin comprises mineralized iron oxide via its native IMEF cargo protein. In yet another embodiment, an IMEF dimer is docked into the encapsulin shell, where ferrous ion enters through the pore in the encapsulin shell and is subsequently bio-mineralized. The resulting encapsulin is, for example, QtEnc + QtIMEF, which is a spherical structure of about 40 nm.

In another embodiment, the genetically controlled structural element organizing the genetically controlled nanoscopy contrast-generating unit is a fusion protein comprising an encapsulin, at least one metal interactor, and a fluorophore. In a preferred embodiment, the fusion protein is eUnaG-MT3-QtEnc (SEQ ID NO: 155). In a particularly preferred embodiment, the fusion protein is eUnaG-MT3-QtEnc or eUnaG-2xMT3-QtEnc. In one embodiment, eUnaG-MT3-QtEnc generates concentric barcodes of about 35-45 nm in diameter, preferably about 40 nm. In one embodiment, said spherical structures harbor a distinct lumenal phenotype showing a contrasted internal ring as well as a bright center spot. In one embodiment, eUnaG-MT3 is localized to the encapsulin lumen. In yet another embodiment, the fusion protein is eUnaG-2xMT3-QtEnc, and the lumen is homogeneously contrasted.

In one embodiment, UnaG-MT3-QtEnc is localized to the cytosol. In another embodiment, UnaG-2xMT3-QtEnc is localized to the cytosol.

In yet another embodiment, the fusion protein is MT3-MxEnc^{FLAG}-cMyc-NLS. In one embodiment, MT3-MxEnc-FLAG-cMyc-NLS is expressed pan-neuronally in *Drosophila melanogaster.* In yet another embodiment, MT3-MxEnc^{FLAG}-cMyc-NLS is expressed pan-neuronally in the optical lobe in *Drosophila melanogaster.*

In a preferred embodiment, MmMT3-QtEnc is from about 35 to 45 nm in diameter, preferably about 39.24 ± 3.51 nm, most preferably about 39 nm. In one embodiment, MmMT3-MxEnc is from about 25 to 35 nm in diameter, preferably about 29.94 ± 2.61 nm, most preferably about 30 nm. In one embodiment, MmMT3-TmEnc is from about 15 to 25 nm in diameter, preferably about 21.2 ± 2.54 nm, most preferably about 21 nm.

In a specifically preferred embodiment, the genetically controlled structural element is an encapsulin and the genetically controlled nanoscopy contrast-generating unit comprises a metal interactor selected from the group of metallothionein, preferably MT3 or a series of different metallothionein species, fatty acid-binding proteins (FABPs), and the lipid-binding domain of the cytochrome P450, preferably the lipid- and heme-binding domain of the cytochrome P450 BM3 (BM3h) or a combination of these metal interactors (SEQ ID NO: 229, 230, 231). In this embodiment, if the metal interactor is one or more metallothionein, one to three copies of the MT3 or one to three different metallothionein species are bound to the N-terminus of the encapsulin. Moreover, one or more MT3, FABP, or BM3h are bound to the surface of the encapsulin, optionally via a spacer, wherein the spacer is optionally a SasG element of variable length. Such organized structural element is configured to generate a distinct shape of a barcode, wherein the barcode is a concentric barcode differentiable at the nanoscale (SEQ ID NO: 228, 232, 233, 234, 235, 236). Such concentric barcode may encode information about the cellular state.

The genetically controlled structural element may comprise a vault. In an embodiment, the vault comprises two hemi-vaults, each comprising 39 subunits of the major vault protein. In yet another embodiment, the vault protein comprises at least one fluorophore, preferably a fluorescent protein. In a preferred embodiment, the vault protein comprises a small fluorescent protein. In a more preferred embodiment, the small fluorescent protein is eUnaG, and the fluorescent protein is mScarlet. In another preferred embodiment, the small fluorescent protein is fused covalently to the artificially introduced internal HLH domain of the vault protein and/or the fluorescent protein is targeted non-covalently via a minimal interaction (mINT) motif. In yet another preferred embodiment, the vault comprises a SNAP25 palmitoylation motif. In a preferred embodiment, the genetically controlled structural element organizing the genetically controlled nanoscopy contrast-generating unit is a fusion protein comprising a vault and at least one metal interactor. In yet another preferred embodiment, the genetically controlled structural element is a vault, and the at least one metal interactor comprises at least one metallothionein. In yet another preferred embodiment, the genetically controlled structural element is a vault, and the at least one metal interactor is at least one murine metallothionein. In even another preferred embodiment, the genetically controlled structural element is a vault, and the at least one metal interactor is tandem MT3. In another preferred embodiment, the genetically controlled structural element is a vault, and the at least one metal interactor is RNA. In yet another preferred embodiment, the genetically controlled structural element is a vault, and the at least one metal interactor is an RNA-binding protein. In even another preferred embodiment, the genetically controlled structural element is a vault, and the at least one metal interactor is a lipid-binding protein, preferably a fatty-acid binding protein. In one embodiment, the genetically controlled structural element is a vault, the at least one metal interactor is RNA and the genetically controlled nanoscopy contrast-generating unit additionally comprises a kinase, IMEF, or a tyrosinase. In yet another preferred embodiment, the genetically controlled structural element is a vault, the at least one metal interactor is an RNA-binding protein and the genetically controlled nanoscopy contrast-generating unit additionally comprises a kinase, IMEF, or a tyrosinase. In even another preferred embodiment, the genetically controlled structural element is a vault, the at least one metal interactor is a lipid-binding protein, preferably a fatty-acid binding protein and the genetically controlled nanoscopy contrast-generating unit additionally comprises a kinase, IMEF, or a tyrosinase.

The genetically controlled structural element may comprise a bacteriophage, in other words, prokaryotic (bacterial and archaeal) viruses. In an embodiment, the genetically controlled structural element comprises a bacteriophage and at least one genetically controlled nanoscopy contrast-generating unit comprising a metal interactor wherein genetically controlled nanoscopy contrast-generating unit is spatially organized by the structural element. In a preferred embodiment, the genetically controlled structural element is selected from the group consisting of MS2 phage (SEQ ID NO: 100), Qbeta phage (SEQ ID NO: 102), AP205 phage (SEQ ID NO: 207). In yet another preferred embodiment, the genetically controlled structural element is a self-assembling and thus, symmetric and multivalent genetically controlled structural element such as for example, MS2 phage and Qbeta phage. In a preferred embodiment, the genetically controlled structural element organizing the genetically controlled nanoscopy contrast-generating unit is a fusion protein comprising a bacteriophage and at least one metal interactor. In a preferred embodiment, the at least one metal interactor is MT3. In yet another preferred embodiment, the genetically controlled structural element is a bacteriophage and the at least one metal interactor comprises at least one metallothionein. In yet another preferred embodiment, the genetically controlled structural element is a bacteriophage and the at least one metal interactor is at least one murine metallothionein. In even another preferred embodiment, the genetically controlled structural element is a bacteriophage, and the at least one metal interactor is tandem MT3. In another preferred embodiment, the genetically controlled structural element is a bacteriophage, and the at least one metal interactor is RNA. In yet another preferred embodiment, the genetically controlled structural element is a bacteriophage and the at least one metal interactor is an RNA-binding protein. In even another preferred embodiment, the genetically controlled structural element is a bacteriophage and the at least one metal interactor is a lipid-binding protein, preferably a fatty-acid binding protein. In one embodiment, the genetically controlled structural element is a bacteriophage, the at least one metal interactor is RNA and the genetically controlled nanoscopy contrast-generating unit additionally comprises a kinase, IMEF or a tyrosinase. In yet another preferred embodiment, the genetically controlled structural element is a bacteriophage, the at least one metal interactor is an RNA-binding protein and the genetically controlled nanoscopy contrast-generating unit additionally comprises a kinase, IMEF, or a tyrosinase. In even another preferred embodiment, the genetically controlled structural element is a bacteriophage, the at least one metal interactor is a lipid-binding protein, preferably a fatty-acid binding protein, and the genetically controlled nanoscopy contrast-generating unit additionally comprises a kinase, IMEF, or a tyrosinase.

The genetically controlled structural element may comprise an engineered mono-to-multivalent hub. Such engineered mono-to-multivalent hub is ,for instance, a SunTag or CCC-tag, preferably an emFP (SEQ ID NOs: 92-99). In another embodiment, the genetically controlled structural element comprises at least one mono-to-multivalent hub and at least one metal interactor by the organization of the genetically controlled nanoscopy contrast-generating unit.

The genetically controlled structural element may comprise one or more filaments. In a preferred embodiment, the one or more filament is DHF 119. In another preferred embodiment, the genetically controlled structural element organizing the genetically controlled nanoscopy contrast-generating unit is a fusion protein comprising at least one filament and at least one metal interactor. In a preferred embodiment, the metal interactor is MT3.

The minimal structural element can thus be constructed from just linkers, such as a GGSG linker (SEQ ID NO:237) that connect individual CGUs such as FABPs (Figure 13).

The genetically controlled structural element may comprise at least one or more fibers. In a preferred embodiment, the genetically controlled structural element organizing the genetically controlled nanoscopy contrast-generating unit is a fiber. In a preferred embodiment, the at least one or more fibers is an amyloid-like fiber. In an even more preferred embodiment, the amyloid-like fiber is functional amyloid. In a preferred embodiment, the functional amyloid is CsgA, preferably *E.coli* CsgA. In another preferred embodiment, the CsgA is optimized for intracellular expression, i.e., the CsgA is codon-optimized for mammalian cells. In another embodiment, the fiber is contrast-enhanced by fusing the fiber with, e.g., to a SpyTag. In a preferred embodiment, the genetically controlled structural element organizing the genetically controlled nanoscopy contrast-generating unit is a fusion protein comprising a fiber and at least one metal interactor. In a preferred embodiment, the metal interactor is MT3, a lipid-binding protein, or RNA. In another preferred embodiment, MT3 is fused to the cognate SpyCatcher binding partner.

In another embodiment, the genetically controlled structural element is spaghetti monster fluorescent proteins and variants thereof such as emFPs (SEQ ID NOs: 92-99) into which amino acid sequences are introduced that in turn can be bound by the set of attachment points conjugated to the genetically controlled nanoscopy contrast generating units such as MT3, FABPS, BM3h, RNA, RNA aptamers, metal binders.

The genetically controlled structural element may be the rigid rod-like protein SasG (SEQ ID NO: 207). In a preferred embodiment, SasG comprises a linear structure composed of concatenated SasG elements. In one preferred embodiment, SasG may contain mono-to-polyvalent hubs such as emFPs SEQ ID NOs: 92-99 instead of the fluorescent proteins, e.g. sfGFP, mTurquoise2, or mCherry (in Figure 17); alternatively, CGUs such as FABPs directly replace the fluorescent proteins (SEQ ID NO: XXX). In one embodiment, said concatenable elements provide attachment points to genetically controlled nanoscopy contrast-generating units according to the invention. For example, in one embodiment, the genetically controlled nanoscopy contrast-generating units comprise a metallothionein, a lipid-binding protein, or RNA, and at least one RNA-binding protein.

The genetically controlled structural element may further comprise at least one attachment point. Said at least one attachment point is selected from the group consisting of nanobodies, frankenbodies, coiled-coil domains, isopeptide-forming partners such as SpyTag or SpyCatcher domains, SnoopTag/SnoopCatcher, DogTag/DogCatcher, bioconjugation tags such as SNAP/Clip-Tag domains, and/or Halo-Tag domains, split-inteins, calmodulin-binding peptides, phosphorylatable peptides, conformation-changing peptides such as troponins, as well as combinations thereof. The genetically controlled nanoscopy contrast-generating unit binds to and/or interacts with the structural element via at least one attachment point. In one embodiment, the genetically controlled structural element comprises internal attachment points. Said internal attachment points are selected from the group consisting of nanobodies, frankenbodies, coiled-coil domains, SpyTag or SpyCatcher domains, SNAP/Clip-Tag domains, and/or Halo-Tag domains, split-inteins, calmodulin-binding peptides, phosphorylatable peptides, conformation-changing peptides such as troponins, as well as combinations thereof. In another embodiment, the genetically controlled structural element comprises external attachment points. Said external attachment points are selected from the group consisting of nanobodies, frankenbodies, coiled-coil domains, SpyTag or SpyCatcher domains, SnoopTag/SnoopCatcher, DogTag/DogCatcher, SNAP/Clip-Tag domains, and/or Halo-Tag domains, split-inteins, calmodulin-binding peptides, phosphorylatable peptides, conformation-changing peptides such as troponins, as well as combinations thereof. In yet another embodiment, the genetically controlled structural element comprises internal attachment points and external attachment points. Said internal and external attachment points are selected from the group consisting of nanobodies, frankenbodies, coiled-coil domains, SpyTag or SpyCatcher domains, SnoopTag/SnoopCatcher, DogTag/DogCatcher, SNAP/Clip-Tag domains, and/or Halo-Tag domains, split-inteins, calmodulin-binding peptides, phosphorylatable peptides, conformation-changing peptides such as troponins, as well as combinations thereof. In one embodiment, the genetically controlled nanoscopy contrast-generating unit interacts or binds to at least one external attachment point. In yet another embodiment, the genetically controlled nanoscopy contrast-generating unit can interact or bind to internal and/or external attachment points, whereby the interaction or binding leads to a distinct, pre-defined shape.

In one embodiment, the genetically controlled nanoscopy contrast-generating unit is N-terminally and/or C-terminally fused to the genetically controlled structural element. In a preferred embodiment, the genetically controlled nanoscopy contrast-generating unit is N-terminally fused to the genetically controlled structural element. In an even more preferred embodiment, the genetically controlled nanoscopy contrast-generating unit is MT3 and is N-terminally fused to an encapsulin. In an equally preferred embodiment, MT3 is N-terminally fused to an encapsulin, and an epitope tag is fused to the C-terminus of encapsulin. In one embodiment, the C-terminal epitope tag is a FLAG tag, or BC2 tag.

In one embodiment, the genetically controlled structural element comprises a small fluorescent protein, which provides both high contrast in EM applications and super-resolution in fluorescence applications. In another embodiment, a small fluorescent protein is N-terminally or C-terminally appended to the genetically controlled structural element. In another embodiment, a small fluorescent protein is N-terminally appended to the genetically controlled structural element. In yet another embodiment, the genetically controlled structural element is n=1, 2, 3, 4, 5, 6, 7, 8, 9, or more. In a preferred embodiment, the small fluorescent protein is UnaG, and UnaG is N-terminally appended to the genetically controlled structural element. In another preferred embodiment, the genetically controlled structural element is an encapsulin. In an even more preferred embodiment, UnaG is N-terminally appended to 1x MT3-QtEnc, resulting in UnaG-1xMT3-QtEnc. In an equally preferred embodiment, UnaG is N-terminally appended to 2xMT3-QtEnc, resulting in UnaG-2xMT3-QtEnc.

In one embodiment, the genetically controlled structural element is loaded with protein cargo. In another preferred embodiment, the genetically controlled structural element is loaded with protein cargo and targeted to an intracellular protein target by an intrabody. In a preferred embodiment, the protein cargo is mScarlet. In one embodiment, the intrabody is directly fused to the C-terminus of the genetically controlled structural element. In a preferred embodiment, the genetically controlled structural element is an encapsulin, preferably QtEnc. In an equally preferred embodiment, the intrabody is covalently attached to the genetically controlled structural element by SpyTag-SpyCatcher chemistry, wherein the SpyTag is presented on the exterior of the genetically controlled structural element, and the intrabody is fused to a SpyCatcher domain. In a preferred embodiment, the genetically controlled structural element is an encapsulin, preferably QtEnc. In another preferred embodiment, the intracellular protein target is membrane localized EGFP (EGFP-CAAX) and the intrabody is an anti-GFP intrabody. In yet another preferred embodiment, a cognate coiled-coil (CC) pair is fused to the exterior of the genetically controlled structural element; the cognate CC part P3 is N-terminally appended to the intracellular protein target. In a preferred embodiment, the genetically controlled structural element is an encapsulin, preferably QEnc; the cognate CC part P3 is N-terminally appended to the membrane-localized EGFP (EGFP-CAAX).

In one embodiment, the genetically controlled structural element is detectable by both electron microscopy and fluorescence microscopy techniques. In yet another embodiment, the genetically controlled structural element is compatible with both electron microscopy and fluorescence microscopy techniques.

In one embodiment, the genetically controlled structural element is expressed in a cell of a given tissue. Said tissue is captured on TEM grids or on silica wafers for subsequent analysis by TEM or SEM, allowing the detection of said genetically structural element in the identical cell. In a preferred embodiment, the genetically controlled structural element is QtEnc-FLAG-NLS. In another preferred embodiment, the genetically controlled structural element can be detected by both TEM and SEM.

In another embodiment, the genetically controlled structural element is detected by FIB-SEM (focused ion beam scanning electron microscopy). In yet another embodiment, MT3-QtEnc is detected by FIB-SEM. In a preferred embodiment, MT3-QtEnc is expressed in *Drosophila melanogaster* neurons and detected by FIB-SEM. In another preferred embodiment, the genetically controlled structural element can be detected by TEM, SEM including FIB-SEM, and fluorescence microscopy.

In one embodiment, the genetically controlled structural element can be directed to the nucleus. In another embodiment, the genetically controlled structural element can be directed to the cytosol. In one embodiment, the genetically controlled structural element is an encapsulin. In a preferred embodiment, the encapsulin is QtEnc. In a preferred embodiment, the genetically controlled structural element is QtEnc N-terminally fused to a fluorescent protein. In an even more preferred embodiment, the genetically controlled structural element is QtEnc, and the N-terminally fused fluorescent protein is miniSOG2. In an equally preferred embodiment, QtEnc is N-terminally fused to miniSOG2, and C-terminally fused to a FLAG epitope, followed by a mammalian NLS (c-myc) or a NES (HIV).

In another embodiment, the N-terminally fused fluorescent protein does not compromise the assembly of the genetically controlled structural element. In a preferred embodiment, the N-terminally fused miniSOG2 does not compromise the assembly of QtEnc.

Another preferred embodiment is the CC-MT3-QtEnc construct, for which an exemplary sequence is given in SEQ ID NO: 21, with a CC-binding partner given in SEQ ID NO: 22.

The genetically controlled structural element may be of any combination of the above listed structural elements in any combination with any genetically controlled nanoscopy contrast generating unit disclosed herein. The structural element may comprise one or more contrast generating units comprising one or more of a metal-interacting polypeptide/protein, metal-binding peptide/protein, lipid-binding protein, RNA-binding protein, RNA or DNA molecule, polymerizing or synthesizing enzyme, or bioconjugation tag. This means, all of the above-listed contrast-providing genetically controlled nanoscopy contrast-generating units, can be combined with each other and can be spatially organized in any one or more of the above-listed structural elements. The genetically controlled structural elements and the genetically controlled nanoscopy contrast-generating units disclosed herein are thus to be seen as modules that may be combinable in form of modular combinatorics.

### Genetically controlled scaffolds

In yet another aspect, the invention refers to a genetically controlled scaffold. The genetically controlled scaffold comprises and spatially organizes one or more genetically controlled structural elements. Such spatial organization generates distinct geometric patterns that can be distinguished at the nanoscale. Preferably, such distinct geometric patterns encode information on cellular states.

Such generated geometric patterns include vaults arrayed via SNAP25 (Figure 11), APH or N-terminal IMEF, encapsulins bound to the membrane via farnesylation tags, or via membrane-localized GPF targets (Figure 16), which can also be used to localize encapsulins to other organelles such as lysosome, multiple encapuslins organized via SasG or RNA as scaffolds (Figure 17).

In one embodiment, the information on cellular states is information in relation to external stimuli, for example, environmental parameters such as pH, temperature, or, influx, or efflux of ions such as calcium, response to ions such as calcium, proteolytic activity, conformational changes, or receptor densities. In another embodiment, the information on cellular states can for example identify a normal, i.e., healthy cell from a diseased cell in a pathological state. In yet another embodiment, the information on cellular states can identify diseases such as neurological diseases or cancer.

The genetically controlled scaffold may be selected from the group consisting of an endogenous cellular scaffold, a *de novo* designed scaffold, one or more SasG elements (SEQ ID NO: 112), CsgA (SEQ ID NO: 112), RNA or DNA structures, or combinations thereof. The genetically controlled scaffold interacts or binds to the genetically controlled structural elements and, therefore, organizes the structural element. In one embodiment, the genetically controlled structural element comprises a member of the encapsulin family, a vault, MS2 phage, Qbeta phage, AP205 phage, mono-to-multivalent hubs, a filament, a linker, or combinations or variants thereof. In a preferred embodiment, the genetically controlled structural element is modified at the C- or N-terminus, suitable modifications are, for example, myristoylation, palmitoylation, isoprenylation, or prenylation such as farnesylation and gernaylgeranylation, or glypation. In another preferred embodiment, the genetically controlled structural element is modified by C-terminal farnesylation. By said C-terminal farnesylation the genetically controlled structural element is targeted to the membranes of endogenous, i.e., cellular scaffolds.

In an embodiment, the genetically controlled scaffolds are endogenous cellular scaffolds, such as cellular membranes of organelles, i.e., the nucleus, endoplasmic reticulum, Golgi apparatus, mitochondria, plastids, lysosomes, the inner cell membrane, and vacuoles. In yet another embodiment, the endogenous cellular scaffold is the cytoskeleton, i.e, actin filaments and/or microtubuli.

In another embodiment, the genetically controlled scaffold is an exogenous scaffold, such as a *de novo* designed scaffold. In one embodiment, the *de novo* designed scaffold is selected from the group consisting of, for example, filaments such as DHF or designed RNA structures. In a preferred embodiment, the *de novo* designed scaffold is DHF or 13-01, a *de novo* designed icosahedron comprising 60 cognate SpyCatcher binding partners.

In one preferred embodiment, the targeted controlled structural element is an encapsulin. In a preferred embodiment, the encapsulin is QtEnc, MxEnc, or TmEnc. In an even more preferred embodiment, the genetically controlled structural element is an iron-mineralizing QtEnc C-terminally modified with a SpyTag, wherein said iron-mineralizing QtEnc is C-terminally modified with a SpyTag covalently clustered with 13-01. The iron-mineralized C-terminally modified QtEnc with a SpyTag that is covalently clustered with 13-01 leads to unbounded clusters. In said embodiment, the genetically controlled scaffold is geometrically unbounded. In a preferred embodiment, the genetically controlled scaffold arranges or organizes an encapsulin, even more preferably QtEnc, even more preferably a modified QtEnc comprising an antiGFP antibody and/or TmEnc with an anti-mCherry intrabody. In said embodiment, the genetically controlled scaffold is geometrically unbounded.

In yet another embodiment, the scaffold is a linear scaffold, such as a linear protein structure such as SasG. In even another embodiment, the scaffold is a concatenable linear protein. In a preferred embodiment, the linear scaffold is a bacterial surface protein, even more preferred SasG from *S. aureus* (SEQ ID NO: 207). In another embodiment, the SasG C-terminus and N-terminus of the genetically controlled structural element are fused to fluorescent proteins, wherein the C-terminus and N-terminus are fused to sfGFR and mCherry, respectively, or wherein the N-terminus and C-terminus are fused sfGFR and mCherry.

The spatial organization of the genetically controlled structural elements by the genetically controlled scaffold yields distinct patterns. In one embodiment, the genetically controlled scaffold yielding distinct patterns is SasG. In a preferred embodiment, the genetically controlled scaffold yielding patterns is concatenated SasG. In another embodiment, a pattern is formed by distinct pre-defined spacers and attachment points within SasG. In another embodiment, the SasG spaces and attachment points are fully controllable such that the resulting SasG pattern is a fully-genetically defined linear barcode, i.e., each genetic element on the DNA and RNA has its corresponding linear element on the protein level, whereby a geometrically defined pattern is formed. In yet another embodiment, the geometrical pattern is bounded. In one embodiment, the genetically controlled structural element is an encapsulin, or ferritin (iron-mineralizing QtEnc C-terminally modified with a SpyTag, wherein in said iron-mineralizing QtEnc is C-terminally modified with a SpyTag covalently clustered with SasG) and the genetically controlled scaffold is SasG. In another embodiment, a pattern is formed by SasG in a linear assembly, i.e., without one or more structural elements.

In a specifically preferred embodiment, the genetically controlled scaffold is SasG of variable lengths, wherein the SasG comprises and spatially organizes encapsulins as structural elements, wherein the encapsulins comprise and spatially organize a genetically controlled nanoscale contrast-generating unit comprising one or more MT3 domains or a series of different metallothionein species (SEQ ID NO: 169) as the metal interactor.

In an embodiment, the genetically controlled scaffold is formed by amyloid curly fibrils such as CsgA from *E. coli* providing attachment points.

In another embodiment, the genetically controlled scaffold is designed from RNA origami structures that folds into rigid assemblies that can span tens of nanometers. In a preferred embodiment, said RNA origami structures contain aptamer loops such as PP7 or MS2 to which proteinaceous contrast-generating units can be attached. In another preferred embodiment, said RNA origami structures contain aptamers for exogenous fluorophores, such as Spinach. In another preferred embodiment, the aptamer structures are circularized via ligase RtcB via the Tornado system to stabilize them in mammalian cells.

In one embodiment, the genetically controlled scaffold comprises at least one attachment point. Said at least one attachment point is selected from the group consisting of nanobodies, frankenbodies, coiled-coil domains, isopeptide-forming partners such as SpyTag or SpyCatcher domains, SnoopTag/SnoopCatcher, DogTag/DogCatcher, bioconjugation tags such as SNAP/Clip-Tag domains, and/or Halo-Tag domains, split-inteins, calmodulin-binding peptides, phosphorylatable peptides, conformation-changing peptides such as troponins, as well as combinations thereof. In one embodiment, the genetically controlled scaffold comprises internal attachment points. Said internal attachment points are selected from the group consisting of nanobodies, frankenbodies, coiled-coil domains, isopeptide-forming partners such as SpyTag or SpyCatcher domains, SnoopTag/SnoopCatcher, DogTag/DogCatcher, bioconjugation tags such as SNAP/Clip-Tag domains, and/or Halo-Tag domains, split-inteins, calmodulin-binding peptides, phosphorylatable peptides, conformation-changing peptides such as troponins, as well as combinations thereof. In another embodiment, the genetically controlled scaffold comprises external attachment points. Said external attachment points are selected from the group consisting of nanobodies, frankenbodies, coiled-coil domains, isopeptide-forming partners such as SpyTag or SpyCatcher domains, SnoopTag/SnoopCatcher, DogTag/DogCatcher, bioconjugation tags for covalent protein labeling such as SNAP/Clip-Tag domains, and/or Halo-Tag domains, split-inteins, calmodulin-binding peptides, phosphorylatable peptides, conformation-changing peptides such as troponins, as well as combinations thereof. In yet another embodiment, the genetically controlled scaffold comprises internal attachment points and external attachment points. Said internal and external attachment points are selected from the group consisting of nanobodies, frankenbodies, coiled-coil domains, isopeptide-forming partners such as SpyTag or SpyCatcher domains, SnoopTag/SnoopCatcher, DogTag/DogCatcher, bioconjugation tags for covalent protein labeling such as SNAP/Clip-Tag domains, and/or Halo-Tag domains, split-inteins, calmodulin-binding peptides, phosphorylatable peptides, conformation-changing peptides such as troponins, as well as combinations thereof. In one embodiment, the genetically controlled scaffold interacts or binds to at least one external attachment point of a structural element.

In one embodiment, the genetically controlled structural elements comprise external attachment points. In a preferred embodiment, the external attachment points are monovalent. In one embodiment, the genetically controlled scaffold is SasG and the genetically controlled structural element is an encapsulin and comprises at least one attachment point, wherein the attachment point is selected from the group consisting of nanobodies, frankenbodies, coiled-coil domains, isopeptide-forming partners such as SpyTag or SpyCatcher domains, SnoopTag/SnoopCatcher, DogTag/DogCatcher, bioconjugation tags for covalent protein labeling such as SNAP/Clip-Tag domains, and/or Halo-Tag domains, split-inteins, calmodulin-binding peptides, phosphorylatable peptides, conformation-changing peptides such as troponins, as well as combinations thereof. In one preferred embodiment, the genetically controlled scaffold is SasG, and the genetically controlled structural element is an encapsulin and comprises at least one fluorescent molecule. In another embodiment, the genetically controlled scaffold is SasG, and the genetically controlled structural element is an encapsulin and comprises two fluorescent molecules such as sfGFP and mCherry. In another embodiment, the genetically controlled scaffold, the genetically controlled structural element, and the two fluorescent molecules are detectable by both electron microscopy and fluorescence microscopy techniques. In yet another embodiment, the genetically controlled scaffold, the genetically controlled structural element, and the two fluorescent molecules are compatible with both electron microscopy and fluorescence microscopy techniques. In one embodiment, SasG fusion proteins, such as sfGFP-SasG-mCherry to which structural elements can bind via bioorthogonal intrabodies to the fluorescent proteins. In a preferred embodiment, the SasG proteins form geometric patterns by connecting the structural elements QtEnc of about 40 nm in diameter with the structural elements TmEnc of about 20 nm diameter. The resulting pattern comprises QtEnc surrounded by TmEnc, wherein the low-contrast space between both structural elements is determined by the length of the rigid SasG linker. In another embodiment, the fusion proteins QtEnc^{antiGFPib} and TmEnc-LaM-4 are disclosed, also resulting in distinct patterns.

In a preferred embodiment, the SasG-organized patterns of multiple encapsulins as structural elements are modularly combined with the concentric barcodes generated inside the lumen of each structural element.

The genetically controlled scaffold may be of any combination of the above-listed scaffolds in any combination with any genetically controlled structural element and any genetically controlled nanoscopy contrast generating unit disclosed herein. The scaffold may comprise at least one structural element, wherein each structural element may comprise one or more contrast generating units comprising one or more of a metal-interacting polypeptide/protein, metal-binding peptide/protein, lipid-binding protein, RNA-binding protein, RNA or DNA molecule, polymerizing or synthesizing enzyme, or bioconjugation tag. This means, all of the above-listed contrast-providing genetically controlled nanoscopy contrast-generating units, can be combined with each other and can be spatially organized in any one or more of the above listed structural elements, wherein all such combined structural elements can be combined with each other and spatially organized by any of the disclosed scaffolds. The genetically controlled scaffolds, genetically controlled structural elements, and genetically controlled nanoscopy contrast-generating units disclosed herein are thus to be seen as modules, which may be combinable in form of modular combinatorics.

### Use of the invention

In another aspect, the present invention relates to the use of a genetically controlled structural element, and/or a genetically controlled scaffold for nanoscopy detection, wherein nanoscopy detection comprises molecular mapping and/or geometric sensing. In certain embodiments, the nanoscopy detection further comprises geometric actuation.

Molecular mapping and/or geometric sensing is preferably performed in cells, organoids, tissue, or model organisms. In one embodiment, the cell is an animal cell selected from the group consisting of the genera *Homo, Rattus, Mus, Sus, Bos, Danio, Canis, Felis, Equus, Salmo, Oncorhynchus, Gallus, Meleagris, Drosophila, or Caenorhabditis.* In a preferred embodiment, the animal cell can be of the species *Homo sapiens, Rattus norvegicus, Mus musculus, Sus scroƒa, Bos taurus, Danio rerio, Danionella translucida, Salmo salar, Canis lupus, Felis catus, Equus caballus, Oncorhynchus mykiss, Gallus, Meleagris gallopavo, Drosophila melanogaster* or *Caenorhabditis elegans.* The animal cell can be from a cow, pig, sheep, goat, bison, horse, donkey, mule, rabbit, chicken, duck, goose, turkey, or pigeon. In a particularly preferred embodiment, the animal cell is a mammalian cell and is selected from the group consisting of neuronal cells, glial cells, cardiomyocytes, glucose-responsive insulin-secreting pancreatic beta cells, hepatocytes, astrocytes, oligodendrocytes, chondrocytes, osteoblasts, retinal pigment epithelial cells, fibroblasts, keratinocytes, dendritic cells, hair follicle cells, renal duct epithelial cells, vascular endothelial cells, testicular progenitors, smooth and skeletal muscle cells, hematopoietic cells, cartilage cells or epidermal cells as well as insulin-secreting β-cells, embryonic stem cells, and mesenchymal cells. In an even more preferred embodiment, the mammalian cell is a human cell and is selected from the group consisting of neuronal cells, glial cells, cardiomyocytes, glucose-responsive insulin-secreting pancreatic beta cells, hepatocytes, astrocytes, oligodendrocytes, chondrocytes, osteoblasts, retinal pigment epithelial cells, fibroblasts, keratinocytes, dendritic cells, hair follicle cells, renal duct epithelial cells, vascular endothelial cells, testicular progenitors, smooth and skeletal muscle cells, hematopoietic cells, cartilage cells or epidermal cells as well as insulin-secreting β-cells, embryonic stem cells, and mesenchymal cells.

In another embodiment, the mapping and/or sensing is performed in organoids ortissues. In yet another embodiment, the mapping and/or sensing is performed in model organisms. In one embodiment, the model organism is, for example, C. *elegans, Drosophila melanogaster, Danio rerio, Danionella translucida, Mus musculus, Taeniopygia Guttata*

The genetically controlled structural element, and/or a genetically controlled scaffold may be used for nanoscopy detection to read-out multiplexed information on the promoter activity and gene expression state of cells. The genetically controlled structural element, and/or a genetically controlled scaffold may furthermore provide information about diffusion constants in different cells and tissues. The genetically controlled structural element, and/or a genetically controlled scaffold may furthermore be used as fiducial markers, throughout entire cells to improve alignment of tilt series, perform drift corrections, or serve as geometrically defined reference markers for image quality assessment, such as point-spread functions or contrast-to-noise-ratios.

The genetically controlled structural element, and/or a genetically controlled scaffold may be used for nanoscopy detection, wherein nanoscopy detection comprises molecular mapping. In this case, the molecular mapping provides information on the subcellular distribution of a molecule of interest within a cell or tissue via the binding of genetically controlled structural elements and/or genetically controlled scaffolds to a molecule of interest which thus determines the subcellular localization of the genetically controlled structural element and/or the genetically controlled scaffolds.

In another embodiment, the information on the subcellular distribution of a molecule of interest within a cell or tissue is provided by the binding to a molecule of interest by the genetically controlled structural element comprising at least one genetically controlled nanoscopy contrast-generating unit, and/or the genetically controlled scaffolds. The molecule of interest may be localized in the lysosomes and endosomes, mitochondria, nucleus, plasma membrane, endoplasmatic reticulum, Golgi apparatus, and/or cytosol. In one embodiment, the genetically controlled structural element and/or the genetically controlled scaffold are targeted to one or more of the subcellular compartments. In another embodiment, the molecule of interest within a cell or a tissue is, for example, a protein, lipid, or carbohydrate. In a preferred embodiment, the molecule of interest is a functionally relevant epitope on organelles, a receptor or channel in a membrane.

In one embodiment, the genetically controlled structural element and/or the genetically controlled scaffold is targeted to an intracellular or extracellular protein target. In a preferred embodiment, the genetically controlled structural element is targeted to an intracellular protein target. In one embodiment, the genetically controlled structural element is targeted to the intracellular protein target by an intrabody. In a preferred embodiment, the genetically controlled structural element is an encapsulin, preferably QtEnc. In one embodiment, the intrabody is directly fused to the C-terminus of encapsulin as the genetically controlled structural element. In another preferred embodiment, the intracellular protein target is membrane localized EGFP (EGFP-CAAX) and the intrabody is an anti-GFP intrabody. In an equally preferred embodiment, the intrabody is covalently attached to the genetically controlled structural element by SpyTag-SpyCatcher chemistry, wherein the SpyTag is presented on the exterior of the genetically controlled structural element, and the intrabody is fused to a SpyCatcher domain. In a preferred embodiment, the genetically controlled structural element is an encapsulin, preferably QtEnc. In yet another equally preferred embodiment, a cognate coiled-coil pair is fused to the genetically controlled structural element and the respective cellular target. In a preferred embodiment, the genetically controlled structural element is an encapsulin, and the cellular target is an intracellular protein target. In one particularly preferred embodiment, the cognate coiled-coil pair is fused to the exterior of QtEnc; the cognate CC part P3 is N-terminally appended to the membrane-localized EGFP (EGFP-CAAX).

In another preferred embodiment, molecular mapping occurs via a heterobifunctional adapter made from inactivated Trim21 (dTrim21) fused to a fluorescent protein to which intrabodies fused to encapsulins as structural elements can bind *(see, e.g.,* Figure 18).

Alternatively, or in addition to the molecular mapping, the genetically controlled structural element, and/or a genetically controlled scaffold may be used for nanoscopy detection, wherein nanoscopy detection comprises geometric sensing. In this case, geometric sensing provides information on a specific cellular state, a cellular process, or the presence or absence of an analyte or environmental parameter of interest within a cell or tissue, or the response to an external stimulus. Such sensing is achieved in that the distinct shape generated by the genetically controlled structural element, and/or the geometric pattern generated by the genetically controlled scaffold changes in response to a pre-defined cellular state, a pre-defined cellular process, at least one analyte or at least one environmental parameter, or an external stimulus. Alternatively, a change in a distinct shape and/or distinct pattern is only generated if such analyte, environmental parameter, or external stimulus is present in sufficient strength, or only in response to specific cellular states or processes.

The change in shape occurs by a spatial rearrangement of the contrast-generating units on a structural element or via the rearrangement of the structural elements on a scaffold. This may occur by a conformational change in the scaffold, the structural element, or the contrast-generating unit.

A specific cellular state is, for instance, a specific constellation of promoter activities, the influx or efflux of ions such as calcium, response to ions such as calcium, proteolytic activity, conformational changes, or receptor densities, a response of a cell, a therapeutic response of the cell and activation of the cell. A specific cellular process is, for instance, protein expression and transport, or mitochondrial fusion or fission. An exemplary analyte is, for instance, calcium or cAMP. An external stimulus is, for instance, a depolarization or the application of electromagnetic or mechanical energy or magnetic gradients. In another embodiment, the at least one environmental parameter is for example, pH, temperature.

In one embodiment, shape change is brought about by an analyte-triggered assembly of a structural element via proteolytic unblocking of a sterically hindered precursor. In one embodiment, the structural elements are blocked, preferably with a triple LBT15 domain. In another embodiment, the blocking domain is cleaved in a calcium-dependent manner. In yet another embodiment, the blocking domain is cleaved when either TEVp or SCANR and ionophore are present. In one embodiment, the genetically controlled structural element assembles following calcium-dependent cleavage of the blocking domain in the presence of either TEVp or SCNAR and ionophore. In even another embodiment, the structural elements assemble upon proteolytic cleavage by a protease. In one embodiment, a degron destabilized NLuc cargo is co-expressed. In another embodiment, the co-expressed cargo serves as a read-out measure. In another preferred instantiation, proteolytic unmasking, which can be triggered by calcium, leads to the concatenation and thus stepwise growth of the scaffold SasG, capped off with distinct fluorescent proteins. In another preferred instantiation, proteolysis unblocks inteins to re-complement split vault monomers.

In another preferred embodiment, a change in the concentric barcode is obtained by the calcium-driven binding of calmodulin, fused to a contrast generating unit FABP, to calmodulin-binding peptides presented on the surface of encapsulins as structural elements.

In another preferred embodiment, the agglomeration state of structural elements such as encapsulin can be changed via calcium-mediated interactions of calmodulin and calmodulin-binding peptides.

In yet another preferred embodiment, a change in patterning can also be achieved by relocalization of structural elements to the membrane via calcium-dependent myristoyl-switch proteins.

In addition to the molecular mapping and geometric sensing, the genetically controlled structural element, and/or a genetically controlled scaffold may be used for nanoscopy detection, wherein nanoscopy detection further comprises geometric actuation. Geometric actuation alters a cellular state or process by a biomechanical and/or biochemical effect or via an external stimulus, ore the depositon of external energy, electromagnetic radiation, mechanical energy, or magnetic gradients.

An example for a biomechanical effect is clustering of a receptor via a multidentate structural element or scaffold, such that cellular signaling is modified. Electromagnetic radiation is, for instance, visible or near-infrared light. Mechanical energy is, for instance, mechanical waves covering the ultrasound frequency range.

Such genetically controlled structural element, and/or the genetically controlled scaffold preferably exerts a biomagnetic, biomechanical, and/or biochemical effect on a biological or non-biological structure.

In one embodiment, genetically controlled structural elements and scaffolds exert biomechanical forces by polyvalent interactions with their surfaces, such as clustering of endogenous receptors in model organisms, which can be monitored by nanoscopy.

In another embodiment, the genetically controlled structural element, and/or the genetically controlled scaffold exerts a biomagnetic effect on a biological or non-biological structure. In one preferred embodiment, encapsulins such as QtEnc are rendered magnetic by co-expression of the ferroxidase IMEF and optionally multicopper oxidase Mnx. The expressing cells can thus be magnetically sorted via Magnetic-activated cell sorting (MACS) and subsequently brought back in culture. In addition, or alternatively, the magnetic encapsulins can be bound to intracellular structures such as organelles to manipulate their subcellular localization via magnetic gradients.

In one embodiment, the biomagnetic effect causes specific (re)-arrangements or (re)-organization and for example, a physiological response. In another embodiment, the genetically controlled structural element, and/or the genetically controlled scaffold exerts a biomechanical effect on a biological or non-biological structure.

The geometric actuation can be combined with geometric sensing in that an analyte or parameter that causes a change in a geometric pattern for geometric sensing (e.g. an agglomeration) may then be the condition for effective geometric actuation (e.g. via magnetic gradient fields, which exert more forces on the agglomerated pattern than the monodisperse structural elements). Furthermore, geometric actuation can be monitored via molecular mapping and geometric sensing.

### Detection of the inventive patterns and shapes

In another embodiment, the geometric pattern is defined by pre-determined shapes of the pre-determined nanoscale-sized genetically controlled structural elements comprising at least one genetically controlled nanoscopy contrast-generating unit.

In one embodiment, element-specific methods built into EM instruments such as for example, EDX, EELS, or nanoSIMS can detect the specific metal localized by the metal interactor an orthogonal dimension for multiplexing, in addition to the geometric shape and pattern. In one preferred embodiment, the pre-determined shape is, for instance, an encapsulin or a vault. In one embodiment, the concatenation of the genetically controlled nanoscopy contrast-generating unit to a genetically controlled structural element produces a distinct shape. In another embodiment, the distinct shape is a function of the 3D distribution of the genetically controlled nanoscopy contrast-generating units within the genetically controlled structural element. In other words, the geometrically distinct shape is generated by the interaction of the genetically controlled nanoscopy contrast-generating units and the genetically controlled structural element. In another preferred emnbodiment, the same shape or pattern can be detected by EM techniques such asTEM, SEM, FIB-SEM and fluorescence of optoacoustics nanoscopy, preferentially with super-resolution techniques.

### Nanobiomaterial

In one aspect, the invention relates to nanobiomaterial consisting of isolated genetically controlled structural elements and/or isolated genetically controlled scaffolds. This nanobiomaterial thus consists of the genetically controlled structural elements and/or genetically controlled scaffolds disclosed herein, which are all isolatable from their originating system, which is a genetically modified cell or a cell-free system.

"Genetically modified" means in this respect that its DNA has been altered by the deletion or addition of genetic material. A "cell-free system" refers to the *in vitro* synthesis of polypeptides in a reaction mix comprising biological extracts and/or defined reagents. The reaction mix will comprise a template for the production of the macromolecule, e.g. DNA, mRNA, *etc.;* monomers for the macromolecule to be synthesized, *e.g.* amino acids, nucleotides, *etc.*; and co-factors, enzymes and other reagents that are necessary for the synthesis, e.g. ribosomes, uncharged tRNAs, tRNAs charged with unnatural amino acids, polymerases, transcriptional factors, tRNA synthetases, *etc..*

In one embodiment, the nanobiomaterial is a genetically controlled structural element. In another embodiment, the nanobiomaterial is a genetically controlled structural element and a genetically controlled scaffold. In yet another embodiment, the nanobiomaterial is a genetically controlled scaffold.

Such nanobiomaterial can be used for any nanoscopy method such as TEM, SEM, SRLM, or optoacoustic superresolution microscopy as described herein, however in a two-step process which comprises the production of the nanobiomaterial, its isolation, and its subsequent use in a biological or non-biological system.

### Vectors

The invention further relates to one or more vectors comprising the nucleic acid encoding the genetically controlled nanoscopy contrast-generating unit, the genetically controlled structural element, and/or the genetically controlled scaffold.

A vector comprising a nucleic acid encoding a genetically controlled structural element such as encapsulin and optionally a genetically controlled nanoscopy contrast-generating unit. In some embodiments, the genetically controlled nanoscopy contrast-generating unit is already present in the cell which is transduced with the vector comprising the structural element. In another embodiment, the vector comprises the nucleic acid encoding a genetically controlled scaffold. Such vector may comprise the nucleic acid encoding the scaffold, the structural element, and the contrast-generating unit. In another embodiment, one of the genetically controlled nanoscopy contrast-generating unit, the genetically controlled structural element, and the genetically controlled scaffold is already present in the cell into with the vector is to be transduced.

The vector may be any vector suitable for the transduction of nucleic acids into cells. The encoded elements may be expressed under the control of a cell-specific promoter. The encoded element may be codon-optimized for cellular expression. Such vector may be a viral vector, a plasmid, an exosome, or any other known vector. A viral vector may be a AAV vector.

### EXAMPLES

### Example 1: Cell expression and specimen preparation for systematic evaluation of contrast-generating units for EM

We generated a library of constructs that could be suitable for a genetically controlled contrast-generating unit based on their ability to spatially control metal-interactors, which may also serve as binders for exogenous fluorophores or chromophores. To this end, we adapted naturally occurring proteins and/or *de novo* designed proteins. For the search of proteins with high content of osmiophilic residues (shown in **Figure 1e****,f)** we used a Python script, which reads protein sequences of an input database such as PDB and calculates the number of osmiophilic residues (C H M W) divided by the length of the protein.

We used standard plasmid lipofection techniques in HEK293 cells to express a library of constructs coding for metal-interactors to screen for sufficient contrast in TEM after standard fixation, post-fixation, and staining protocols **(****Figures 1-6****).** All DNA constructs were either custom synthesized by IDT DNA Technologies or assembled from multiple fragments via HiFi assembly, traditional ligation cloning, or PCR-based mutagenesis methods and cloned into pcDNA 3.1 (+) Zeocin for mammalian expression.

Low passage number HEK293T cells (ECACC: 12022001, obtained via Sigma-Aldrich) were cultured in advanced DMEM with 10 % FBS and penicillin-streptomycin at 100 µg/ml at 37 °C and 5% CO2. HEK293T cells were transfected using X-tremeGENE HP (Roche) transfection reagent according to the manufacturer's protocol (3:1 µl reagent:µg DNA). DNA amounts (ratio shell to cargos) were kept constant in all transient experiments to yield reproducible DNA-Lipoplex formation.

Expression was confirmed by native and SDS gel electrophoresis (12 % BioRad TGX gels, run at 200 V for 40 min) **(****Figures 1****,** **2****,** **3****,** **5****,** **6****).** Constructs resulting in robust, non-toxic expression were then subjected to EM sample preparation using standard EM fixation, post-fixation, and staining protocols compatible with protocols also used for tissue from model organisms.

For EM sample preparation, the staining method was adapted from Deerinck et a/., Microsc. Microanal. (2010). HEK293 cells were harvested 36 hours post-transfection, using accutase treatment, and pelleted by centrifugation. The pellets were then fixed with 2.5% Glutaraldehyde in 0,1 M sodium cacodylate buffer (pH 6,7-7,0) for 20 min. For *Drosophila melanogaster* brains, the fixation duration varied between 20 minutes and 24 hours. After removal of fixative, the material was post-fixed using the 1:1 mixture of 4% OsO4 with 0,3M CB, containing 3% potassium hexacyanoferrate (II) for 20 minutes on ice. The post-fixative solution was removed, and the material was washed twice with 0,1 M sodium cacodylate buffer for 2 minutes, followed by 2x5 min Milli-Q washing steps. Milli-Q water was removed, and the material was optionally treated with 1% tannic acid (10 min, RT). Then, tannic acid was decanted, and the material was washed 5 times for 5 minutes using Milli-Q water. The material was then treated with 1 % uranyl acetate solution (30 min, RT), with subsequent 5 times 5 minutes washing steps with Milli-Q water. Afterward, the material was subjected to treatment with 3% lead aspartate (15 min, RT). Again, the material was washed (5x5 min in Milli-Q water) before proceeding to epoxy embedding. The epoxy embedding process was conducted over 2 days. On the first day, the material was incubated on ice with 75% EtOH for 10 minutes, followed by incubation in 90% EtOH for 10 minutes and 100% EtOH for 30 minutes, then 100% EtOH for 30 min on ice. The EtOH solution was replaced with pure propylene oxide and incubated for 5 minutes under RT. Next, the solution was discarded, and the material was incubated in 1:1 mixture of EPON and Propylene oxide for 30 minutes under RT. Subsequently, the material was sequentially incubated at room temperature in 100% EPON for 30 minutes and again with fresh EPON solution overnight. On the next day, the old epoxy was removed, the material was briefly rinsed in fresh 100% epoxy and left in newly-poured 100% epoxy for 72 hours under 60°C. The *Drosophila m.* brain prior to final EPOXY curation was oriented such that several optical lobes were located parallel to the block's slicing plane. The resulting blocks were subjected to trimming and slicing. For cellular material, the trimming of excess EPOXY from the block's surface was done using an EM TRIM milling system (Leica Microsystems, Germany). Using an UltraCut E microtome (Reichert/Leica, Germany) the prepared blocks were prepared with a histo-knife (DIATOME, Switzerland) and then sequentially cut with an ultra-knife (DIATOME, Switzerland) at a slice thickness of 70 nm, verified by the slices' interference pattern. The slices were deposited either on the surface of a 200 mesh copper grid or the polished side of a silicon wafer.

TEM images were acquired on a Libra120 TEM (Carl Zeiss GmbH, Germany), equipped with a CCD camera (TRONDLE Restlichtverstärkersysteme, Germany) using ImageSP software (SYSPROG, Belarus). Before image acquisition, all grid-supported specimens were pre-irradiated with 120 kV beam voltage, 13 µA beam current, 200 µrad illumination angle without insertion of apertures. The actual image acquisition took place with the activated BIO-AIS condenser aperture system and 60 µm objective aperture under the same beam conditions as used for pre-irradiation, except for the illumination angle that was specified to be equal to 100 µrad. The target magnification for the presented images was selected as 8000X in the microscope control software, exposure timing was set equal to 1000 s. Selection of regions of interest for computing contrast-to-noise-ratios (CNR) was performed using ImageJ.

### Example 2: Complementation of contrast-generating units with light-dependent microscopy methods

Constructs combing metal interactors and fluorophores were expressed in HEK293 cells as described in detail in **Example 1.** 36 hours post-transfection, fluorescence microscopy was conducted on a confocal microscope (Leica SP5 using the 488 nm laser line, Leica Microsystems) or epifluorescence microscope (EVOS FL Auto Cell Imaging System (Thermo Fisher Scientific) **(****Fig. 7****)**. Fluorescence on-gel analysis was performed by illuminating the CN-PAGE gel by UV light and capturing the image on a standard CCD camera **(****Figure 5c** after incubation with DAPI, **Figure 7c****, d,** **Figure 8a** direct fluorescence). Multimodal detection, complementing EM contrast, was also extended to bioluminescence detection, via a luciferase targeted to the inside of the encapsulin via bioorthogonal coiled-coil adapters. Luminescence measurements were taken after the addition of fumirazine on a luminometer (Berthold Centro LB 960 (Berthold Technologies) at 0.1 s acquisition time.) **(****Fig. 8c****)**. In a similarly modular fashion, the enzyme tyrosinase was encapsulated inside the TmEnc, and the formation of melanin was confirmed on a native gel by photoabsorbance, which is also the basis of the photoacoustic signal (Fig. 20c). Alternatively, fluorophores can also be attached via SNAP or HaloTags or can also be presented on the outer surface (e.g., via C-terminal eUnaG fusion) of structural elements such as encapsulins, which leaves the lumen of encapsulins unaltered for organizing contrast-generating units for EM. Since entire fluorescent proteins fused to the outward-facing N-terminus of encapsulins tend to disrupt encapsulin assembly, we decided to reconstitute the smaller fragments of tripartite split-GFPnon the surface as explained in **Figure 7g****-i** and verified assembly and fluorescence by BN-PAGE and fluorescent microscopy, respectively.

### Example 3: Geometric multiplexing with concentric barcodes based on spherical structural elements

We genetically concatenated multiple contrast-generating units from metallothionein 3 (MT3) spherical structural elements based on variants of encapsulins and expressed these structural elements in HEK293 cells **(****Figure 9a****-h,** see detailed protocol in **Example 1),** as well as in *Drosophila* neurons **(****Figure 9i****-k).** To this end, a vector was generated for transgenesis by cloning the respective constructs into pJFRC7-20XUAS-IVS or pJFRC19-13XLexAop2-IVS and submitting those to a commercial *Drosophila* Embryo Injection Service for transgenesis. Sample preparation for TEM from HEK cells and freshly dissected *Drosophila* brains was performed as described in detail in **Example 1.** Histograms of the distribution of diameters were generated with the measurement tools implemented in ImageJ **(****Figure 9e****).** Line profiles shown in **Figure 9f** were computed by using ImageJ and custom-written python scripts.

We furthermore conducted comparative imaging via TEM and SEM of the identical nuclei of *Drosophila* neurons expressing MT3-QtEnc-Flag-NLS or MT3-MxEnc-Flag-NLS **(****Figures 9h****-j).** To this end, ultrathin sections were captured on either TEM grids or on silica wafers for imaging via TEM or SEM, respectively. SEM images were acquired with an SEM Gemini 360 (Carl Zeiss GmbH, Germany), equipped with a Bio-BSD detector (Carl Zeiss GmbH, Germany) using ATLAS software (Carl Zeiss GmbH, Germany). Silicon wafers supporting the samples were glued to the stage with silver glue (PLANO GmbH, Germany). Image acquisition parameters were: 6.5 kV beam voltage, 2 nm nominal pixel size with a 30 µm objective aperture at a working distance of 3.7 mm. The stage was subjected to a bias voltage of 5 kV.

Furthermore, the different encapsulin: metallothionein combinations can be detected via focused ion beam (FIB)-SEM as shown in **Figure 9k****).** To this end, FIB-SEM image acquisition was performed with the SEM Crossbeam 550 (Carl Zeiss GmbH, Germany), equipped with the InLens, BSE, SESI detectors (Carl Zeiss GmbH, Germany) running ATLAS software. The metal-sputtered block with the specimen of interest was positioned on the stage with silver glue (PLANO GmbH, Germany) to ensure the proper adhesion and discharging of the specimen. A guiding pad was deposited in the proximity of the ROI to meet the requirements of image capture alignment during the three-dimensional volume recording. The following settings were used for image acquisition: SEM beam voltage 1.3 kV, a working distance of 5 mm, 4 nm nominal voxel size. The FIB Ga beam, accelerated by 30 kV voltage and holding a current of 700 pA, was used for three-dimensional slicing of the block's volume.

As shown in **Figure 10****,** by concatenating one or multiple metallothioneins, different concentric barcodes can be generated that provide rotational invariant contours also on non-isotropic imaging slices (i.e. in cross-sections or maximum intensity projections) defined by multiple rings of contrast in the lumen of encapsulins (line plots in **Figure 10d** generated by ImageJ). Those concentric barcodes can be complemented by additional rings **(****Figure 10e****)** using any of the contrast-generating units detailed in previous Examples and Figures, for instance, by FABPs targeted via an isopeptide bond formation. Shown in **Figure 10f****)** is the schematic of the genetic constructs for binding MT3 or FABP to the surface to the spherical QtEnc via SpyTag/SpyCatcher isopeptide formation (also compare **Figure 3b****).**

### Example 4: Geometric multiplexing with non-spherical structural elements

Different modifications of the major vault protein were assembled from synthesized DNA and expressed in HEK293 cells, as detailed in **Example 1.** Fluorescent cargo proteins fused to the enzyme APEX2 were co-expressed and imaged via confocal fluorescence microscopy on an SP5 Imaging System, Confocal Microscope (Leica, Wetzlar, Germany). Histochemistry was performed using a standard protocol for APEX2, applying 3,3'-diaminobenzidine (DAB) and hydrogen peroxide (H2O2) **(****Figure 11a****, b**).TEM imaging was performed as described in detail in **Example 1 (****Figure 11c****, d).** Epifluorescence microscopy (EVOS FL Auto Cell Imaging system (Thermo Fisher Scientific) was performed to confirm the dual fluorescence labeling of the major vault protein by UnaG, fused to the internal HLH domain, and the red fluorescent protein mScarlet, targeted to the lumen via an mINT motif.

We furthermore generated non-spherical structural elements by fusing MT3 to auto-assembling Csga monomers **(****Figure 12a****, b),** fibril- or lattice-forming elements **(****Figure 12 c-f),** which were evaluated by TEM as described in detail in **Example 1.** In addition, structural elements can be formed by the concatenation of the nanoscopy contrast-generating units based on FABPs connected via minimal linkers (as detailed in **Figure 13****).**

### Example 5: Monovalent-to-polyvalent hubs as structural elements and control of valency on structural elements

We constructed monovalent adapters to multiple contrast-generating units by constructing variants of spaghetti monster (emFP, **Fig. 14a****-e)** that harbor several epitopes for intrabodies or frankenbodies, which can be fused to the respective nanoscopy contrast-generating units as detailed in **Figure 14b****).** In order to test, whether those variants are still fluorescent, we expressed them in HEK293 cells as described in **Example 1** and imaged them on an EVOS FL Auto Cell Imaging system (Thermo Fisher Scientific) **(****Figures 14a****, c).** Furthermore, we evaluated via combined fluorescence imaging and immunohistochemistry whether the emFPs could be targeted to subcellular structures and still provide the binding sites for the multiple binders. **Figure 14c** shows emFPv4 yielding green fluorescence via mGreenLantern that partially overlaps with Cy-3 fluorescence indicating binding of the antiMoon-Tag intrabody. We furthermore demonstrated the successful interaction on the monovalent-to-polyvalent hub by using CN-PAGE, which shows higher-molecular band species under UV illumination corresponding to bound anti-moon intrabody fused to different metal-interactors **(Figure 14i).** As an alternative to the use of monovalent-to-polyvalent hubs, we evaluated programmable translational read-through to control the stoichiometry of attachment points on the surface of encapsulins to systematically reduce the valency of the surface-presented attachment points (which would otherwise only be determined by the icosahedral symmetry). To this end, we expressed the genetic constructs depicted in **Figures 15a****, b,** such that variable amounts of the bioluminescent tag HiBit were surface-expressed on QtEnc. We subsequently conducted a HiBit assay (Promega) following the protocols suggested by the commercial system using a standard luminometer.

### Example 6: Geometric multiplexing by spatially organizing structural elements on cellular scaffolds

Given the nanometer-scale resolution of electron microscopy and super-resolution microscopy techniques, multiplexed information can be readily encoded by modifying the subcellular localization of the structural elements detailed in previous figures via scaffolds expressed at distinct subcellular locations inside cells. We demonstrate several examples of this strategy by expressing in HEK293 cells - followed by TEM analysis - both as described in **Example 1,** either a membrane localization signal (farnesylation tag, **Figure 16a****),** a peptide from a cargo adaptor which binds to endogenous motor proteins (KinTag, **Figure 16b****),** or an anti-GFP intrabody targeted to lysosomal-localized GFP **(****Figure 16c****),** or membrane-localized GFP **(****Figure 16d****,** in the control condition on the right, a Flag epitope was expressed instead of the anti-GFP intrabody).

### Example 7: Geometric multiplexing by designer scaffolds

As an alternative to using endogenous scaffolds, we demonstrate the versatile application of the rigid hetero-bifunctional protein from *S.aureus* as an engineered scaffold, whose length can be systematically varied to present bio-orthogonal adapters at both ends **(****Figure 17a****).** To demonstrate the precise linear extension of the designed SasG linker when expressed in mammalian cells, we expressed a version of variable length with the FRET pair sfGFP and mCherry on either end, expressed them in HEK293 cells (described in **Example 1),** and subjected the cell lysate to CN-PAGE under UV illumination. This analysis shows a FRET effect indicated by a yellow signal for only the direct fusion of sfGPF and mCherry. In distinction, the bands corresponding to the constructs with longer spacers between the fluorescent proteins give a green fluorescent signal indicating the absence of FRET and the isolated fluorescence from sfGFP **(****Figure 17b****).** We furthermore validated the rigid hetero-bifunctional crosslinker SasG in combined fluorescence microscopy and TEM **(****Figure 17c****, d)** based on the protocols described in detail in the previous examples. The results show distinct spatial patterns of two differently sized encapsulins (QtEnc and TmEnc), indicating that constraints from steric hindrance are sufficient to obtain distinct barcoding patterns from the combination of several structural elements and contrast-generating units **(****Figure 17e****, f).** In addition to the use of a single SasG element, several of these linear connectors can be expressed to generate more complexly shaped structural elements such as triangles and other platonic shapes. By expressing 3 bio-orthogonal pairs of adapters chosen in this case from isopeptide-forming pairs and inteins, triangular shapes can be enforced upon co-expression of the respective components in HEK293 cells and using on-gel fluorescent analysis of the individual combinations of co-expressed components. The results also show a high-molecular species corresponding to the combination of all three elements (last lane in **Figure 17h****).** Alternatively, to proteinaceous scaffolds such as SasG, scaffolds can also be designed from RNA as shown in **Figure 17g****,** which may in parallel provide contrast based on the motifs detailed in **Figure 4****.**

For completion, in addition to the geometrically precise and bounded scaffolds, one can also use simpler clustering of structural elements such as encapsulins surface-presenting SpyTags with multivalent crosslinkers (in this case SpyCatcher mounted to the *de novo* cage l3-01 **(****Figure 17i****).** The EM contrast, in this case, is provided by the ferroxidase IMEF, resulting in a complete filling of the encapsulin lumen. Alternatively, modular combinations with the concentric barcodes detailed in **Example 3** can be used.

### Example 8: Molecular Mapping and Geometric sensing

For mapping the subcellular distribution of biomolecules of interest, structural elements or scaffolds can be targeted to the biomolecule of interest via a range of attachment points whose valency can be controlled via mono-to-polyvalent hubs, which allow for the attachment of several contrast-generating units or structural elements to a bioorthogonal attachment point to the biomolecule of interest **(****Figure 14****)** or via read-through control of the stoichiometries of the attachment points **(****Figure 15****).** Targeting of the target biomolecule of interest can also be achieved via the intracellular delivery of exogenous antibodies (via electroporation or microinjection) that can be connected to a structural element or scaffold via a deactivated Trim21 molecule (dTrim21), which can be fused to an attachment point such as a fluorescent protein **(****Figure 18l****).** This "sandwich" arrangement allows binding of a structural element and additional mapping capabilities via fluorescence microscopy).

To demonstrate that the geometrically distinct structural elements can be made responsive to cellular states, we expressed sterically hindered variants of encapsulins as structural elements containing the contrast-generating unit MT3 (genetic constructs shown in **Figure 18a****)** that allow for proteolytic unmasking by the tobacco etch virus-derived (TEV) bio-orthogonal TEV protease, of which a calcium-dependent version has been generated that is termed SCANR. After expression in HEK293T cells and inducing calcium influx by addition of increasing concentrations of the calcium ionophore (Calcimycin A23187, Sigma-Aldrich), a higher molecular weight band appears on the CN-PAGE **(****Figure 18b****),** corresponding to the accumulation of a band corresponding to the proteolytically cleaved protein on SDS-PAGE **(****Figure 18c****).** In addition, we co-expressed degron-destabilized luciferase (NanoLuc) such that we could use a luciferase signal as an indicator for the amount of NanoLuc that was encapsulated inside the lumen of the encapsulins (via the appropriate encapsulation signal, QtSig). Thus, we could use simple bioluminescent detection with the luciferase-substrate fumirazine to quantify the calcium-dependent proteolytically triggered encapsulin assembly via the amount of luciferase that was protected inside the encapsulin lumen to give a bioluminescent signal **(****Figure 18d****).** The corresponding TEM image shows the expected ring-shaped MT3-QtEncFlag structural elements after calcium influx **(****Figure 18e****).**

As an extension to geometric sensing via changing the shape obtained from structural elements, we also demonstrate how the geometric pattern formed by several structural elements can be altered by altering the geometry of the scaffold as a function of an analyte of interest. **Figures 18f****-i** show how SasG scaffolds can be concatenated via split-intein-mediated processes. These components thus realize a step-wise growing counter/timer mechanism, which can be triggered or stopped by using caged inteins, or modifying the concentration of unmasked Base or Cap modules **(****Figure 18h****, i).**

We furthermore show that the concept of the proteolytic unmasking of sterically-hindered precursors to structural elements can also be applied to non-spherical structural elements such as vaults. **Figure 18j** shows a schematic demonstrating a split major vault protein that is modified by split-intein pairs blocked by masking peptide connected via a TEV-protease cleavage site **(****Figure 18j****).** To show that vault auto-assembly occurs upon co-expression of TEV protease, we co-expressed the fluorescent protein mScarlet with an mINT targeting signal addressing it to the vault lumen and subjected the respective HEK293 cells to epifluorescence microscopy (EVOS FL Auto Cell Imaging system (Thermo Fisher Scientific)). The images show that a cytosolic accumulation of fluorescence signal occurs upon TEV co-expression, which is similar to the signal observed when non-sterically blocked vault monomers (right column) were expressed **(****Figure 18k****).**

In addition to the mechanism of proteolytic unmasking of the building blocks of structural elements, concentric barcodes can be added to the outside of spherical structural elements via, for instance, the surface presentation of calmodulin-binding peptides such as M13 to which calmodulin (CaM) fused to contrast-generating units can bind **(Figure 19a)** similar to the interaction of isopeptide-forming partners displayed in **Figure 3b****, c.** We have furthermore co-expressed QtEnc surface-modified with the CaM-binding peptide aFod and a multi-dentate cross-linker constructed from a ferritin variant from *Pyrococcus ƒuriosus* surface-modified with CaM **(****Figure 19b****)** and the degron-destabilized fluorescent protein mEos4b. We subsequently conducted epifluorescence microscopy and observed the formation of fluorescent intracellular clusters after minutes of triggering calcium influx via the calcium ionophore A23187 (Sigma-Aldrich). These data thus demonstrate the agglomeration of multi-dentate structural elements as a reporter mechanism. The analogous mechanism can be established for phosphorylatable peptides and phosphopeptide-binding proteins such as SEQ ID NOs: 239, 206. Similarly, relocalization of structural elements, demonstrated on the example of vaults in **Figure 19c****,** can be demonstrated by expressing a variant of vaults modified with a myristoyl-switch motif adapted from recoverin, which relocalizes to the membrane upon binding to calcium. The confocal microscopy images show the formation of fluorescent clusters upon pharmacological triggering of calcium influx (30 µM A23187), derived from the co-expressed vault-packaged mEos4b.

### Example 9: Geometric actuation

We have generated several *Drosophila* lines as described in **Example 1** in which a multi-dentate QtEnc surface-modified with anti-GFP intrabodies (QtEncantiGFPIB) is expressed via a panneuronal promoter (Elav) in a standard temperature-dependent fashion via the GaI4:UAS system. When this line is crossed with a line expressing a GFP-tagged receptor GluCI in a transposon Minos-mediated integration cassette (MiMIC) (Venken *et al.,* 2011), the intraneuronal localization of receptors can be controlled via biomechanical actuation through multi-dentate binding to the gradually expressed multi-dentate nanoscopy-visible structural element, which is detected by immunohistochemistry to the Flag-tag in **Figure 20a****.** Please note the temperature-dependent release (switch from 18°C to 29°C, using the standard method to control the conventional GAL4-UAS system via a temperature-sensitive allele of GAL80) of the receptor to the neuropil of the respective *Drosophila* neurons.

In addition to the biomechanical manipulation of the subcellular distribution of biomolecules of interest, we demonstrate in **Figure 20b** how cells can be magnetically sorted when they express encapsulins that are expressing the ferroxidase IMEF. The graph shows the selective retention of HEK293 cells expressing QtEnc and IMEF on commercially available columns (Miltenyi), demonstrating that the magnetic-field-dependent force is sufficient to retain entire cells on magnetic columns. Importantly, all examples here show biomagnetic encapsulins as structural elements, which are patterned by either de novo (the cage I3_01 or the filament DHF, **Figure 12d****)** or natural (farnesylation, **Figure 16a****)** scaffolds which can localize magnetic actuation in the cell while the precise patterns can be read out by nanoscopy, confirming the distinct geometry for the magnetically actuatable structures.

In addition to biomagnetic manipulation with EM-visible nanoscale patterns, we furthermore demonstrate in **Figure 20c** that the strongly photoabsorbing and thus photoablatable polymer melanin, visible standard EM stains, can be formed inside the geometrically distinct structural element such as TmEnc. After the expression of the different genetic combinations of coiled-coil adapter pairs (e.g., P1/P2 for encapsulating *Bacillus megaterium* BmTyr-Pl to P2-TmEnc as specified in **Figure 20c****,** melanin formation could be demonstrated by color formation on CN-PAGE.

### SEQUENCES

The following sequences have been used in the preparation of the invention. It is clear to the skilled person that such sequences are exemplary sequences. Any functional fragment or variant of a sequence is comprised within the scope of the invention. Within the scope of the invention are also sequences having 80%, 81%, 82%, 83%, 84%, 85%, 86%, 87%, 88%, 89%, 90%, 91%, 92%, 93%, 94%, 95%, 96%, 97%, 98%, 99% sequence identity with the given sequences.

**Table 1: Sequences used for this invention**

| **SEQ ID NO** | **Name** | **Used in Fig.** | **Comment** |
|---|---|---|---|
| 1 | MmMT3 (MT3) | Figure 1 d, Figure 9 b,c,d,f,g,h,i,j,k | Metallothionein 3 from *Mus musculus* |
| 2 | His-6 MmMT3 (His-6-1×MT3) | Figure 1 related | Metallothionein 3 from *Mus musculus* with an N-terminal His6 tag for Co/Ni binding |
| 3 | His-9 MmMT3 | Figure 1 related | Metallothionein 3 from *Mus musculus* with an N-terminal His9 tag for Co/Ni binding |
| 4 | 2xMmMT3 (2xMT3) | Figure 1 a,b,d Figure 9 f | Tandem of Metallothionein 3 from *Mus musculus* connecter via GGGSGGGSGGSA linker. |
| 5 | 3x chimeric MT (3xMT) | Figure 10 b,d | Chimeric Metallothionein triplet consisting of MmMT3, Metallothionein SmtA from *Synechococcus elongatus,* and the Metallothionein EC1 from *Tricium aestivum* connected via short GS linkers. |
| 6 | 30xMMKPDM repeats | Figure 1 e | Methionine-rich repeat protein consisting of 30 MMKPDM repeats. Major protein component of the hinge ligament of the pearl oyster *Pinctada ƒucata* |
| 7 | PDB: 2m17 | Figure 1 f | Cysteine Rich peptide from *Panax ginseng* containing many osmiophilic residues |
| 8 | PDB: 4nl8 | Figure 1 f | Cysteine Rich peptide from *Klebsiella pneumoniae* containing many osmiophilic residues |
| 9 | PDB: 2jyt | Figure 1 f | Cysteine Rich peptide from *Homo sapiens* containing many osmiophilic residues |
| 10 | PDB: 5lah | Figure 1 f | Cysteine Rich peptide from *Urticina eques* containing many osmiophilic residues |
| 11 | PDB: 3hcu | Figure 1 f | Cysteine Rich peptide from *Homo sapiens* containing many osmiophilic residues |
| 12 | MeLanM (Lanmodulin) | Figure 14 b,d,e | Lanthanide-binding protein from Methylobacterium extorquens |
| 13 | Lanthanide binding tag 15 (LBT15) | Figure 2 d | Synthethic lanthanide binding tag |
| 14 | AssVanabin2 (Vanabin 2 | Figure 14 b,d,e | Vanadmium-binding protein from *Ascidia sydneiensis samea* |
| 15 | EcCusF | Figure 14 b,d,e | Silver and Copper-binding protein CusF from *Escherichia coli* |
| 16 | QtSig | Figure 1 e, | Encapsulation signal for the Encapsulin system of *Quasibacillus thermotolerans* |
| 17 | PfHRPII-QtSig | Figure 1 e | Histidine-rich protein II of *Plasmodium ƒalciparum* fused to a QtSig via a GGGGSGGGGS linker. |
| 18 | eUnaG-MT3 | Figure 7 a,b,c,d,e,f | Fusion protein consisting of enhanced UnaG(V2L) from *Anguilla japonica* and MmMT3 |
| 19 | SNAP-Tag | related to Figure 7 | human O6-alkylguanine-DNA-alkyltransferase variant that forms covalent bonds with benzylguanine derivatives |
| 20 | Halo-Tag | related to Figure 7 | modified bacterial dehalogenase that forms covalent bonds with chloroalkane derivatives |
| 21 | P2-MT3-QtEnc-FLAG | related to Figure 7 | Fusion of a synthetic coiled-coil (CC) domain (P2), the CGU MmMT3, the QtEnc shell-forming monomer from the Encapsulin system of *Q.thermotolerans* and a FLAG-epitope |
| 22 | P1-mEosEM-hPEST | related to Figure 7 | Fusion of synthetic coiled-coil (CC) domain P1 (cognate partner of P2), OsO4 post-fixation stable switchable fluorescent protein mEosEM and a human PEST degradation sequence |
| 23 | sGFP_S1-9 | Figure 7g | Strand 1 to 9 of a Tripartite split superfolderGFP |
| 24 | QtEnc_sGFP-S1-9 | related to Figure 7 | The shell forming monomer of the Ecapsulin system of *Quasibacillus thermotolerans* fused to superfolder GFP strands 1 to 9 |
| 25 | QtEnc_sGFP-S10 | Figure 7 g | The shell forming monomer of the Ecapsulin system of *Quasibacillus thermotolerans* fused to superfolderGFP strand 10 |
| 26 | QtEnc_sGFP-S11 | Figure 7 g | The shell forming monomer of the Ecapsulin system of *Quasibacillus thermotolerans* fused to superfolderGFP strand 11 |
| 27 | QtEnc_eUnaG | Figure 7-related | The shell forming monomer of the Ecapsulin system of *Quasibacillus thermotolerans* fused to enhanced UnaG(V2L) from *Anguilla japonica* and MmMT3 |
| 28 | MxEnc_eUnaG | Figure 7-related | The shell forming monomer of the Ecapsulin system of *Myxococcus xanthus* fused to enhanced UnaG(V2L) from *Anguilla japonica* and MmMT3 |
| 29 | BM3h (PDB:6H1O) | Figure 3 d | Heme domain of BM3 a natural fusion protein constructed of cytochrome P450 and NADPH-cytochrome P450 reductase domains from *Priestia megaterium* |
| 30 | Liver human FABP | Figure 3 a | Soluble protein binding fatty acids expressed in human liver |
| 31 | Intestinal human FABP | Figure 3-related | Soluble protein binding fatty acids expressed in human intestine |
| 32 | Heart human FABP | Figure 3-related | Soluble protein binding fatty acids expressed in human heart |
| 33 | Adipocyte human FABP | Figure 3-related | Soluble protein binding fatty acids expressed in human adipocytes |
| 34 | Epidermal human FABP | Figure 3-related | Soluble protein binding fatty acid expressed in human epidermis |
| 35 | Ileal human FABP | Figure 3-related | Soluble protein binding fatty acid expressed in human ileum |
| 36 | Brain human FABP | Figure 3 a | Soluble protein binding fatty acid expressed in human brain |
| 37 | Myelin human FABP PMP2 | Figure 3-related | Soluble protein binding fatty acid expressed in human peripheral nerves |
| 38 | Testis human FABP | Figure 3-related | Soluble protein binding fatty acid expressed in human testis |
| 39 | Retinal human FABP | Figure 3-related | Soluble protein binding fatty acid expressed in human retina |
| 40 | Farn-Signal (Farn) | Figure 16 a | 281-290 of rat H-Ras, C-terminal farnesylation signal |
| 41 | SNAP25-derived palmitoylation signal (SNAP25) | Figure 1 a,b,c,d, also relates to Figure 3 | Amino acids 85-120 of Rattus norvegicus SNAP25 |
| 42 | Fyn-Kinase derived N-terminal myristoylation signal | relates to Figure 3 | N-terminal myristoylation signal of human Fyn-Kinase, Amino acids 1-15 |
| 43 | Microtubule-associated proteins 1A/1B light chain 3A (PDB:MLP3A_MOUSE) | relates to Figure 3 | Microtubule-associated proteins 1A/1B light chain 3A (PDB:MLP3A_MOUSE) |
| 44 | MSP26 | relates to Figure 3 | Engineered apo-lipoprotein A-1 (MSP1) flanked by DnaE split intein fragments C-intein (AA 103-137) on the N-term and N-Intein (AA 1-102) on the C-term (*Homo sapiens, Nostoc punctiforme*): |
| 45 | MUP2 | Figure 2 a | Designed uranyl binding proteins with 6 binding sites for uranyl ions |
| 46 | MUP5 | Figure 2 b | Designed uranyl binding proteins with 6 binding sites for uranyl ions |
| 47 | DD-CgPPK2b-QtSig | Figure 5 a,b,c,d | Degron-tagged polyphosphate kinase 2b of *Corynebacterium glutamicum* C-terminanlly tagged with the encapsulation signal of *Quasibacillus thermotolerans* |
| 48 | CgPPK2b | Figure 5 a,b,c,d | Polyphopsphate kinase 2b of *Corynebacterium glutamicum* |
| 49 | AtPCS1-QtSig | Figure 5 e | Phytochelatin synthase 1 of *Arabidopsis thaliana* with a C-terminal encapsulation signal |
| 50 | AtPCS2-QtSig | relates to Figure 5 e | Phytochelatin synthase 2 of *Arabidopsis thaliana* with a C-terminal encapsulation signal |
| 51 | BmTyr | Figure 5 f | *B.megaterium* tyrosinase |
| 52 | QtIMEF | Figure 1 b, Figure 6 a,b,c,d, Figure 16 a, Figure 17 i, Figure 20 b | Iron-mineraizing ferritin-like cargo protein from the Encapsulin system of *Q.thermotolerans* |
| 53 | PP7 phage coat protein (PCP) | Figure 4-related | The coat protein of the single-stranded RNA bacteriophage *Pseudomonas phage* |
| 54 | PP7 phage coat protein tandem dimer (tdPCP) | Figure 4 a | Tandem dimer of the coat protein of the single-stranded RNA bacteriophage *Pseudomonas phage* binding the PP7 stem loop |
| 55 | Spinach Aptamer | Figure 4 c | Synthetically derived RNA aptamer designed to be an RNA mimic of green fluorescent protein (GFP) |
| 56 | PP7 stem loop | Figure 4 b | Small RNA sequence binding partner of the tandem PCP dimer from *Pseudomonas phage* |
| 57 | Dir2s Aptamer | Figure 4 d | Synthetically derived RNA aptamer designed to be an RNA mimic of red fluorescent protein (RFP) |
| 58 | Metal-binding RNA-oligomer | Figure 4-related | Small synthetic aptamer coordination metal ions in homodimeric form |
| 59 | RhoBAST Aptamer | Figure 4 e | Synthetic aptamer that binds a fluorogenic rhodamine dye with fast association and dissociation kinetics |
| 60 | RRM domain | Figure 4 f | RNA-binding domain that are known to bind single-stranded RNAs. |
| 61 | KH2 domain | Figure 4 f | Protein domain that binds RNA, and can function in RNA recognition |
| 62 | Zinc Finger CCHC | Figure 4 f | Zinc finger domain subspecies know to bind RNA |
| 63 | L7Ae | Figure 4 f related | Multifunctional RNA-binding protein that recognizes the K-turn motif in ribosomal RNA |
| 64 | BIV-tat | Figure 4 f related | BIV-Tat from the *Bovine immunodeficiency virus* binds to a hairpin structure at the 5'-end of all nascent viral mRNAs |
| 65 | QtEnc | Figure 1, 2, 3, 5, 6, 7, 8, 9, 15, 16, 17, 18, 19, 20 | Shell forming monomer of the Encapsulin system of *Q.thermotolerans* |
| 66 | MxEnc | Figure 1, 5, 8, 9, 10 | Shell forming monomer of the Encapsulin system of *M.xanthus* |
| 67 | TmEnc | Figure 1 ,8 ,9 ,10 | Shell forming monomer from the encapsulin system of T. *maritima* (AA 1-264) |
| 68 | P1 | Figure 8 | Synthetic coiled-coil domain which pairs with P2 |
| 69 | P2 | Figure 8 | Synthetic coiled-coil domain which pairs with P1 |
| 70 | P3 | Figure 8 | Synthetic coiled-coil domain which pairs with P4 |
| 71 | P4 | Figure 8 | Synthetic coiled-coil domain which pairs with P3 |
| 72 | DHD13a | Figure 8 | Synthetic coiled-coil domain which pairs DHD13b |
| 73 | DHD13b | Figure 8 | Synthetic coiled-coil domain which pairs DHD13a |
| 74 | RT9s | Figure 15 | DNA sequence promoting ribosomal (translational) read-through after a STOP codon |
| 75 | K-turn motif | related to Figure 4 | RNA motif binding to L7Ae |
| 76 | RT20s | Figure 15 | DNA sequence promoting ribosomal (translational) read-through after a STOP codon |
| 77 | Major vault protein (RnMVP) | Figure 11 | Vault forming protein from *Rattus norvegicus* |
| 78 | Major vault protein with SNAP25-derived palmitoylation signal (85-120) (RnMVP-SNAP25) | Figure 11 | Vault forming protein from *Rattus norvegicus* with SNAP25-derived palmitoylation signal (AA 85-120) inserted in helix-loop-helix region |
| 79 | RnMVP-APH | Figure 11 | Vault forming protein from *Rattus norvegicus* with antiparallel coiled-coil domain inserted into helix-loop-helix region |
| 80 | RnMVP-APH-short | Figure 11 | Vault forming protein from *Rattus norvegicus* with a shortened antiparallel coiled-coil domain inserted into helix-loop-helix region |
| 81 | RnMVP-caged-Gp41-1-TEV v1 | Figure 11 | TEV protease inducible Vault based on caged Gp41-1 split inteins, Version 1 |
| 82 | RnMVP-caged-Gp41-1-TEV v2 | Figure 11 | TEV protease inducible Vault based on caged Gp41-1 split inteins, Version 2 |
| 83 | MT3-mINT | Figure 11 | MmMT3 fused to endogenous vault cargo protein mINT |
| 84 | RnMVP_HLH-MT3 | Figure 11 | MmMT3 inserted into rat Vault Helix-Loop-Helix motif |
| 85 | RnMVP_Nterm_BCR_H LH-His6_HLH_MT3 | Figure 11 | Rat Vault with N-terminal anti-parallel coiled-coil domain stabilizing the holo-vault configuration and MT3 and His6-tag in the HLH domain |
| 86 | DeNovoTIM15 | Figure 14 related | Synthetic Hyperstable *de novo* TIM barrel variant |
| 87 | Porcine Ribonuclease Inhibitor | Figure 14 g | Porcine Ribonuclease Inhibitor from Sus scrofa wich exposes modifiable helices |
| 88 | Helical Assembly of the Anbu Complex from *Pseudomonas aeruginosa* | Figure 14 g | Proteasome that forms a dodecameric complex including modifiable helices |
| 89 | gp37-gp38 adhesin tip complex. Chain A | Figure 14 h | Adhesin complex of the bacteriophage S16 long tail fiber. The variable loops can be extended to display binding tags. |
| 90 | gp37-gp38 adhesin tip complex. Chain B | Figure 14 h | Adhesin complex of the bacteriophage S16 long tail fiber. The variable loops can be extended to display binding tags. |
| 91 | Adenovirus species 26 knob protein | Figure 14h | Knob protein from human adenovirus 26. Its loop region can be engineered to display binding tags. |
| 92 | em FPv1 | all in Figure 14 a,b,c,d,e | Engineered fluorescent protein based on sfGFP, with ALFA-tags (SRLEEELRRRLT) inserted into loop region and at the termini. |
| 93 | em FPv3 | all in Figure 14 a,b,c,d,e | Engineered fluorescent protein based on mGreenLantern, with HA-tags (YPYDVPDYA) inserted into loop region and at the termini. |
| 94 | em FPv4 | all in Figure 14 a,b,c,d,e | Engineered fluorescent protein based on mGreenLantern, with Moon-Tags(KNEQELLELDKWASL) inserted into loop region and at the termini. |
| 95 | emFPv5 | all in Figure 14 a,b,c,d,e | Engineered fluorescent protein based on mScarlet-I with PepTags (AVERYLKDQQLLGIW) inserted into loop region and at the termini. |
| 96 | emFPv6 | all in Figure 14 a,b,c,d,e | Engineered fluorescent protein based on mEosEM, with MoonTags (EELLSKNYHLENEVARLKK) inserted inserted into loop region and at the termini. |
| 97 | emFPv7 | all in Figure 14 a,b,c,d,e | Engineered fluorescent protein based on mScarlet-I, with triple FLAG-tags (DYKDHDGDYKDHDIDYKDDDDK) inserted into loop region and at the termini. |
| 98 | 24xGCN_v4(SunTag)-emFPv4-24xGCN_v4(SunTag) | Figure 14 a,b,c,d,e - related | Engineered fluorescent protein based on emFPv4 with 24x SunTags on both termini. |
| 99 | CCC-Tag(10xP3)-em FPv4-CCC-Tag(10xP3) | Figure 14 a,b,c,d,e - related | Engineered fluorescent protein based on emFPv4 with 10x P3 CCC tags on each terminus. |
| 100 | MS2 phage capsid protein | Figure 14f | Coat protein of the RNA bacteriophage MS2 which binds a specific stem-loop structure in viral RNA to accomplish encapsidation of its genome. |
| 101 | MS2 phage Maturation protein A | Figure 14f | Maturation protein of the RNA bacteriophage MS2 required for the typical attachment of the phage to the side of the bacterial pili. Binds to sequences located toward each end of the genome, hence circularizing it. |
| 102 | Qbeta phage capsid protein | Figure 14f | Coat protein of the Escherichia virus Qbeta which binds a specific stem-loop structure in viral RNA to accomplish encapsidation of its genome. |
| 103 | Qbeta phage Maturation protein A2 | Figure 14f | Maturation protein of the Escherichia virus Qbeta required for the typical attachment of the phage to the side of the bacterial pili; Binds to sequences located toward each end of the genome, hence circularizing it. |
| 104 | QtEnc-FLAG | Figure 9 h, 18 a | Capsid forming monomer from the encapsulin system of Q.thermotolerans tagged with C-termina FLAG epitope |
| 105 | SasG G51-G52 | Related to figure 10 d, 13 a, 17 a, b, c, d, e, f, g, 18 f, g, h, 20 a | G51-E1-G52 domains of *S.aureus* protein SasG (amino acids 419-629) and mCherry |
| 106 | QtEnc-antiGFPintrabody (QtEnc^{aGFPIB}) | Figure 16 d, 18 k, 20 a, b | Capsid forming monomer from the encapsulin system of *Q.thermotolerans* tagged with a C-terminal anti-GFP intrabody |
| 107 | MxEnc-FLAG | Figure 1 | Capsid forming monomer from the encapsulin system of *M.xanthus* tagged with C-terminal FLAG epitope |
| 108 | Tm Enc-BC2Tag | related to Figure 9 | Capsid forming monomer from the encapsulin system of *T.maritima* (amino acids 1-264) tagged with C-termina BC2 epitope |
| 109 | P2-TmEnc-antimCherry intrabody (LaM-4) | Figure 17 c, d | Capsid forming monomer from the encapsulin system of *T.maritima* (amino acids 1-264) N-terminally fused to a synthetic coiled-coil domain 'P2' (Lebar et al., 2020) and C-terminally fused to an anti-mCherry Intrabody (LaM-4) |
| 110 | MT3-DHF119-GFP | Figure 12 c, d | Fusion protein consisting of MT3, a *de novo* designed fibril forming protein(Shen et al. 2018) and GFP |
| 111 | His6-SpyCatcher-I3-01-MmMT3 | Figure 17 i | Fusion protein consisting of hexa-Histidine Tag, a SpyCatcher domain (Zakeri et al. 2012), a *de novo* designed, single component icosahedron(Hsia et al. 2016) and an MT3 domain |
| 112 | CsgA-SpyTag003 | Figure 12 a, b | Fusion protein of *E.coli* CsgA where the secretion peptide and N22 were deleted (amino acids 36-144) and SpyTag peptide |
| 113 | QtEnc-KinTag | Figure 16 b | Fusion protein of capsid forming monomer of the Encapsulin system of *Q.thermotolerans* and a synthetic peptide interacting with motor proteins (KinTag(Cross et al. 2021)) |
| 114 | sfGFP-SasG(G51-G52)-mCherry | Figure 17 a, c | Fusion protein consisting of super-folder GFP, the G51-E1-G52 domains of *S.aureus* protein SasG (amino acids 419-629) and mCherry |
| 115 | Gp41-1_sfGFP_SasG_G51-G54_SpyCatcher003 | Figure 17 g | Fusion protein consisting of Gp41-1 C-intein, superfolder GFP, SasG (comprising 4 G5 domains yielding GEGEGEG), and engineered SpyCatcher003 |
| 116 | SnoopTag_mTurquoise _SasG_G51-GS4_Gp41-1-N-lnt | Figure 17 g | Fusion protein consisting of a SnoopTag, mTurquoise2, SasG (comprising 4 G5 domains yielding GEGEGEG) and the N-intein split fragment of Gp41-1 |
| 117 | SpyTag003_mCherry_S asG_G51-G54_SnoopCatcher | Figure 17 g | Fusion protein consisting of SpyTag003, the red fluorescent protein mCherry, SasG (comprising 4 G5 domains yielding GEGEGEG) and a SnoopCatcher domain |
| 118 | sfGFP_SasG_G_NpuDn aE_N-Int_V2 | Figure 18 f, g | Fusion protein consisting of superfolder GFP, the G51 domain of SasG and the Npu DnaE N-intein split part |
| 119 | NpuDnaE_GEP_C-Int_SasG_EG_mCherry _V2 | Figure 18 h | Fusion protein consisting of Npu DnaE GEP C-Intein split fragment, SasG EG domains, where the proline residue in the G51-E junction was mutated to cysteine to serve as nucleophile in the splicing reaction, and mCherry |
| 120 | NpuDnaE_GEP_C-Int_SasG_EG_Npu_Dna E_N-Int_V2 | Figure 18 g, h | Fusion protein consisting of Npu DnaE GEP C-Intein split fragment, SasG EG domains and the Npu DnaE N-intein split part on its C-terminus. This yields a self-growing fragment which can be capped off with NpuDnaE_GEP_C-Int_SasG_EG_mCherry_V2 |
| 121 | sfGFP_SasG_G_VidaL-N-Int | Figure 18 g | Fusion protein consisting of superfolder GFP, the G51 domain of SasG and the VidaL N-Intein split part |
| 122 | VidaL-C-Int_SasG_EG_mCherry | Figure 18 h | Fusion protein consisting of VidaL C-Intein split part, SasG EG domains and mCherry |
| 123 | VidaL-C-Int_SasG_EG_VidaL-N-Int | Figure 17 a, 18 g, h | Fusion protein consisting of VidaL C-Intein split part, SasG EG domains and VidaL N-Intein split part. This building block is a self-growing unit and can be capped off with VidaL-C-Int_SasG_EG_mCherry |
| 124 | Tobacco etch virus protease (TEVp) | related to Figure 18 b, j | Tobacco etch virus protease |
| 125 | plum pox virus protease (PPVp) | related to Figure 18 | plum pox virus protease, alternative to TEVp |
| 126 | soybean mosaic virus protease (SbMVp) | related to Figure 18 | soybean mosaic virus protease, alternative to TEVp |
| 127 | sunflower mild mosaic virus protease (SuMMVp) | related to Figure 18 | sunflower mild mosaic virus protease, alternative to TEVp |
| 128 | 3xLBT15-TEVsite-MT3-QtEnc-FLAG | Figure 18 a | Fusion protein consisting of a triple engineered Lanthanide binding domain (LBT15(Sculimbrene and Imperiali 2006)), a |
| | | | TEV cleavage site, the capsid forming monomer of the Encapsulin system of *Q.thermotolerans* and a FLAG epitope |
| 129 | FLAG-N-TEV-1-118-M13 | Figure 18 a | Fusion protein consisting of a FLAG epitope, the N-terminal fragment of the Tobacco etch virus protease (TEV) (amino acids 1-118 and a C-terminal M13 calmodulin-binding peptide |
| 130 | Myc-Calmodulin-TEV-119-219 | Figure 18 a | Fusion protein consisting of a Myc epitope, Calmodulin and the C-terminal split fragment of Tobacco etch virus protease (amino acids 119-219, S153N(Sanchez and Ting 2020)) |
| 131 | MxEnc-RS20 | Figure 19 | Fusion of Encapsulin forming monomer of the Encapsulin system *M.xanthus* and the Calmodulin binding peptide RS20 |
| 132 | QtEnc-RS20 | Figure 19 | Fusion of Encapsulin forming monomer of the Encapsulin system *Q.thermotolerans* and the Calmodulin binding peptide RS20 |
| 133 | Tm Enc-RS20 | Figure 19 | Fusion of Encapsulin forming monomer of the Encapsulin system *T.maritima* and the Calmodulin binding peptide RS20 |
| 134 | CaM-HpFtn | Figure 19 b | Fusion protein of human calmodulin and *H.pylori* ferritin (AA 5-167, N19Q) |
| 135 | CaM-MT3 | Related to figure 19 b | Fusion of human Calmodulin and MmMT3 which will be recruited to the scaffold (Enc) to form a contrast halo around the element when elevated Ca²⁺ levels are present |
| 136 | MT3-TnCmin-QtSig | Figure 19 a | Fusion protein of MT3, an engineered minimal Troponin C domain of *Opsanus tau* and the Encapsulin signal of the Encapsulin system of *Q.thermotolerans* |
| 137 | MT3-TnCmin-QtEnc-FLAG | Figure 19 a | Calcium-responsive contractile linker to MT3. |
| 138 | mCherry-dTrim21 | Figure 18 I | Fusion of E3 ubiquitin-protein ligase TRIM21, with inactivating mutations I79R and S80A, and mCherry fluorescence protein |
| 139 | sfGFP-dTrim21 | Figure 18 I | Fusion of E3 ubiquitin-protein ligase TRIM21, with inactivating mutations I79R and S80A, and superfolder GFP |
| 140 | pcDNA 3.1 (+) Zeocin | Figure 4 g | mammalian expression vector |
| 141 | pIRES | Figure 4 g | internal ribosome entry site |
| 142 | QtEnc^{FLAG} | Figure 1 b | Shell forming monomer of the Encapsulin system of Quasibacillus thermotolerans with a C-terminally appended FLAG epitope connected via a short GSG linker. |
| 143 | 2xMB-QtEnc^{FLAG} | Figure 1 b, 4 g | A tandem of the *Mus musculus* Metallothionein 3 connected via GGGGSGGGGS linker fused to the shell-forming monomer of the Encapsulin system of *Quasibacillus thermotolerans* with a C-terminally appended FLAG epitope connected via a short GSG linker. |
| 144 | MT3-TmEnc^{BC2-Tag} | Figure 1 d, 9 b | Fusion of *Mus musculus* Metallothionein 3 fused to the shell forming monomer of the Encapsulin system of *Thermotoga maritima* fused to a C-terminal BC2-Tag |
| 145 | LBT1S-QtEnc^{FLAG} | Figure 2 d | Synthetic Lanthanide-bindering tag 15 fused to the shell forming monomer of the Encapsulin system of *Quasibacillus thermotolerans* fused to a C-terminal FLAG tag- |
| 146 | Twin-dLBT3-QtEnc^{FLAG} | Figure 2 d | Engineered tandem Lanthanide-binding tag fused to the shell forming monomer of the Encapsulin system of *Quasibacillus thermotolerans* fused to a C-terminal FLAG tag |
| 147 | QtEnc-SpyTag(003) | Figure 3 a, e | Capsid forming monomer from the encapsulin system of *Q.thermotolerans* tagged with C-terminal SpyTag003 |
| 148 | MUP2-QtEnc | Figure 2 c | Designed Uranyl-binding protein MUP2 fused to the shell forming monomer of the Encapsulin system of *Quasibacillus thermotolerans* |
| 149 | MUP5-QtEnc | Related to figure 2 a | Designed Uranyl-binding protein MUP5 fused to the shell forming monomer of the Encapsulin system of *Quasibacillus thermotolerans* |
| 150 | B-FABP-SpyCatcher003 | Figure 3 b | Engineered SpyCatcher 003 domain fused to fatty acid binding protein FABP |
| 151 | BM3h-SpyCatcher003 | Figure 3 e | Heme binding domain BM3h fused to engineered SpyCatcher 003 domain |
| 152 | MS2 stem loop | Figure 4 a | RNA motif for MCP |
| 153 | 2xGGGGS linker | Figure 5 a | flexible linker |
| 154 | DD-N | Figure 5 a | Engineered FKBP-derived degron |
| 155 | eUnaG-MT3-QtEnc^{FLAG} | Figure 7 b | Fusion of engineered ultrasmall fluorescent protein eUnaG followed by MT3, QtEnc and a FLAG-tag |
| 156 | eUnaG-2xMT3-QtEnc^{FLAG} | Figure 7 c | Fusion of engineered ultrasmall fluorescent protein eUnaG followed by 2xMT3, QtEnc and a FLAG-tag |
| 157 | mGreenLantern-MT3-QtEnc^{FLAG} | Figure 7 d | Fusion of the engineered green fluorescent protein mGreenLantern, MT3, QtEnc and a FLAG epitope |
| 158 | CaMPARI2-MT3-QtEnc^{FLAG} | Figure 7 d | Fusion of the engineered fluorescent Calcium integrator CaMPARI2, MT3, QtEnc and a FLAG tag |
| 159 | P2-TmEnc^{BC2-Tag} | Figure 5 f, 8 a, 20 c | Synthetic coiled-coil domain P2 (partner of P1) fused to the TmEnc (AA 1-264) with a C-terminal BC2-Tag |
| 160 | DHD13a-MxEnc^{StrepTagll} | Figure 8 a | Synthetic coiled-coil domain DHD13a (partner of DHD13b) fused to MxEnc with a StrepTagll |
| 161 | P3-QtEnc^{FLAG} | Figure 8 a | Synthetic coiled-coil domain P3 (partner of P4) fused to QtEnc with a FLAG-Tag |
| 162 | DD-N-mScarlet-I-P1 | Figure 8 a | Fusion of DD-N degron with mScarlet-I and synthetic coiled-coil domain P1 (partner of P2) |
| 163 | DD-N-mTaBFP2-DHD13b | Figure 8 a | Fusion of DD-N degron with mTagBFP2 and synthetic coiled-coil domain DHD13b (partner of DHD13a) |
| 164 | DD-N-mGreenLantern-P4 | Figure 8 a | Fusion of DD-N degron with mGreenLantern and synthetic coiled-coil domain P4 (partner of P3) |
| 165 | MT3-QtEnc^{FLAG} | Figure 9 b | Fusion of *Mus musculus* Metallothionein 3, the shell forming monomer of the Encapsulin system of *Quasibacillus thermotolerans* and a C-terminal FLAG-Tag |
| 166 | MT3-MxEnc^{FLAG} | Figure 9 c | Fusion of *Mus musculus* Metallothionein 3, the shell forming monomer of the Encapsulin system of *Myxococcus xanthus* and a C-terminal FLAG-Tag |
| 167 | MT3-MxEnc-FLAG-cMyc-NLS | Figure 9 h, j | Fusion of *Mus musculus* Metallothionein 3, the shell forming monomer of the Encapsulin system of *Myxococcus xanthus* and a C-terminal FLAG-Tag followed by a cMyc-derived nuclear localization signal (NLS) |
| 168 | MT3-QtEnc-FLAG-cMyc-NLS | Figure 9 i | Fusion of Mus musculus Metallothionein 3, the shell forming monomer of the Encapsulin system of *Quasibacillus thermotolerans* and a C-terminal FLAG-Tag followed by a cMyc-derived nuclear localization signal (NLS) |
| 169 | 3xchimericMT (3xMT)-QtEnc^{FLAG} | Figure 10 a | Fusion of 3xMT with QtEnc and a C-terminal FLAG-Tag. |
| 170 | mINT | Figure 11 a | attachment signal to vault |
| 171 | MT3-SpyCatcher003 | Figure 10 f, 12 b | MT3 fused to SpyCatcher003 |
| 172 | antiALFATag-intrabody-2xMT3-GB1-FLAG-RXXG | Figure 14 a, b | Fusion of antiALFATag intrabody with an MT3 tandem, GB1 domain, a FLAG-Tag and C-terminal RXXG degron. |
| 173 | antiHA-frankenbody-2xMeLanM-GB1-FLAG-RXXG | Figure 14 a, b | Fusion of antiHA frankenbody with a MeLanM tandem, GB1 domain, a FLAG-Tag and C-terminal RXXG degron. |
| 174 | antiMoonTag-intra body-2xAssVanabin2-GB-1-FLAG-RXXG | Figure 14 a, b | Fusion of antiMoonTag intrabody with a AssVanabin2 tandem, GB1 domain, a FLAG-Tag and C-terminal RXXG degron. |
| 175 | antiMoonTag-intrabody-2xMeLanM-GB-1-FLAG-RXXG | Figure 14 a, b | Fusion of antiMoonTag intrabody with a MeLanM tandem, GB1 domain, a FLAG-Tag, and C-terminal RXXG degron. |
| 176 | antiMoonTag-intrabody-4x(P3)-GB-1-FLAG-RXXG | Figure 14 a, b | Fusion of antiMoonTag intrabody with a 4xP3 CCC-Tag, GB1 domain, a FLAG-Tag, and C-terminal RXXG degron. |
| 177 | antiMoonTag-intrabody-2xMT3-GB-1-FLAG-RXXG | Figure 14 a, b | Fusion of antiMoonTag intrabody with an MT3 tandem, GB1 domain, a FLAG-Tag, and C-terminal RXXG degron. |
| 178 | antiMoonTag-intrabody-2xEcCusF-GB-1-FLAG-RXXG | Figure 14 a, b | Fusion of antiMoonTag intrabody with an EcCusF tandem, GB1 domain, a FLAG-Tag, and C-terminal RXXG degron. |
| 179 | LifeAct-em FPv4 | Figure 14 b, d | Fusion of LifeAct-peptide to emFPv4 |
| 180 | QtEnc^{FLAG-}(TGA) RT20s-antiGFPintrabody-HiBit | Figure 15 b | DNA sequence encoding QtEnc-FLAG followed by a TGA stop codon, followed by the read-through promoting sequence "RT20s" followed by an anti-GFPintrabody with a C-terminal HiBit peptide. |
| 181 | QtEnc^{FLAG-}(TGA) RT20s-Ssp DnaE (N159A)QtEnc-antiGFPintrabody-HiBit | Figure 15 b | DNA sequence encoding QtEnc-FLAG followed by a TGA stop codon, followed by a read-through promoting sequence "RT20s", followed by an Ssp DnaE intein mutant (N159A) which exhibits hyperfast N-terminal cleavage, followed by a P2A peptide followed by a second full-length QtEnc with a C-terminal antiGFP-intrabody and a HiBit peptide. |
| 182 | P2A peptide | Figure 15 b | |
| 183 | 2xMT3-QtEnc-antiGFPintrabody | Figure 16 c | Tandem MT3 fused to QtEnc which has a C-terminal anti GFP intra body |
| 184 | sfGFP | Related to figure 12 c, d, 17 a, b, c, d, e, f, g, 18 g, k | |
| 185 | mCherry | Figure 17 a, b, c, d, e, f, g, 18 g, k | |
| 186 | antiGFP-intrabody | Figure 17 a, 18 l | |
| 187 | antimCherry intrabody (LaM-4) | Figure 17 a, 18 l | |
| 188 | sfGFP-SasG-G51-G54-mCherry | Figure 17 a | SasG capped off with two fluorscent proteins |
| 189 | MT3-QtEnc-antiGFPintrabody | Figure 17 c | MT3 fused to QtEnc with a C-terminal anti GFP intrabody |
| 190 | BmTyr-P2 | Figure 20 c | Fusion of BmTyr and synthetic coiled-coil domain P2 (partner of P1) |
| 191 | B-FABP-sfGFP | Figure 10 e | Fusion of Brain FABP and superfolder GFP |
| 192 | BM3h-sfGFP | Figure 10 e | Fusion of BM3h and superfolder GFP |
| 193 | cpFABP | Figure 13 b | Circularly permuted FABP, Nterm and Cterm are both alpha helices |
| 194 | Intein C-6xB-FABP-Intein N | Figure 13 a | Fusion of six B-FABP flanked by DnaE split intein fragments C-intein (AA 103-137) on the N-term and N-Intein (AA 1-102) |
| 195 | Intein C-3x(B-ABP-MmMT3)-Intein N | Figure 13 a | Fusion of three repeats of B-FABP fused to MmMT3 flanked by DnaE split intein fragments C-intein (AA 103-137) on the N-term and N-Intein (AA 1-102) |
| 196 | Intein C-2xB-FABP-SpyTag003-2xB-FABP-Spycatcher003-2xB-FABP-Intein N | Figure 13 c | Fusion of three repeats of 2x B-FABP intermixed by SpyTag003 and Spycatcher003 and flanked by DnaE split intein fragments C-intein (AA 103-137) on the N-term and N-Intein (AA 1-102) |
| 197 | QtEnc-M13 | Figure 19 a | Fusion of QtEncapsulin and M13 a synthetic peptide binding Calmodulin |
| 198 | B-FABP-CaM | Figure 19 a | Fusion of Brain FABP and Calmodulin |
| 199 | AG4 | related to Figure 2 | Synthetic Silver mineralizing peptide 4 |
| 200 | dCas13 | related to Figure 4 | Catalytically inactive Cas13b is from Prevotella sp as an RNA-binding protein |
| 201 | Pumilio homology domain | related to Figure 4 | RNA-binding protein Uniprot: Q14671 |
| 202 | eUnaG | Related to fiigure 7 a, b, c, e, f, 11 e, | Enhanced Ultra small fluorescent protein from eel |
| 203 | mGreenLantern | Figure 7 d | Improved green fluorescent protein based on GFP |
| 204 | CaMPARI 2 | Figure 7 d | Engineered fluorescent Calcium integrator |
| 205 | mRFP | | Monomeric red fluorescent protein |
| 206 | FHA2 | related to Figure 19b | phostphopetptide binding Forkhead-associated domain |
| 207 | AP205 Phage | Fig. 10 b, d, 14 f | Coat protein |
| 208 | mEos4b:APEX2:mINT | Figure. 11a,b | Fusion of mEos4b together with APEX2 to a C-terminal minimal interaction domain (mINT) capable to interact with the interior of the vault complex. |
| 209 | MmuGap43-palmi:MVP | Figure. 11a,b | Fusion of mouse Gap43 palmitoylation signal to the N-terminus of the major vault protein (MVP). |
| 210 | SNAPtag-QtEnc^{FLAG} | Figure 8 b | N-terminal fusion of SNAP-tag connected via GS linker to QtEnc with a C-terminal FLAG epitope |
| 211 | Halotag-QtEnc^{FLAG} | Figure 8 b | N-terminal fusion of Halo-tag connected via GS linker to QtEnc with a C-terminal FLAG epitope |
| 212 | MT3-PfEnc^{FLAG} | Figure 9 d | Fusion of MT3 to AA 110-346 of *Pyrococcus furiosus* Encapsulin monomer with a C-terminal FLAG epitope. |
| 213 | PfFt | Figure 19 | Ferritin from *Pyrococcus furiosus* |
| 214 | mEos4b | Related to figure 11 a, b | https://www.fpbase.org/protein/meos4b/ |
| 215 | mEosEM | Related to figure 7 | https://www.fpbase.org/protein/meosem/ |
| 216 | iRFP713 | | https://www.fpbase.org/protein/irfp713/ |
| 217 | TeAPCα | | https://www.fpbase.org/protein/teapc/ |
| 218 | DMT1 | Figure 6 d | Human divalent metal transporter 1 |
| 219 | Zip14 | Figure 6 d | Human divalent metal transporter Zip14 |
| 220 | Cystathionine gamma-lyase-QtSig | Figure 6 e | Fusion of Cystathionine gamma-lyase with QtSig |
| 221 | Secreted alkaline phosphatase-QtSig | Figure 6 e | Fusion of SEAP, a secreted form of Human embryonic alkaline phosphatase, and QtSig |
| 222 | MnxG-QtSig | Figure 6 e | Fusion of MnxG, component of the *Bacillus* sp. Multimeric multicopper oxidase Mnx, and QtSig |
| 223 | MnxE | Figure 6 e | Component of the *Bacillus* sp. Multimeric multicopper oxidase Mnx |
| 224 | MnxF | Figure 6 e | Component of the *Bacillus* sp. Multimeric multicopper oxidase Mnx |
| 225 | SUP | alternative to MUP2 and MUP5 in Figure 2 | Designed uranyl binder with femtomolar affinity |
| 226 | P3-MT3-QtEnc | related to Figure 8 | Coiled-coil adapter to recruit an encapsulated cargo, such as the fluorescent protein mEosEM fused to P4 to an encapsulin already harboring an MT3 for dual EM and fluorescence detection. |
| 227 | Mt3-QtEnc-eUnaG | alternative option to fluorescent protein complementation shown in Figure 7 | Fusion of the fluorescent protein eUnaG to the outer surface of encapsulin |
| 228 | MT3-sfGFP | alternative option to fluorescent protein complementation shown in Figure 7 | CGU for attaching to the outer surface of encapsulin via anti-GFP antibody |
| 229 | QtEnc-B-FABP | Figure 10, particularly e,f | Fusion of QtEnc and Brain FABP, embodiment of concentric barcodes added to the outer surface of encapsulins |
| 230 | QtEnc-Bm3h | Figure 10, particularly e,f | Fusion of QtEnc and Bm3h, embodiment of concentric barcodes added to the outer surface of encapsulins |
| 231 | QtEnc-MT3 | Figure 10 e | Fusion of QtEnc and MT3, embodiment of concentric barcodes added to the outer surface of encapsulins |
| 232 | B-FABP-sfGFP | e.g. Figure 10e | Fusion of Brain FABP and superfolder GFP, for attachment via anti-GFP intrabody attachment points, e.g. to the outer surface of Encapsulins. |
| 233 | Bm3h-sfGFP | e.g. Figure 10e | Fusion of Bm3h and superfolder GFP, or attachment via anti-GFP intrabody attachment points, e.g. to the outer surface of Encapsulins. |
| 234 | MT3-Lam4 | related to Figure 17a | Fusion of MT3 and Lam4, for attachment to mCherry, e.g. on SasG |
| 235 | B-FABP-Lam4 | related to Figure 17a | Fusion of Brain FABP and Lam4, for attachment to mCherry, e.g. on SasG |
| 236 | Bm3h-Lam4 | related to Figure 17a | Fusion of Bm3h and Lam4, for attachment to mCherry, e.g. on SasG |
| 237 | GGSG linker | Figure 13 | General linker used for protein fusions, including direct fusions of CGUs to achieve the nanometer sizes of a structural element |
| 238 | Recoverin | Figure 19c | myristoyl switch motif for calcium-dependent re-localization a structural element such as vaults to the membrane |
| 239 | synthide-2-FHA2 | related to Figure 19b | synthide-2 binding for FHA2 |

## Claims

1. A genetically controlled nanoscopy contrast-generating unit comprising a metal interactor,
wherein the metal interactor is compatible with nanoscopy fixation protocols, nanoscopy post-fixation protocols, and nanoscopy metal staining protocols,
wherein the metal interactor is a molecule to which metal ions can bind to or react with, and
wherein the metal interactor is not a ferroxidase or an enzymatic product from an exogenous substrate.

2. The genetically controlled nanoscopy contrast-generating unit according to claim 1,
wherein the metal interactor comprises:
a metal-interacting peptide/protein,
a metal-binding peptide/protein,
a lipid-binding protein,
an RNA-binding protein,
a DNA or RNA molecule,
a polymerizing or synthesizing enzyme,
a biomineralizing enzyme,
a bioconjugation tag,
or a combination thereof.

3. The genetically controlled nanoscopy contrast-generating unit according to claim 1 or 2,
wherein the metal interactor comprises one or more of the following:
a metallothionein,
osmiophilic amino acids,
a lead binder,
a lanthanide binder,
a uranyl-binder,
a vanadium binder,
a copper and silver-binder,
a cobalt or nickel binder,
a fatty-acid-binding protein,
the lipid-binding domain of a cytochrome P450,
an RNA aptamer-binder:RNA aptamer pair,
a programmable RNA binding protein,
a polymerizing kinase,
a phytochelatin synthase,
a tyrosinase.

4. The genetically controlled nanoscopy contrast-generating unit according to any one of claims 1-3,
wherein the genetically controlled nanoscopy contrast-generating unit further comprises a fluorophore or a chromophore,
wherein the fluorophore is a fluorescent protein, a co-factor in a fluorescent protein, or an exogenous fluorophore,
wherein the chromophore is a chromoprotein, a co-factor in a chromoprotein, or an exogenous chromophore, and
wherein the exogenous fluorophore or the exogenous chromophore can bind directly to the metal interactor.

5. The genetically controlled nanoscopy contrast-generating unit according to any one of claims 1-4,
wherein the metal interactor comprises at least one metallothionein, at least one fatty-acid binding protein, at least one lipid-binding domain, or at least one RNA molecule, and a fluorophore,
wherein the fluorophore is a fluorescent protein.

6. The genetically controlled nanoscopy contrast-generating unit according to any one of claims 1-5,
wherein the genetically controlled nanoscopy contrast-generating unit further comprises at least one attachment point, and
wherein said at least one attachment point is selected from the group consisting of
nanobodies,
frankenbodies,
coiled-coil domains,
isopeptide-forming partners,
bioconjugation-tags,
split-inteins,
calmodulin-binding peptides,
phosphorylatable peptides, and
conformation-changing peptides including troponins,
as well as combinations thereof.

7. A genetically controlled structural element,
wherein said genetically controlled structural element comprises and spatially organizes the genetically controlled nanoscopy contrast-enhancing unit of any one of claims 1 to 6, wherein the genetically controlled structural element possesses a pre-determined shape and pre-determined nanoscale size, and
wherein the genetically controlled structural element generates a shape that can be differentiated at the nanoscale, and
wherein the shape is optionally a barcode.

8. The genetically controlled structural element according to claim 7,
wherein the genetically controlled structural element comprises
a member of the encapsulin family,
a vault,
an MS2 phage,
a Qbeta phage,
an AP205 phage,
an engineered mono-to-polyvalent hub,
a filament, or
a linker,
or combinations or variants thereof.

9. The genetically controlled structural element according to claim 7 or 8,
wherein the genetically controlled structural element further comprises at least one attachment point,
wherein said at least one attachment point is selected from the group consisting of
nanobodies,
frankenbodies,
coiled-coil domains,
isopeptide-forming partners,
bioconjugation-tags,
split-inteins,
calmodulin-binding peptides,
phosphorylatable peptides, and
conformation-changing peptides including troponins,
as well as combinations thereof, and
wherein the genetically controlled nanoscopy contrast-generating unit according to any one of claims 1 to 6 interacts or binds to the at least one attachment point.

10. The genetically controlled structural element according to any one of claims 7-9,
wherein the genetically controlled structural element is an encapsulin,
wherein the genetically controlled contrast-generating units are selected from the group of metallothionein MT3, a series of different metallothionein species, fatty acid-binding proteins (FABPs), and the lipid-binding domain of the cytochrome P450, or combinations thereof,
wherein the lipid-binding domain of the cytochrome P450 is optionally the lipid- and heme-binding domain of the cytochrome P450 BM3 (BM3h),
wherein one to three copies of the MT3 or one to three different metallothionein species are bound to the N-terminus of the encapsulin, and
wherein one or more MT3s, FABPs, or BM3hs are bound to the surface of the encapsulin, optionally via a spacer, wherein the spacer is optionally a SasG of variable length, and
wherein such organization is configured to generate the distinct shape of a barcode,
wherein the barcode is a concentric barcode differentiable at the nanoscale, and optionally
wherein said concentric barcode encodes information on cellular states.

11. A genetically controlled scaffold,
wherein said genetically controlled scaffold comprises and spatially organizes the genetically controlled structural elements according to any one of claims 7 to 10,
wherein such spatial organization generates distinct geometric patterns that can be differentiated at the nanoscale, and optionally
wherein said distinct geometric patterns encode information on cellular states.

12. The genetically controlled scaffold according to claim 11,
wherein the genetically controlled scaffold is selected from the group consisting of
an endogenous cellular scaffold,
a *de novo* designed scaffold,
a CsgA element,
a SasG element,
an RNA or DNA structure,
or combinations thereof.

13. The genetically controlled scaffold according to claim 11 or 12, comprising
a SasG of variable length,
encapsulin as structural element, and
at least one contrast generating unit MT3, or a series of different metallothionein species,
wherein the encapsulins are connected and spatially organized via SasG, and
wherein the encapsulins comprise the MT3, or a series of different metallothionein species contrast generating units.

14. Use of the genetically controlled structural elements according to any of claims 7 to 10, and/or the genetically controlled scaffolds according to any of claims 11-13 for nanoscopy detection, wherein nanoscopy detection comprises molecular mapping and/or geometric sensing.

15. The use of claim 14, wherein the nanoscopy detection is molecular mapping, and
wherein said molecular mapping provides information on the subcellular distribution of a molecule of interest within a cell or tissue via the binding of genetically controlled structural elements according to any of claims 7 to 10, and/or genetically controlled scaffolds according to any of claims 11 or 13 to a molecule of interest, which thus determines the subcellular localization of the genetically controlled structural elements and/or the genetically controlled scaffolds.

16. The use of claim 14, wherein the nanoscopy detection is geometric sensing, and
wherein said geometric sensing provides information on a specific cellular state, a cellular process, or the presence or absence of an analyte or environmental parameter of interest within a cell or tissue, or the response to an external stimulus, and
wherein the shape generated by the genetically controlled structural elements and/or the geometric pattern generated by the genetically controlled scaffolds changes in response to a pre-defined cellular state, a pre-defined cellular process, at least one analyte, or at least one environmental parameter, or an external stimulus.

17. The use according to claim 14, wherein the nanoscopy detection comprising mapping and/or the geometrical sensing further comprises geometric actuation,
wherein said geometric actuation alters a cellular state or process by a biomechanical, and/or biochemical effect or via an external stimulus, or the deposition of external energy, electromagnetic radiation, mechanical energy, or magnetic gradients.

18. The genetically controlled nanoscopy contrast-generating unit according to any of claims 1-6, the genetically controlled structural element according to any of claims 7-10 and/or the genetically controlled scaffold according to claim 11-13,
wherein said contrast generating unit, structural element and/or scaffold is compatible with intact cell systems, preferably with mammalian cells and organoids or biomedical model organisms.

19. Nanobiomaterial consisting of isolated genetically controlled structural elements according to any of claims 7 to 10, and/or isolated genetically controlled scaffolds according to any of claims 11-13,
wherein the genetically controlled structural elements and/or genetically controlled scaffolds are isolatable from genetically modified cells or cell-free systems.

20. A vector comprising the nucleic acid encoding the genetically controlled unit according to any of claims 1-6, the genetically controlled structural element according to any of claims 7-10, and/or the genetically controlled scaffold according to claims 11-13.
